(19) Europäisches Patentamt
European Patent Office
Office européen des brevets

(11) **EP 4 625 420 A1**

(12) **EUROPEAN PATENT APPLICATION**
published in accordance with Art. 153(4) EPC

(43) Date of publication:
**01.10.2025 Bulletin 2025/40**

(21) Application number: **23900564.8**

(22) Date of filing: **01.12.2023**

(51) International Patent Classification (IPC):
**G16B 40/00** (2019.01)      **C07K 16/00** (2006.01)
**C12P 21/08** (2006.01)      **C40B 40/10** (2006.01)

(52) Cooperative Patent Classification (CPC):
**C07K 16/00; C40B 40/10; G16B 40/00**

(86) International application number:
**PCT/JP2023/043058**

(87) International publication number:
**WO 2024/122449 (13.06.2024 Gazette 2024/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.12.2022  JP 2022194776**

(71) Applicants:
- **RevolKa Ltd.**
  **Sendai-shi, Miyagi 980-8579 (JP)**
- **Tohoku University**
  **Sendai-shi, Miyagi 980-8577 (JP)**

(72) Inventors:
- **KUWABARA, Naoyuki**
  **Sendai-shi, Miyagi 980-8579 (JP)**
- **SUZUKI, Emi**
  **Sendai-shi, Miyagi 980-8579 (JP)**
- **KONDO, Taishi**
  **Sendai-shi, Miyagi 980-8579 (JP)**
- **TAMURA, Yuki**
  **Sendai-shi, Miyagi 980-8579 (JP)**

- **NAKAZAWA, Hikaru**
  **Sendai-shi, Miyagi 980-8577 (JP)**
- **YAMAZAKI, Ryo**
  **Sendai-shi, Miyagi 980-8579 (JP)**
- **ITO, Tomoyuki**
  **Sendai-shi, Miyagi 980-8577 (JP)**
- **TAKAHASHI, Misaki**
  **Sendai-shi, Miyagi 980-8579 (JP)**
- **KAWADA, Sakiya**
  **Sendai-shi, Miyagi 980-8577 (JP)**
- **UMETSU, Mitsuo**
  **Sendai-shi, Miyagi 980-8577 (JP)**
- **KATAOKA, Shiro**
  **Sendai-shi, Miyagi 980-8579 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **ANTIBODY DESIGN METHOD THROUGH MACHINE LEARNING**

(57)    The present invention relates to a method of producing an antibody through machine learning. More specifically, the present invention relates to a method of producing an antibody that is optimized for a plurality of characteristics including at least two of an expression level, binding activity, stability, and solubility; and the like.

**EP 4 625 420 A1**

## Fig.3

## Description

**Technical Field**

Related Application

The present application claims priority to Japanese Patent Application No. 2022-194776 (filed on December 6, 2022), the contents of which are incorporated herein by reference.

Technical Field:

The present invention relates to a method of producing an antibody through machine learning. More specifically, the present invention relates to a method of producing an antibody that is optimized for a plurality of characteristics including at least two of an expression level, binding activity, and stability.

**Background Art**

Since antibody molecules have high specificity for their targets, they have been used as biopharmaceuticals and diagnostic agents, and new antibodies can be obtained with a certain degree of certainty using a hybridoma method or an *in vitro* selection method. Also, as a method for improving the affinity or altering the specificity of antibodies, a method of combining a random mutagenesis method using error-prone PCR or the like, and a phage display method, which is an *in vitro* selection method, is a general method. However, as a technique for improving the stability or solubility, reliable techniques that are commonly used have not been established. As a technique for stabilizing the structure, there are only a few methods into which mutations are introduced into complementarity-determining regions (CDRs) that recognize targets, whereas there are more methods of introducing mutations into framework regions (FRs), which are important for structure formation. On the other hand, as a technique for improving the solubility, it is considered that solvent-exposed residues, residues with hydrophobicity and/or charge, and the vicinity of paratopes affect the solubility, and there are therefore many studies for introducing mutations into these regions.

For stabilizing the structure of antibodies, a technique of combining a somatic hypermutation introduction method and a mammalian cell display method, and/or CDR grafting for grafting CDR regions using antibodies that have stable framework structures as a scaffold have been attempted; but there are many cases where the stability and binding properties have a trade-off relationship because the conformational changes in the CDRs are unavoidable. As a technique of using an amino acid sequence information, there are a method of mutating each residue into an amino acid of a high appearance frequency from an alignment of a group of sequences with high homology to the amino acid sequence of the protein of interest, and a method of predicting aggregation-prone regions (APRs) from properties of individual amino acids on the sequence and mutating them into amino acids having properties of suppressing aggregation. However, trade-offs, in which the binding activity is reduced because of slight structure changes in the FR due to mutation, often occur, and there is thus a need to use conformational information or the like. In addition, as a technique of mainly using a three-dimensional structure information, there have been reported a method of predicting domain interface interactions and mutating into amino acids such that packing efficiency improves, and a method of simulating protein movement under stressed conditions such as heat and pH and introducing mutations in which the free energy of folding is minimized; but they require conformational information, and/or they are not highly reliable in achieving the binding activity and structural stability at the same time.

On the other hand, regarding the solubility of proteins including antibodies, a technique of predicting the solubility from the physiochemical characteristics of amino acids and the like has been proposed; but its prediction accuracy is not high, and there are some cases where it has a trade-off relationship with the structural stability and/or functional characteristics.

Recently, studies have been conducted to more efficiently modify the functions of proteins such as antibodies and enzymes. In these studies, a mutant library is produced in a certain scale, and amino acid sequences and functions of mutants are experimentally measured to obtain data linked therewith, and then the data is used as training data to construct a machine learning model for predicting functions. Accordingly, by using the constructed machine learning model, mutants which are expected to improve the functions are predicted.

Regarding datasets for machine learning, two datasets: a direct linked dataset and an indirect linked dataset of amino acid sequences and values of the functions and physical properties, are applied. For the direct linked dataset, values of the functions and physical properties of each mutant are measured for each mutant and linked to a corresponding sequence of the mutant (Non Patent Literature 1 and the like). On the other hand, for the indirect linked dataset, values of the functions and physical properties are not directly measured, and the read counts for amino acid sequences by deep sequence analysis or the like are used as alternatives to the values of the functions and physical properties to create a dataset (Non Patent Literatures 2 and 3).

The direct linking between amino acid sequences and values of the functions and physical properties has a possibility to be a high-quality dataset for machine learning; but generating a large-sized dataset is difficult, only tens to hundreds of datasets can be generated, and sequences for exploring are also limited. On the other hand, although the data quality of the indirect linked dataset is lower than that of the direct linked dataset, larger-sized data of amino acid sequences that can be obtained by deep sequence analysis can be used. Therefore, when the positions and the number of mutant residues and expressed amino acids are limited, the direct linked dataset is applied, and for discovering antibody lead molecules by a molecule presentation method, the indirect linked dataset is often applied.

One of the interesting points of machine learning is that a solution can be derived in complex phenomena where a simultaneous solution across multiple conditions is required (multi-task machine learning). To predict amino acid sequences that optimize a plurality of functions and physical properties of proteins in a simultaneous process, it is necessary to link the amino acid sequences with respective values of the functions and physical properties; but the values of the various functions and physical properties are difficult to express from the deep sequence analysis indirectly, and it is therefore desirable to use the direct linked dataset in which a measurement method suitable to obtain respective values of the functions and physical properties can be used. Nevertheless, a plurality of different functions and physical properties based on different measurement data are often contradictory to each other (e.g., antigen-binding functions versus expression levels and heat resistance of an antibody), and there are often cases where a plurality of functions and physical properties do not have common factors or useful characteristics within a target spatial sequence that can be handled by the direct linked dataset. For that, an approach has been proposed in which machine learning is conducted on each single value of a function and physical property by direct linking, and then, the obtained results are combined to improve a plurality of functions and physical properties (Patent Literatures 1 and 2).

The present inventors have confirmed that the following characteristics based on one piece of measurement data of fluorescence spectrum: fluorescence intensity and yellow fluorescence ratio, can be simultaneously optimized (Non Patent Literature 1 cited above). However, as mentioned above, a plurality of functions and physical properties based on different measurement data are often contradictory to each other, and hence, no approach has yet been reported in which a plurality of characteristics based on different measurement data can be optimized in a simultaneous process.

**Citation List**

**Patent Literature**

Patent Literature 1: WO2005/012877
Patent Literature 2: WO2005/013090

**Non Patent Literature**

Non Patent Literature 1: Saito et al., "Machine-Learning-Guided Mutagenesis for Directed Evolution of Fluorescent Proteins" ACS Synth Biol. 2018;7(9):2014-2022
Non Patent Literature 2: Liu et al., "Antibody complementarity determining region design using high-capacity machine learning" Bioinformatics, 2020;36(7):2126-2133
Non Patent Literature 3: Saka et al., "Antibody design using LSTM based deep generative model from phage display library for affinity maturation" Scientific Reports, 2021;11(1):5852

**Summary of Invention**

**Technical Problem**

An object of the present invention is to provide a method of producing an antibody that is optimized for a plurality of characteristics, such as a protein design (production) method in which an antibody protein is optimized for a plurality of characteristics, including at least two of an expression level, binding activity, stability, and solubility in a simultaneous process.

**Solution to Problem**

The present inventors have evaluated a plurality of characteristics including the expression level, binding activity, stability, and solubility on some in a mutant library prepared by modifying at least some of residues that are not the amino acid of the highest appearance frequency in a group of antibody sequences into the amino acid of the highest appearance frequency, scored the characteristics as one value per mutant, and conducted machine learning by using the score as training data, and thereby succeeded in optimizing the characteristics in a simultaneous process to predict amino acid sequences.

That is, the present invention relates to the following [1] to [26].

[1] A method of producing an antibody that is optimized for a plurality of characteristics including at least two of an expression level, binding activity, and stability of the antibody, comprising:

> 1) providing a library composed of mutants prepared by modifying at least some of residues not being an amino acid of the highest appearance frequency in a group of antibody sequences of a target antibody, into the amino acid of the highest appearance frequency;
> 2) determining respective characteristic values indicating the plurality of characteristics of some of the mutants in the library, and scoring the characteristic values as one value per mutant by normalizing and integrating the characteristic values;
> 3) conducting machine learning by using the score values and ranking the mutants included in the library; and
> 4) selecting an antibody that is optimized for the plurality of characteristics, based on the ranking results.

[2] The method according to [1], wherein the characteristic values are values each obtained by converting measurement data related to the characteristics of each mutant into numerical values, as a ratio or difference relative to a target value.

[3] The method according to [1] or [2], wherein the scoring is performed according to the following formula (I):

Score value = $f$(1st characteristic value - reference value of 1st characteristic) $\times$ $f$(2nd characteristic value - reference value of 2nd characteristic)... $\times$ $f$(nth characteristic value - reference value of nth characteristics)      [Formula (1)]

wherein n is the number of characteristics to be optimized, f(x) is any selected from the group consisting of a sigmoid function (x), a hyperbolic tangent function (x), a Gaussian function (x), a lognormal distribution function (x), a ReLU function (x), a linear function (x), an n-dimensional function (x), an exponential function (x), a logarithmic function (x), a hyperbolic function (x), and a combination thereof.

For example, in the case of a plurality of characteristics including the expression level, binding activity, and stability, the scoring is performed according to the following formula (I):

Score value = $f$(characteristic value of expression level - reference value of expression level) $\times$ $f$ (characteristic value of binding activity - reference value of binding activity) $\times$ $f$(characteristic value of stability - reference value of stability)... $\times$ $f$(nth characteristic value - reference value of nth characteristic)      [Formula (1)]

[4] The method according to [3], wherein f(x) is a sigmoid function (x), a hyperbolic tangent function (x), a Gaussian function (x), or a lognormal distribution function (x).

[5] The method according to any of [1] to [4], wherein the machine learning is conducted by any selected from Bayesian linear regression, linear regression, Gaussian process regression, logistic regression, decision tree, simple perceptron, multilayer perceptron, neural network, deep neural network, k-nearest neighbor algorithm, and support vector machine.

[6] The method according to any of [1] to [5], wherein the plurality of characteristics further include solubility. For example, the plurality of characteristics are or include "expression level, binding activity, and solubility", "expression level, stability, and solubility", "binding activity, stability, and solubility", or "expression level, binding activity, stability, and solubility".

[7] The method according to any of [1] to [6], wherein the stability is functional thermostability and structural thermostability.

[8] A method of producing an antibody that is optimized for a plurality of characteristics including at least two of an expression level, binding activity, and stability, comprising:

1) providing a first library composed of mutants prepared by modifying at least some of residues not being an amino acid of the highest appearance frequency in a group of antibody sequences of a target antibody, into the amino acid of the highest appearance frequency;

2) determining respective characteristic values indicating the plurality of characteristics of some of the mutants in the first library, and scoring the plurality of characteristic values as one value per mutant by normalizing and integrating the plurality of characteristic values;

3) conducting machine learning by using the score values and ranking the mutants;

4) obtaining a second library, based on the ranking results; and

5) screening the second library to determine an antibody that is optimized for the plurality of characteristics.

[9] A method of producing a library consisting of a protein that is optimized for a plurality of characteristics including at least two of an expression level, binding activity, and stability, comprising:

1) providing a first library composed of mutants prepared by modifying at least some of residues not being an amino acid of the highest appearance frequency in a group of antibody sequences of a target antibody, into the amino acid of the highest appearance frequency;

2) determining respective characteristic values indicating the plurality of characteristics of some of the mutants in the first library, and scoring the plurality of characteristic values as one value per mutant by normalizing and integrating the plurality of characteristic values;

3) conducting machine learning by using the score values and ranking the mutants; and

4) obtaining a second library, based on the ranking results.

[10] The method according to [8] or [9], wherein the plurality of characteristic values are values each obtained by converting measurement data related to the characteristics of each mutant into numerical values, as a ratio or difference relative to a target value.

[11] The method according to any of [8] to [10], wherein the scoring is performed according to the following formula (I):

Score value = $f$(1st characteristic value - reference value of 1st characteristic) $\times$ $f$(2nd characteristic value - reference value of 2nd characteristic)... $\times$ $f$(nth characteristic value - reference value of nth characteristics)  [Formula (1)]

In the above formula, n is the number of characteristics to be optimized.

Here, n is the number of characteristics to be optimized, f(x) is any selected from the group consisting of a sigmoid function (x), a hyperbolic tangent function (x), a Gaussian function (x), a lognormal distribution function (x), a ReLU function (x), a linear function (x), an n-dimensional function (x), an exponential function (x), a logarithmic function (x), a hyperbolic function (x), and a combination thereof.

For example, in the case of a plurality of characteristics including the expression level, binding activity, and stability, the scoring is performed according to the following formula (I):

Score value = $f$(characteristic value of expression level - reference value of expression level) $\times$ $f$(characteristic value of binding activity - reference value of binding activity) $\times$ $f$(characteristic value of stability - reference value of stability) ... $\times$ $f$(nth characteristic value - reference value of nth characteristic)  [Formula (1)]

[12] The method according to [11], wherein f(x) is a sigmoid function (x), a hyperbolic tangent function (x), a Gaussian function (x), or a lognormal distribution function (x).

[13] The method according to any of [8] to [12], wherein the machine learning is conducted by any selected from Bayesian linear regression, linear regression, Gaussian process regression, logistic regression, decision tree, simple perceptron, multilayer perceptron, neural network, deep neural network, k-nearest neighbor algorithm, and support vector machine.

[14] The method according to any of [8] to [13], wherein the plurality of characteristics further include solubility. For example, the plurality of characteristics are or include "expression level, binding activity, and solubility", "expression level, stability, and solubility", "binding activity, stability, and solubility", or "expression level, binding activity, stability, and solubility".

[15] The method according to any of [8] to [14], wherein the stability is functional thermostability and structural thermostability.

[16] A method of producing an antibody that is optimized for a plurality of characteristics including at least two of a binding activity and solubility comprising:

1) providing a library composed of mutants prepared by modifying at least some of residues not being an amino acid of the highest appearance frequency in a group of antibody sequences of a target antibody, into the amino acid of the highest appearance frequency;
2) determining respective characteristic values indicating the plurality of characteristics of some of the mutants in the library, and scoring the characteristic values as one value per mutant by normalizing and integrating the characteristic values;
3) conducting machine learning by using the score values and ranking the mutants included in the library; and
4) selecting an antibody that is optimized for the plurality of characteristics, based on the ranking results.

[17] The method according to [16], wherein the plurality of characteristic values are values each obtained by converting measurement data related to the characteristics of each mutant into numerical values, as a ratio or difference relative to a target value.

[18] The method according to [16] or [17], wherein the scoring is performed according to the following formula (I):

Score value = $f$(1st characteristic value - reference value of 1st characteristic) $\times$ $f$(2nd characteristic value - reference value of 2nd characteristic)... $\times$ $f$(nth characteristic value - reference value of nth characteristics) [Formula (1)]

wherein n is the number of characteristics to be optimized, f(x) is any selected from the group consisting of a sigmoid function (x), a hyperbolic tangent function (x), a Gaussian function (x), a lognormal distribution function (x), a ReLU function (x), a linear function (x), an n-dimensional function (x), an exponential function (x), a logarithmic function (x), a hyperbolic function (x), and a combination thereof.

[19] The method according to [18], wherein f(x) is a sigmoid function (x), a hyperbolic tangent function (x), a Gaussian function (x), or a lognormal distribution function (x).

[20] The method according to any of [16] to [19], wherein the machine learning is conducted by any selected from Bayesian linear regression, linear regression, Gaussian process regression, logistic regression, decision tree, simple perceptron, multilayer perceptron, neural network, deep neural network, k-nearest neighbor algorithm, and support vector machine.

[21] An antibody Fv variant, wherein amino acid residues at positions 60 and 85 in a light chain amino acid sequence set forth in SEQ ID NO: 1 are substituted with aspartic acid and aspartic acid, respectively, and amino acid residues at positions 16, 21, and 79 in a heavy chain amino acid sequence set forth in SEQ ID NO: 2 are substituted with glycine, serine, and tyrosine, respectively.

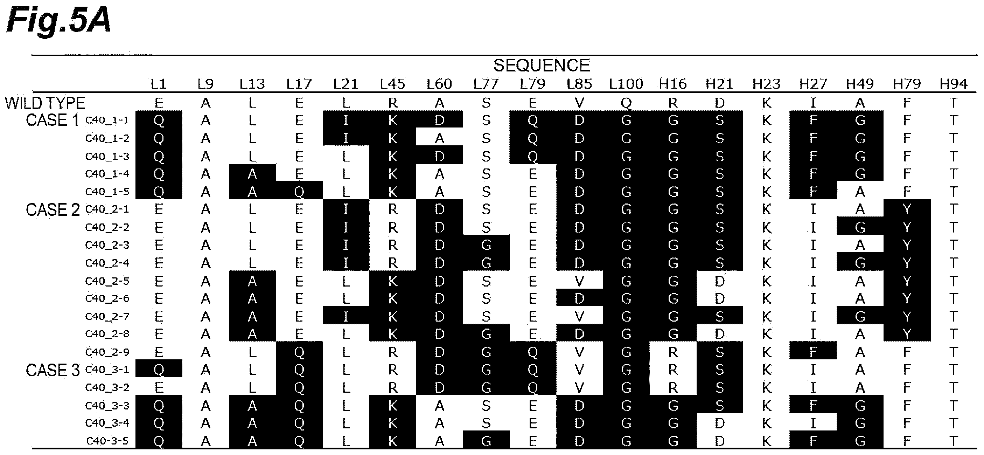

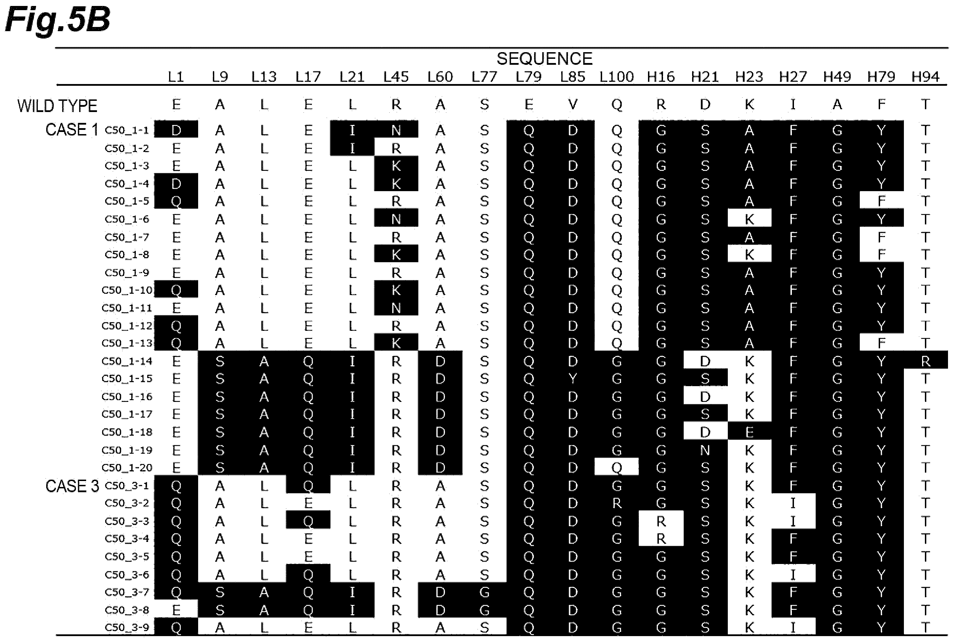

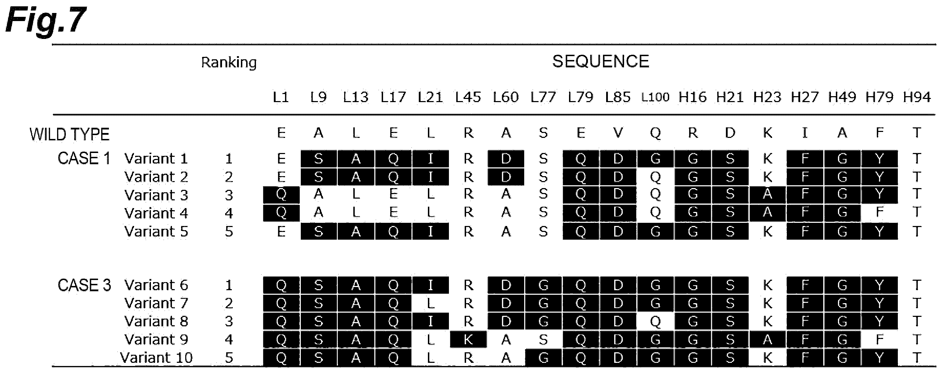

[22] An antibody Fv variant, wherein at least some of the amino acid residues at positions 1, 9, 13, 17, 21, 45, 60, 77, 79, 85, and 100 in the light chain amino acid sequence set forth in SEQ ID NO: 1, and the amino acid residues at positions 16, 21, 23, 27, 49, 79, and 94 in the heavy chain amino acid sequence set forth in SEQ ID NO: 2 are substituted, preferably an antibody Fv variant, wherein the amino acid residues are each substituted with the sets of the amino acids shown in Figures 5 and 7.

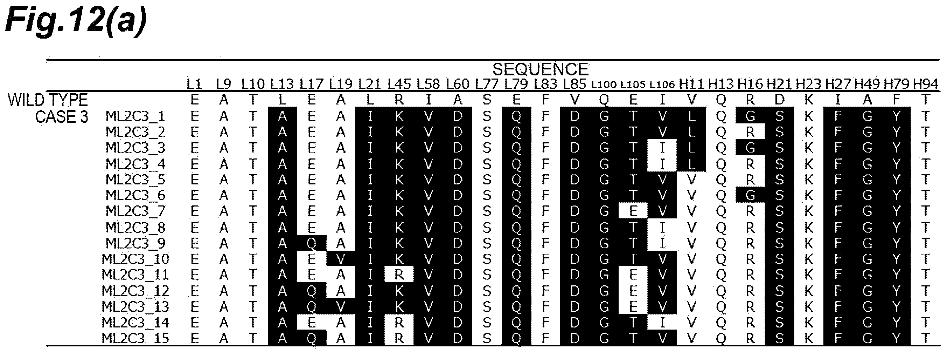

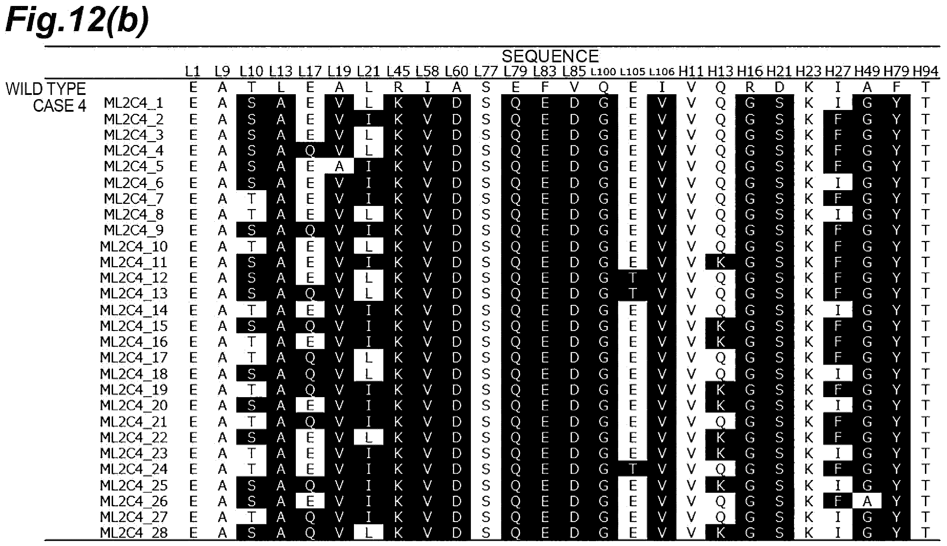

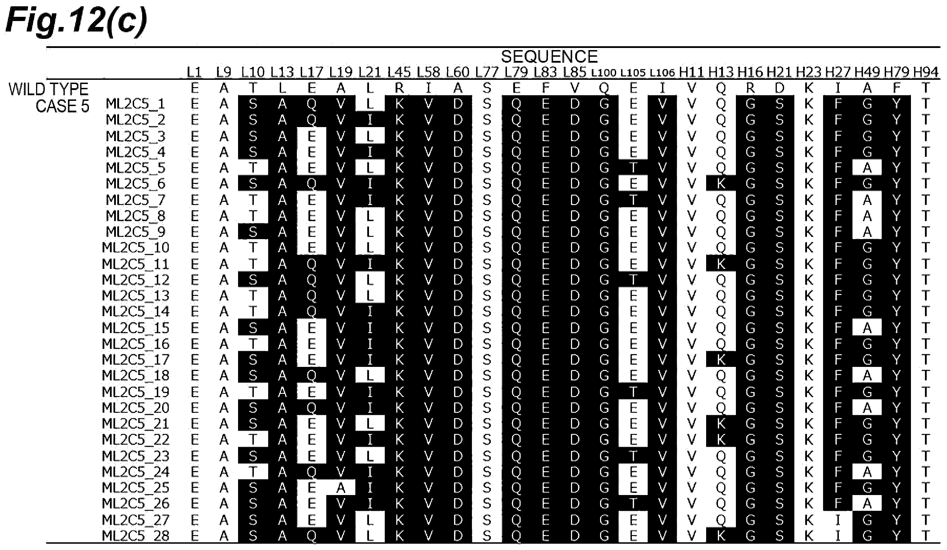

[23] An antibody Fv variant, wherein at least some of the amino acid residues at positions 1, 9, 10, 13, 17, 19, 21, 45, 58, 60, 77, 79, 83, 85, 100, 105, and 106 in the light chain amino acid sequence set forth in SEQ ID NO: 1, and the amino acid residues at positions 11, 13, 16, 21, 23, 27, 49, 79, and 94 in the heavy chain amino acid sequence set forth in SEQ ID NO: 2 are substituted, preferably an antibody Fv variant, wherein the amino acid residues are each substituted with the sets of the amino acids shown in Figure 12.

[24] An antibody Fv variant, wherein the amino acid residues at positions 21, 60, and 77 in the light chain amino acid sequence set forth in SEQ ID NO: 1 are substituted with isoleucine, aspartic acid, and glycine, respectively, and the amino acid residues at positions 16 and 23 in the heavy chain amino acid sequence set forth in SEQ ID NO: 2 are substituted with glycine and alanine, respectively.

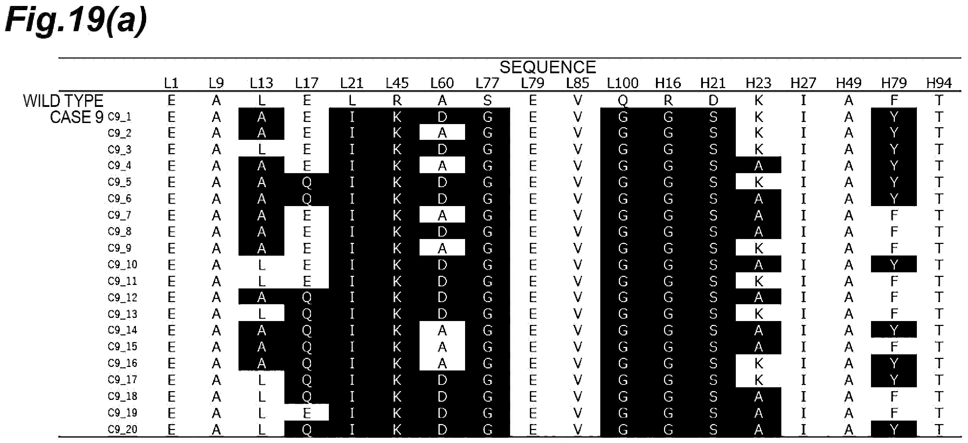

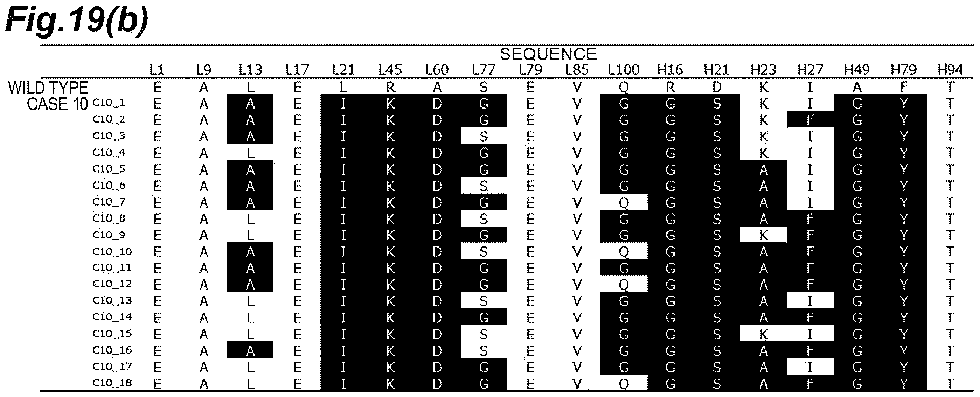

[25] An antibody Fv variant, wherein the amino acid residues at positions 1, 9, 10, 13, 17, 19, 21, 45, 58, 60, 77, 79, 83, 85, 100, 105, and 106 in the light chain amino acid sequence set forth in SEQ ID NO: 1, and the amino acid residues at positions 11, 13, 16, 21, 23, 27, 49, 79, and 94 in the heavy chain amino acid sequence set forth in SEQ ID NO: 2 are each substituted with the sets of the amino acids shown in Figure 19.

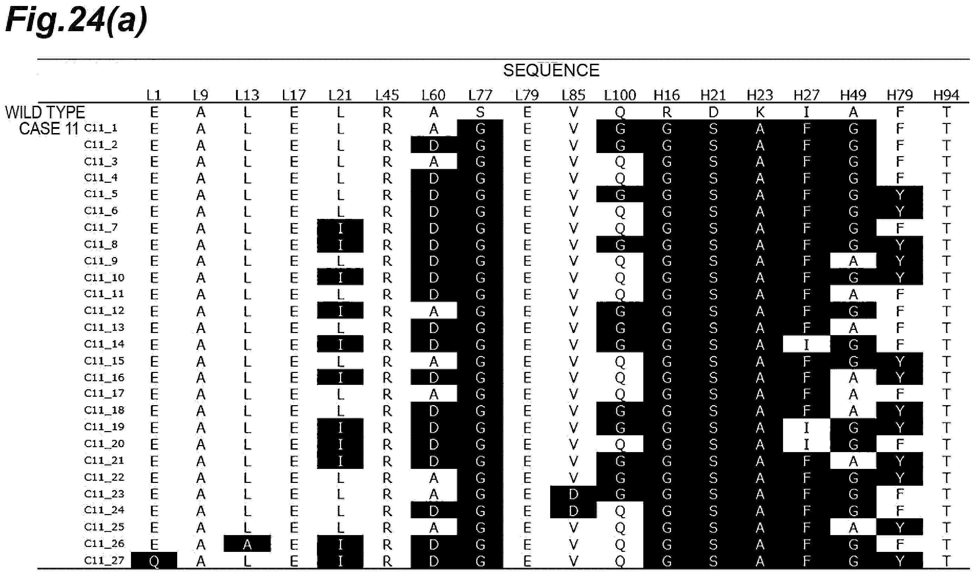

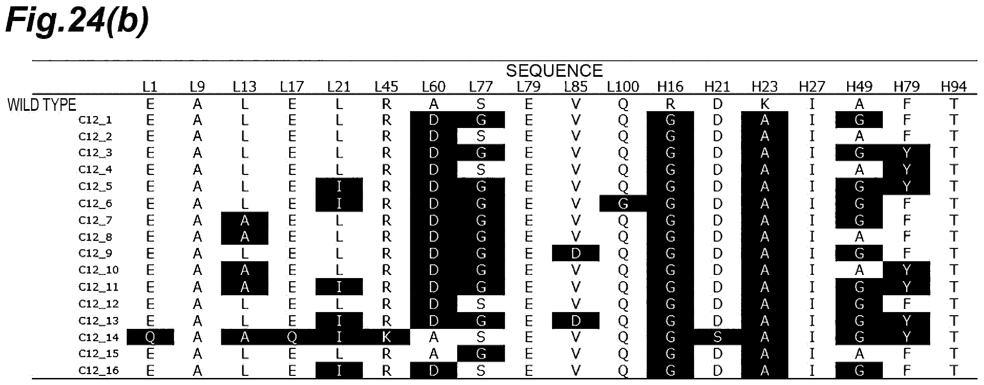

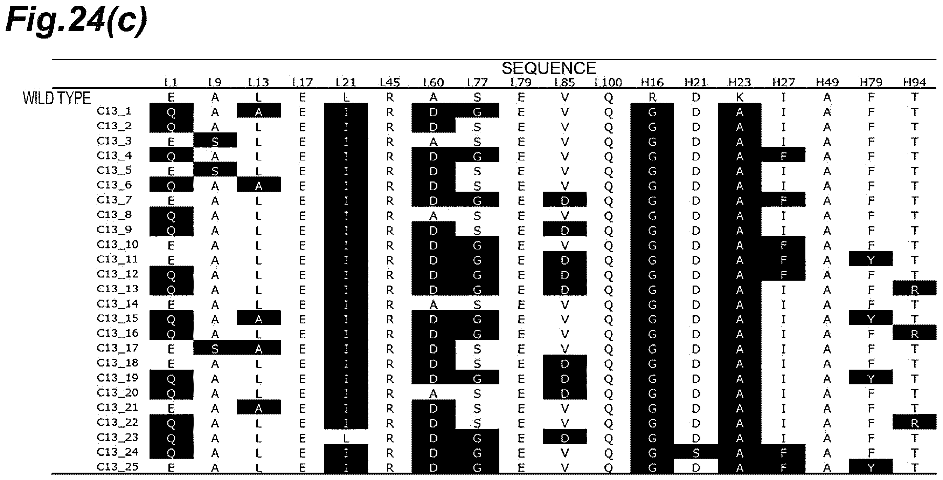

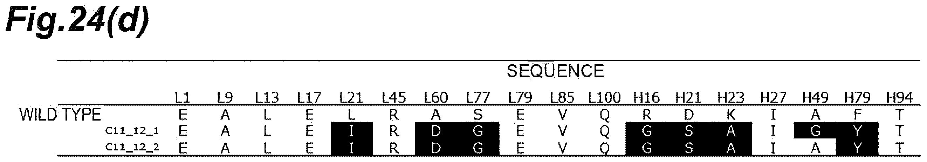

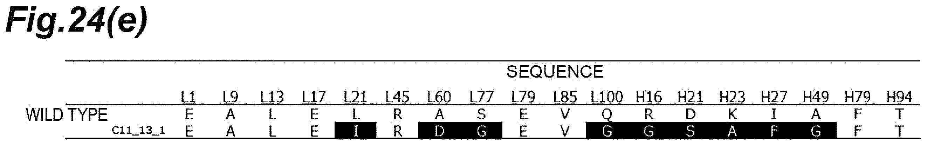

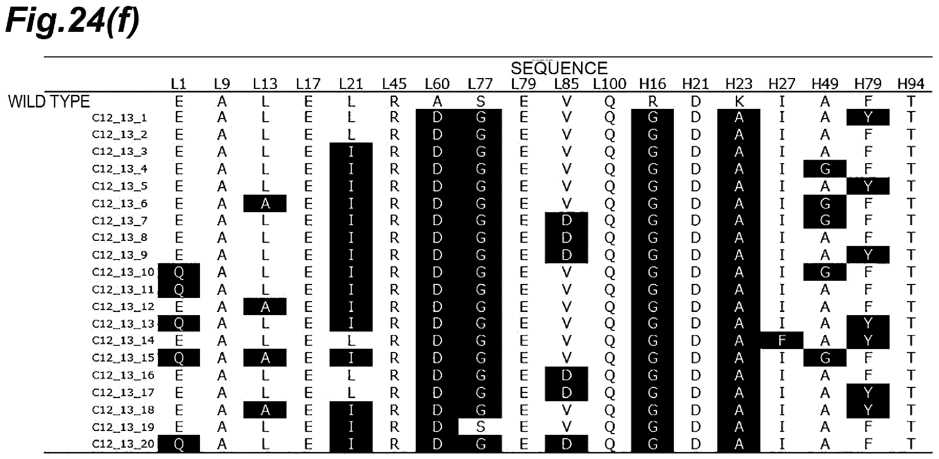

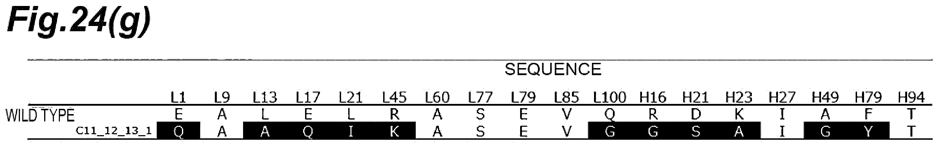

[26] An antibody Fv variant, wherein the amino acid residues at positions 1, 9, 10, 13, 17, 19, 21, 45, 58, 60, 77, 79, 83, 85, 100, 105, and 106 in the light chain amino acid sequence set forth in SEQ ID NO: 1, and the amino acid residues at positions 11, 13, 16, 21, 23, 27, 49, 79, and 94 in the heavy chain amino acid sequence set forth in SEQ ID NO: 2 are each substituted with the sets of the amino acids shown in Figure 24.

[27] An antibody Fv variant, wherein at least some of the amino acid residues at positions 1, 9, 10, 13, 17, 19, 21, 45, 58, 60, 77, 79, 83, 85, 100, 105, and 106 in the light chain amino acid sequence set forth in SEQ ID NO: 3, and the amino acid residues at positions 1, 5, 13, 24, 30, 49, 91, and 94 in the heavy chain amino acid sequence set forth in SEQ ID NO: 4 are substituted.

**Advantageous Effects of Invention**

According to the present invention, it is possible to efficiently improve functions of antibody proteins.

**Brief Description of Drawings**

[Figure 1] Figure 1 shows the amino acid sequence of Nivolumab (anti-PD-1 antibody).
[Figure 2] Figure 2 shows sites to be mutated during library production.
[Figure 3] Figure 3 shows the specific expression level, specific binding activity, and specific functional thermostability (heat treatment at 40°C) of mutants (O: wild type; ●: mutant).
[Figure 4] Figure 4 shows the specific expression level, specific binding activity, and specific functional thermostability (heat treatment at 50°C) of mutants (O: wild type; ■: mutant).
[Figure 5] Figure 5 shows the amino acid sequences of mutants produced with E. coli by using expression vectors for a mutant library produced to contain as many of the top 20 as possible of each case (a: the results that the machine learning had predicted by using training data scored with characteristic values obtained by heat treatment at 40°C were used; b: the results that the machine learning had predicted by using training data scored with characteristic values obtained by heat treatment at 50°C were used).

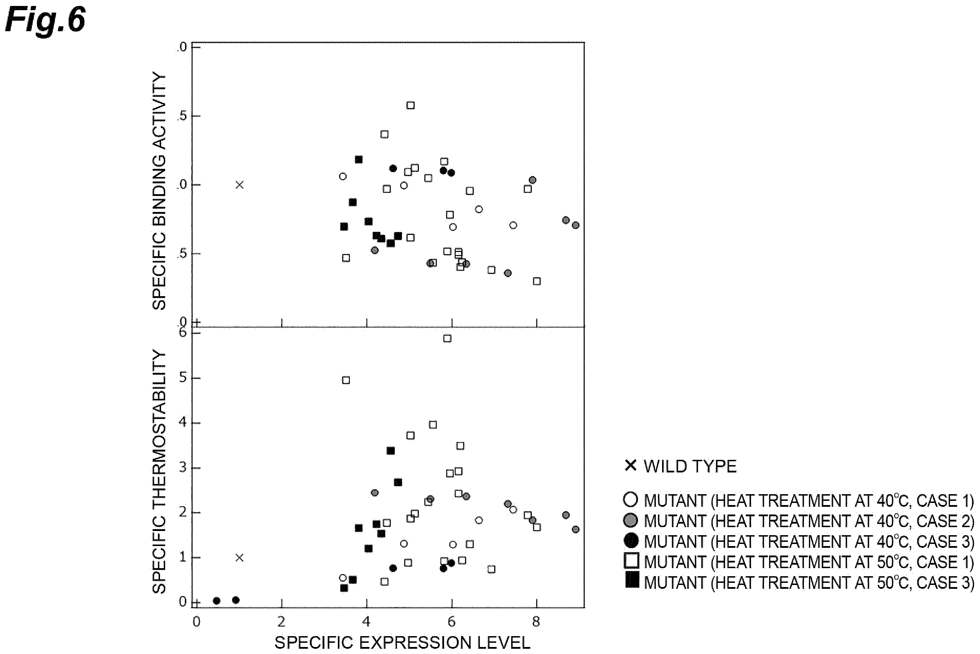

[Figure 6] Figure 6 shows the specific expression level, specific binding activity, and specific functional thermostability (heat treatment at 50°C) of mutants (×: wild type; O: mutant that the machine learning predicted by using training data scored with characteristic values obtained by heat treatment at 40°C in Case 1; O: mutant that the machine learning predicted by using training data scored with characteristic values obtained by heat treatment at 40°C in Case 3; ●: mutant that the machine learning predicted by using training data scored with characteristic values obtained by heat treatment at 40°C in Case 4; □: mutant that the machine learning predicted by using training data scored with characteristic values obtained by heat treatment at 50°C in Case 1; and ■: mutant that the machine learning predicted by using training data scored with characteristic values obtained by heat treatment at 50°C in Case 4).
[Figure 7] Figure 7 shows the amino acid sequences of the Top 5 mutants that the machine learning predicted by using training data scored with characteristic values obtained by heat treatment at 50°C.
[Figure 8A] Figure 8A shows the amounts prepared, thermal denaturation mid-points, dissociation equilibrium constants, and predicted immunogenicity of the Top 5 mutants that the machine learning predicted by using training data scored with characteristic values obtained by heat treatment at 50°C.

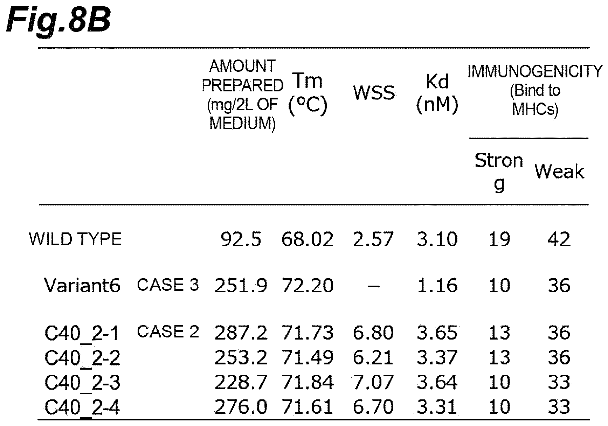

[Figure 8B] Figure 8B shows the amounts prepared, thermal denaturation mid-points, dissociation equilibrium constants (measured with Octet RED384 for Variant 6, and Biacore T200 for the others), and predicted immunogenicities of the full-length antibodies obtained using Fv of Variant 6 , which is the Top 1 mutant in Case 3 predicted by the machine learning using training data scored with characteristic values obtained by heat treatment at 50°C and Fv of the four mutants (C40_2-1, C40_2-2, C40_2-3, and C40_2-4) with significantly improved expression levels of scFv in Case 2. The predicted immunogenicity was predicted with the sequence of Fv alone.
[Figure 9] Figure 9 shows the results of a heat degradation test of respective full-length antibodies having Fv of the Top 1 mutant (Variant 6) that the machine learning predicted by using training data scored with characteristic values obtained by heat treatment at 50°C in Case 3, and the four mutants (C40_2-1, C40_2-2, C40_2-3, and C40_2-4) with significantly improved expression levels in Case 2.
[Figure 10A] Figure 10A shows the extraction of consensus sequences.
[Figure 10B] Figure 10B shows the amounts prepared, thermal denaturation mid-points, WSS values (for the full-length antibodies only), and dissociation equilibrium constants of the full-length antibodies and scFv into which mutations were introduced into consensus sequences (H16G, H21S, H79Y, L60D, and L85D) only.
[Figure 10C] Figure 10C shows the results of a heat degradation test of the full-length antibodies having Fv into which mutations were introduced into consensus sequences (H16G, H21S, H79Y, L60D, and L85D) only.
[Figure 10D] Figure 10D shows the amino acid sequences of the wild type and its mutants of Teprotumumab (anti-IGF-1R antibody) (a: wild type; b: mutant into which the consensus sequence in Figure 10A(4) was introduced; and c: mutant modified to the same framework sequence as that of C40_20-3 mutant in Figure 5(a)).
[Figure 10E] Figure 10E shows the amounts prepared, thermal denaturation mid-points, and EC$_{50}$ of Teprotumumab scFv mutants (consensus sequence: mutant into which the consensus sequence in Figure 10A(4) was introduced; and C40_2-3 mutant sequence: mutant that has been modified to have the same framework sequence as that of 5(a) C40_20-3 mutant).

[Figure 11] Figure 11 shows the specific expression level, specific binding activity, and specific thermostability (specific functional thermostability and specific structural thermostability) of mutants.

[Figure 12] Figure 12 shows the amino acid sequences of mutants produced with E. coli by using expression vectors for a mutant library produced to contain as many of the top 100 as possible of each case (a: Case 3; b: Case 4; and c: Case 5).

[Figure 13] Figure 13 shows the specific expression level, specific binding activity, and specific thermostability (specific functional thermostability and specific structural thermostability) of mutants that the machine learning proposed (×: wild type; ○: mutant that the machine learning predicted by using training data scored in Case 3; □: mutant that the machine learning predicted by using training data scored in Case 4; and △: mutant that the machine learning predicted by using training data scored in Case 5).

[Figure 14] Figure 14 shows the amino acid sequence of Daratumumab (anti-CD38 antibody).

[Figure 15] Figure 15 shows sites to be mutated during library production.

[Figure 16] Figure 16 shows the specific expression level, specific binding activity, specific functional thermostability (heat treatment at 60°C), specific structural thermostability, and specific functional thermostability (heat treatment at 55°C) of mutants.

[Figure 17] Figure 17 shows the specific expression level, specific binding activity, specific functional thermostability, and specific structural thermostability of mutants that the machine learning proposed.

[Figure 18] Figure 18 shows the specific expression level, specific binding activity, and specific solubility of Nivolumab scFv mutants (O: wild type; ●: mutant).

[Figure 19] Figure 19 shows the amino acid sequences of mutants produced with E. coli by expression vectors for a mutant library produced to contain as many of the top 100 as possible of each case (a: Case 9; and b: Case 10).

[Figure 20] Figure 20 shows the specific expression level, specific binding activity, and specific solubility of scFv mutants that the machine learning proposed (×: wild type; ○: Case 9; and □: Case 10).

[Figure 21] Figure 21 shows the amounts prepared of the full-length antibodies having Fv of the mutants predicted in Case 9.

[Figure 22] Figure 22 shows the solubility mid-points of the full-length antibodies having Fv of the mutants predicted in Case 9.

[Figure 23] Figure 23 shows the specific expression level, specific binding activity, specific solubility, and specific structural thermostability ($\Delta$Tm, specific WSS) of full-length antibody mutants.

[Figure 24] Figure 24 shows the amino acid sequences of Fv of mutants produced with Expi293F cells by using expression vectors for a mutant library of full-length antibodies produced to contain as many of the Top 100 as possible of each case (a: mutants not produced in Cases 12 and 13 among the mutants produced to contain as many of the top 100 as possible of Case 11; b: mutants not produced in Cases 11 and 13 among the mutants produced to contain as many of the top 100 as possible of Case 12; c: mutants not produced in Cases 11 and 12 among the mutants produced to contain as many of the top 100 as possible of Case 13; d: mutants also produced in Case 12 among the mutants produced to contain as many of the top 100 as possible of Case 11; e: mutants also produced in Case 13 among the mutants produced to contain as many of the top 100 as possible of Case 11; f: mutants also produced in Case 13 among the mutants produced to contain as many of the top 100 as possible of Case 12; and g: mutants also produced in Cases 12 and 13 among the mutants produced to contain as many of the top 100 as possible of Case 11).

[Figure 25] Figure 25 shows the specific expression level, specific binding activity, specific solubility, and specific structural thermostability of the full-length antibody mutants that the machine learning proposed (×: wild type; ○: Case 11 only; □: Case 12 only; △: Case 13 only; ●: Cases 11 and 12; ■: Cases 11 and 13, ▲: Cases 12 and 13, and ▼: Cases 11, 12, and 13).

[Figure 26] Figure 26 shows $EC_{50}$ of the full-length antibodies having Fv of the mutants predicted in Cases 11, 12, and 13.

[Figure 27] Figure 27 shows WSS values of the full-length antibodies having Fv of the mutants predicted in Cases 11, 12, and 13.

[Figure 28] Figure 28 shows the amount prepared of the full-length antibodies having Fv of the mutants predicted in Cases 11, 12, and 13.

[Figure 29] Figure 29 shows the solubility mid-points of the full-length antibodies having Fv of the mutants predicted in Cases 11, 12, and 13.

## Description of Embodiments

The present invention relates to a method of producing an antibody that is optimized for a plurality of characteristics including at least two of an expression level, binding activity, stability, and solubility, and a method of producing a library consisting of proteins each of which are optimized for the characteristics. Hereinafter, terms and each procedure of the present invention will be described.

1. Production of Initial Library (First Library)

A library consisting of mutants prepared by modifying at least some of residues not being an amino acid of the highest appearance frequency in a group of antibody sequences of a target antibody, into the amino acid of the highest appearance frequency is provided. As used herein, this big library that is provided at the beginning is referred to as "initial library" or "first library" in order to distinguish it from a library after enrichment, which will be described later. The "initial library" and "first library" are interchangeably used herein.

The "target antibody" is not particularly limited, and may be a full-length antibody, an antibody variable region fragment (Fv) alone, a single-chain variable region fragment (scFv), a recombinant antibody obtained by combining a plurality of Fv such as tandem scFv, or a recombinant antibody obtained by combining Fv with an Fc fragment or other proteins. The origin of the antibody is also not particularly limited, and may be a human antibody, non-human antibody such as mouse antibody, humanized antibody, or chimeric antibody.

For example, in the case of the antibody variable region fragment Fv, its binding activity is reduced through modification of a residue position in the framework region (FR) of Fv that uses an amino acid of a lower appearance frequency in an antibody sequence database, into an amino acid of a higher frequency.

For mutagenesis, techniques known in the art can be used such as an error-prone PCR method, a random primer method, an overlap extension PCR method, an inverse PCR method, DNA shuffling, a staggered PCR method, a Kunkel method, a quick change method. Commercially available mutagenesis kits can also be used.

The size of the library is not particularly limited and is appropriately determined according to the number of mutation sites. As there are 20 natural amino acids, when the mutation sites are on 3 residues, for example, the size is $20^3$ and about 8,000, when 4 residues, the size is $20^4$ and about 160,000. In addition, when the number of amino acids appeared is 2, and the mutation sites are on 20 residues, then, the size is $2^{20}$ and about 1,000,000, when the number of amino acids appeared is 3, and the mutation sites are on 15 residues, then, the size is $3^{15}$ and about 15,000,000.

2. Assessment and Scoring of Plurality of Characteristics Including Expression Level, Binding Activity, Stability, and Solubility

Next, a plurality of characteristics including the expression level, binding activity, stability, and solubility of some of mutants in the library are assessed. The number of mutants for which characteristics are assessed is not particularly limited as long as training data meaningful to artificial intelligence can be provided. Preferably data of 100 or more, more preferably 100 to 200 are provided.

The "plurality of characteristics" include at least two of the expression level, binding activity, stability (functional thermostability, structural thermostability, pH stability, salt stability, pressure stability, reduction stability, and denaturant stability), and solubility. The "functional thermostability" means stability of function (e.g., binding affinity) against heat, while the "structural thermostability" means stability of structure against heat. The functional thermostability is assessed by, for example, ELISA after heat treatment, while the structural thermostability is assessed by, for example, a Tm value, WSS value, or heat degradation test. Examples of the "plurality of characteristics" include, in addition to these characteristics, biological activity, affinity (binding activity), target specificity, catalytic activity, substrate specificity, aggregability, protease resistance, cytotoxicity, enzyme inhibitory activity, antibacterial activity, signal inhibitory activity, regulatory factor inhibitory activity, and immunogenicity.

The characteristics of respective mutants are measured and/or assessed as numerical values (characteristic values) based on different measurement data related to respective characteristics. For example, respective characteristic values of mutants are assessed as relative values to the target value or standardized values as appropriate. Specifically, characteristic values are assessed as a ratio or difference (relative value) relative to the characteristic values that a wild type of the target protein (target protein before mutagenesis) or a protein to be compared possesses or as a standardized value thereof. The protein to be compared may be any protein having a "characteristic to be targeted" or "characteristic to be exceeded". For example, in the case of expression level, binding activity, stability, and solubility, the characteristic values are each assessed as specific expression level, specific binding activity, specific stability, and specific solubility relative to the expression level, binding activity, stability, and solubility of a wild-type protein or a protein to be compared, or as standardized expression level, standardized binding activity, standardized stability, and standardized solubility.

In the present invention, the two or more characteristics can be simultaneously optimized by normalizing and integrating the plurality of characteristic values, and scoring the values as one value per mutant.

As used herein, the term "normalization" means unification of the scales of characteristic values that vary in scale (size, unit, or the like). As mentioned above, a plurality of different functions and physical properties based on different measurement data are completely different in size, unit and variance, which are often contradictory to each other. When scoring, it is preferable to normalize respective characteristic values to make their "weight" equal. Methods for normalization are well-known in the art, and are performed by selecting appropriate functions according to the respective characteristic values and distribution thereof. It is preferable that the numerical values after normalization be scaled from 0 to 1 (0 to -1) for every characteristic. The normalized characteristic values are scored as one value by integration.

The scoring is performed for example according to the following formula.

Score value = $f$(1st characteristic value - reference value of 1st characteristic) $\times$ $f$(2nd characteristic value - reference value of 2nd characteristic)... $\times$ f(nth characteristic value - reference value of nth characteristics)　　　[Formula (1)]

In the above formula, n is the number of characteristics to be optimized, and for example, the first characteristic is the expression level, the second characteristic is the binding activity, and the third characteristic is stability.

As the function "f(x)", a sigmoid function (x), a Gaussian function (x) (e.g., normal distribution function), a lognormal distribution function (x), a ReLU function (x), a linear function (x), an n-dimensional function (x), an exponential function (x), a logarithmic function (x), a trigonometric function (x), a hyperbolic function (x) (e.g., hyperbolic tangent function), and a combination thereof can be used.

The term "reference value" means a value that the characteristic value should exceed or should be targeted (minimum required characteristic value or target value).

If it is optimal when the characteristic values are directed to a value higher than the reference value, the reference value is the "minimum required characteristic value". For example, when aiming for a characteristic value higher than that of the wild type, the characteristic value of the wild type becomes the reference value. The function for normalization is appropriately selected according to the possible values for the characteristic value. For example, when the rate of change of the characteristic value is maximized near the reference value, the characteristic value can be normalized using the functions such as sigmoid function, exponential function, n-dimensional function (n = odd number), logarithmic function, hyperbolic tangent function, or the like. On the other hand, when the characteristic value equal to or lower than the reference value, the characteristic value is constant, and in the case where the characteristic value increases when exceeding the reference value, the characteristic value can be normalized using ReLU function or the like.

If it is optimal when the characteristic values are directed to a certain target value, the reference value is the target value. For example, when aiming to obtain a mutant exhibiting X times the characteristics of the wild type, the characteristic value (target value) that is X times the wild type becomes the reference value. The function for normalization is appropriately selected according to the possible values for the characteristic value. For example, when the characteristic value exhibits the maximum value near the target value, the characteristic value can be normalized using the functions such as Gaussian function (normal distribution function), lognormal distribution function, n-dimensional function (n = even number), hyperbolic function, or the like.

3. Machine Learning by Score Values

Machine learning is conducted by using the score values as training data and ranking the library. In other words, artificial intelligence is trained on score values obtained for some of the mutants in the library and corresponding sequence information on the mutants to predict scores for all mutants in the library and rank the scores.

Amino acid sequence information is input by converting characters into numerical values (numerical vectors). Examples of such a method include a method known in the art, and T-scale, Z-scale, ST-scale, BLOSUM, FASGAI, MSWHIM, ProtFP, ProtFP-Feature, VHSE, Aromaphilicity, PSSM, and the like can be used (van Westen et al., J Cheminform. 2013; 5: 41).

Machine learning can be conducted by Bayesian linear regression, linear regression, Gaussian process regression, logistic regression, decision tree, simple perceptron, multilayer perceptron, neural network, deep neural network, k-nearest neighbor algorithm, and support vector machine. Among others, Bayesian linear regression is preferable.

The decision tree is an algorithm that has a hierarchical tree structure composed of a plurality of conditional branches. Ensemble learning by combining two or more decision trees includes random forest, gradient boosting decision tree, and the like.

The "neural network" is an algorithm to which arbitrary function is applied to activation functions of multilayer perceptron. Examples of arbitrary functions include a sigmoid function, hyperbolic tangent function, ReLU function, and the like.

The "deep neural network" is an algorithm having two or more hidden layers of a neural network, and is also referred to as deep learning. Application examples thereof include a convolutional neural network, recurrent neural network, LSTM, GRU, and the like.

The "Gaussian process regression" is a regression approach using a stochastic process on the assumption that the joint probability distribution is Gaussian distribution, and is one of the machine learning approaches that determine the optimal values (maximum value or minimum value) of an unknown function (black-box function). However, as the number of dimensions of input increases, the amount of calculation increases exponentially, and therefore, a bigger number of dimensions thereof causes the calculation to be practically difficult. On the other hand, the "Bayesian linear regression" is a regression approach using a linear function, and is a machine learning approach that can determine the optimal values (maximum value or minimum value) of an unknown function (black-box function) the same as Gaussian process regression while it can handle a bigger number of dimensions. Each candidate point is represented as a numerical vector called a descriptor, a machine learning model is trained using data of the candidate points previously assessed, and using the trained model, predicted values and predicted variance of the model functions for the remaining candidate points are calculated.

The "Bayesian optimization" is to use a machine learning model constructed by a regression using a Bayesian estimation such as Gaussian process regression and Bayesian linear regression, to calculate scores depending on predicted values and predicted variance, and thereby to set the candidate point with the biggest score as a next assessment point to repeat the function evaluation. The new data obtained here is added to the training data.

For the "Bayesian optimization", known software can be used. Known examples thereof include 2DMAT (https://www.pasums.issp.u-tokyo.ac.jp/2dmat/) COMmon Bayesian Optimization Library (COMBO) (Ueno et al., Mater. Discov., 4, 18-21 (2016), https://github.com/tsudalab/combo), CrySPY (https://tomoki-yamashita.github.io/CrySPY_doc/), PHYSBO (optimization tools for PHYsics based on Bayesian Optimization) (https://www.pasums.issp.u-tokyo.ac.jp/physbo/), and the like, but not limited thereto. Among others, COMBO and PHYSBO are preferable.

4. Production of Second Library

By machine learning using data of some of the mutants, artificial intelligence predicts score values obtained for all the mutants in the library and ranks them. By selecting suitable mutants based on the prediction results, a new library enriched from the initial library can be produced. This new library as used herein is referred to as "second library". The "second library" is an enriched library composed of mutants more suitable for a plurality of characteristics including at least two of the expression level, binding activity, stability, and solubility.

The enrichment of the library may be performed two or more times, if necessary. In other words, a second library is produced from the initial library, and then the obtained second library is used as an initial library to produce a third library. Enrichment can be performed as many times as possible by repeating this process.

5. Determination of Protein that is optimized for Plurality of Characteristics Including At Least Two of Expression Level, Binding Activity, Stability, and Solubility

By functional prediction through machine learning, it is possible to select mutants that are optimized for the plurality of characteristics, from the initial library, or the second, third or subsequent libraries. The "optimized" mutants include mutants for which at least one characteristic is improved over the wild type, and other characteristics are equal to or better than the wild type. The predicted mutants are actually expressed, and their characteristics are assessed and confirmed to select the best ones. For the consideration of industrial applicability, it is generally preferable that the number of mutation sites be small. Therefore, the optimal proteins (mutants) are ultimately determined in consideration of functional improvement and the number of mutations introduced.

6. Optimized Protein

The present invention also provides a protein variant that is optimized by the method of the present invention. For example, the present invention provides an antibody Fv variant, in which at least some of the amino acid residues at positions 1, 9, 13, 17, 21, 45, 60, 77, 85, and 100 in a light chain amino acid sequence set forth in SEQ ID NO: 1, and the amino acid residues at positions 16, 21, 23, 27, 49, 79, and 94 in a heavy chain set forth in SEQ ID NO: 2 are substituted. Preferably, the present invention provides an Fv variant, in which the amino acid residues are each substituted with any of the amino acid sets shown in Figures 5 and 7. These Fv variants are superior in expression level and stability (functional thermostability) to Fv of known anti-PD-1 antibodies, which are the parent antibodies, while maintaining the binding activity.

The present invention also provides an antibody Fv variant, in which at least some of the amino acid residues at positions 1, 9, 10, 13, 17, 19, 21, 45, 58, 60, 77, 79, 83, 85, 100, 105, and 106 in the light chain amino acid sequence set forth in SEQ ID NO: 1, and the amino acid residues at positions 11, 13, 16, 21, 23, 27, 49, 79, and 94 in the heavy chain set forth in SEQ ID NO: 2 are substituted. Preferably, the present invention provides an Fv variant, in which the amino acid residues are each substituted with any of the amino acid sets shown in Figure 12. These Fv variants are superior in expression level and stability (functional thermostability) to Fv of known anti-PD-1 antibodies, which are the parent antibodies, while maintaining the binding activity.

The present invention also provides an antibody Fv variants, in which at least some of the amino acid residues at positions 1, 9, 10, 13, 17, 19, 21, 45, 58, 60, 77, 79, 83, 85, 100, 105, and 106 in the light chain amino acid sequence set forth in SEQ ID NO: 3, and the amino acid residues at positions 1, 5, 13, 24, 30, 49, 91, and 94 in the heavy chain set forth in SEQ ID NO: 4 are substituted. These Fv variants are superior in expression level and stability (functional thermostability) to Fv of known anti-CD38 antibodies, which are the parent antibodies, while maintaining the binding activity.

**Examples**

Hereinafter, the present invention will be described in more detail by reference to Examples; but the present invention is not limited to these Examples.

Example 1: Maturation of Anti-PD-1 Human Antibody Variable Region Fragment

In a maturation process of an antibody, target affinity, stability, immunogenicity, solubility, and the like are improved, but the functions and physical properties thereof are often contradictory to each other. In this example, simultaneous optimization of the expression level, binding activity, and thermostability (mainly functional thermostability) was attempted for the variable region fragment (Fv) of the anti-PD-1 human antibody Nivolumab.

1.1 Modification of Fv

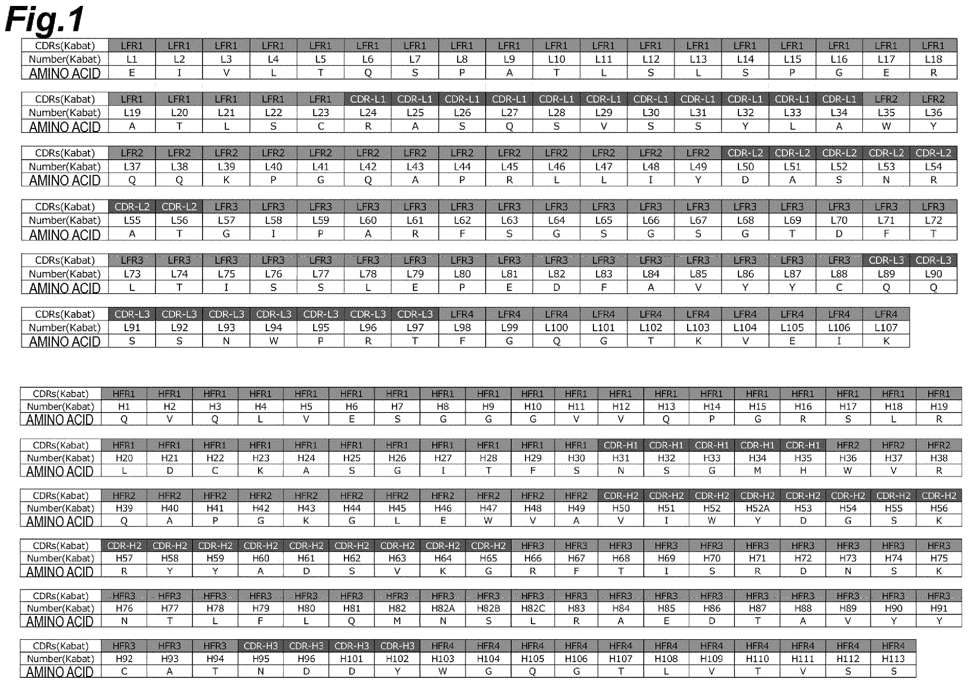

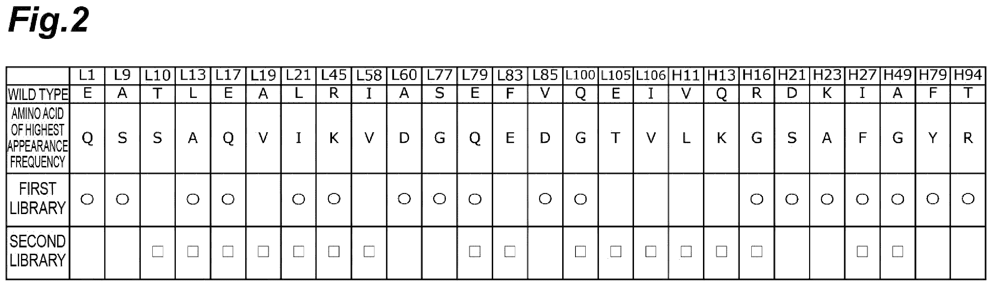

A sequence alignment of Fv of Nivolumab (light chain: SEQ ID NO: 1; heavy chain: SEQ ID NO: 2, Figure 1) was performed using the antibody database abYsis (Swindells, M. B. et al. J. Mol. Biol. 429, 356-364 (2017)) to calculate the appearance frequency of amino acids at respective residue positions in a human antibody. Then, the Fv was compared with the amino acid sequence, and in order to produce a mutant library in which the original amino acid (wild type) or amino acids of high appearance frequencies appear at residue positions different from those of the amino acids of high appearance frequency, mutants (89 mutants) were produced by defining the 18 residue positions (O marks in Figure 2) among the mutation residue positions as mutation library introduction sites.

The mutants were expressed as scFv in E. coli, the expression level was assessed by Western blotting, and the binding activity and functional thermostability were assessed by ELISA. Specifically, E. coli transformed with respective expression vectors containing a gene fragment having an FL Western blotting tag at the C-terminus of the wild-type and respective mutants was cultured, and then, the resulting medium supernatant fractions were used to assess the expression level by Western blotting, and the binding activity and functional thermostability were assessed by ELISA. The expression level was assessed by the band intensity stained with an anti-FLAG tag antibody after performing electrophoresis using Wes (ProteinSimple, Inc.). The binding activity was assessed by values that were obtained by adding a medium supernatant on a plate immobilized with a PD-1 protein, which was a target protein, and washing, followed by measuring the amount of each mutant remained on the plate using an HRP-conjugated anti-FLAG tag antibody, and standardizing the measured values with the assessed values of the expression level. The functional thermostability was assessed by values that were obtained by adding a medium supernatant that was heat treated at 40°C or 50°C for 1 hour, on a plate immobilized with a PD-1 protein, measuring the amount of each mutant remained on the plate using an HRP-conjugated anti-FLAG tag antibody, and standardizing the measured values with the assessed values of the binding activity. 80 mutants were measured and assessed when heat-treated at 40°C, and 90 mutants when treated at 50°C (both including the wild type).

It was suggested that the expression level as the soluble fraction may be correlated with aggregation suppressing properties (high solubility rate) and structural thermostability (thermal denaturation mid-point Tm) (Niwa et al., PNAS March 17, 2009 106 (11) 4201-4206; and Ito et al., Chemistry Letters, (2021) 50, 1867-187), and the expression level may be an indicator of not only the amount produced as a simple soluble fraction but also physical properties of aggregation suppressing properties and structural thermostability (thermal denaturation mid-point Tm).

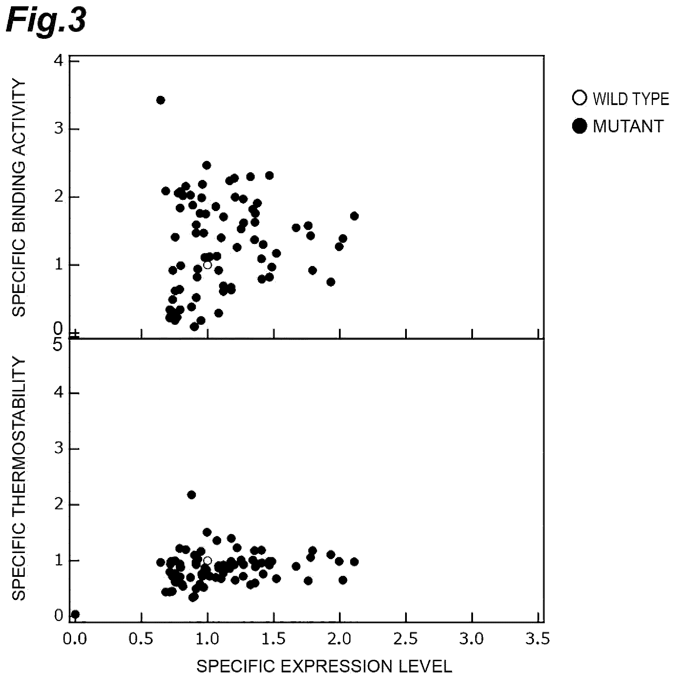

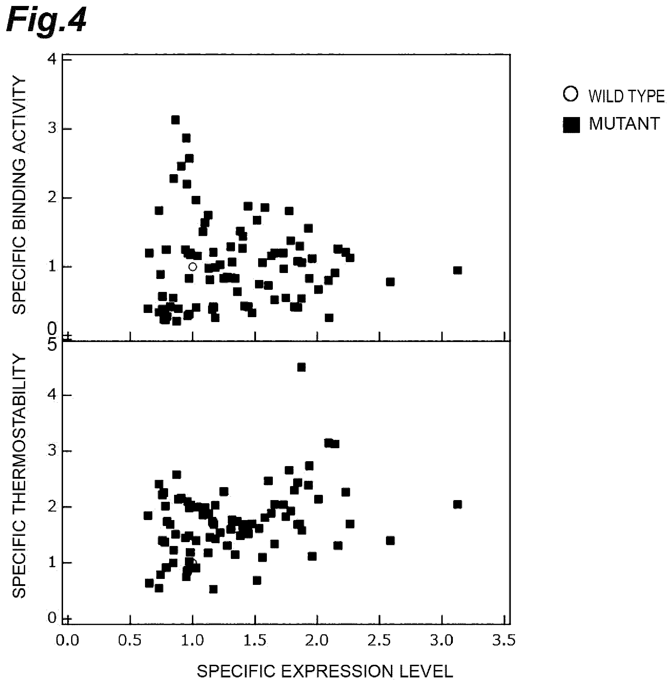

The expression level, binding activity, and functional thermostability of each mutant were determined as a ratio (specific expression level, specific binding activity, and specific functional thermostability) relative to the assessed values of the expression level, binding activity, and functional thermostability of the wild type before mutation, respectively. Plotting the specific expression level and specific binding activity, and specific expression level and specific functional thermostability revealed that there were a certain number of mutants in which both the expression level and functional thermostability were improved compared to the wild type without reducing the target binding properties (Figure 3: heat treatment at 40°C; Figure 4: heat treatment at 50°C).

1.2 Production of Prediction System through Machine Learning

The above data was used as training data to conduct machine learning in which the functional assessment values of unknown mutants were predicted from the amino acid sequences. A prediction system was produced by Bayesian linear regression using COMBO, which is highspeed Bayesian Optimization software (e.g., Ueno et al., 2016, supra). The sequence data of the mutants were represented by using an adequate indicator or combination thereof among those expressed as 1 to 10-dimensional vector per residue according to the known reports. (van Westen et al., 2013, supra).

The three characteristic values (specific expression level, specific binding activity, and specific functional thermostability) were scored as one value by the following 3 methods.

Case 1

Score value = f1(specific expression level - 1) $\times$ f2(specific binding activity - 1) $\times$ f3(specific functional thermostability - 1)

f1(x) = sigmoid (x)
f2(x) = sigmoid (x)
f3(x) = sigmoid (x)

Case 2

Score value = f1(specific expression level - 1) $\times$ f2(specific binding activity) $\times$ f3(specific functional thermostability)

f1(x) = sigmoid (x)
f2(x) = gaussian (x), $\mu$ = 1, $\sigma$ = 0.5
f3(x) = gaussian (x), $\mu$ = 1.13, $\sigma$ = 0.3

Case 3

Score value = f1(specific expression level - 1) $\times$ f2(specific binding activity - 1)

f1(x) = sigmoid (x)
f2(x) = sigmoid (x)

The sigmoid function was used when conducting machine learning so that score values were high when the characteristic values were higher than the reference value. In addition, when forming an aggregate, ELISA intensity sometimes showed a high value, and to correct this, a Gaussian function was used when conducting machine learning as a certain value being the optimal value.

In Case 1, the sigmoid function was used for all for training for evolution such that the specific expression level, specific binding activity, and specific functional thermostability become higher than the reference values (multiple number of standardized values when the specific expression level, specific binding activity, and specific functional thermostability of the wild type are 1: 1 $\times$ 1 for all in the case of the specific expression level, specific binding activity, and specific functional thermostability).

In Case 2, the sigmoid function was used for the specific expression level, and the Gaussian function was used for the specific binding activity and specific functional thermostability for training for evolution such that the specific expression level is higher than the reference value, and the optimal values of the specific binding activity and specific functional thermostability are 1 time and 1.13 times the reference values, respectively.

In Case 3, the sigmoid function was used for the specific expression level for training for evolution such that the specific expression level and specific binding activity are higher than the reference values. The specific functional thermostability was not set as a target for training.

1.3 Selection of Promising Mutant by Prediction System

For the construction of prediction systems, the dataset of the heat treatment at 40°C and the dataset of the heat treatment at 50°C were separately treated, and prediction systems were constructed for each. For the dataset of the heat treatment at 40°C, Cases 1, 2, and 3 were performed, and for the dataset of the heat treatment at 50°C, Cases 1 and 3 were performed. Using the constructed prediction system, predicted score values of all mutants (excluding 79 mutants which were already measured as training data and wild type for heat treatment at 40°C, and 89 mutants which were already measured as training data and wild type for heat treatment at 50°C) contained in the sequence space formed by 18 residues (○ marks in Figure 2) were calculated. Among those, the design of library genes such that they contain as many of the Top 20 as possible of each case was performed by selecting a similar template sequence from the training data and using degenerate codons to produce a mutant library (second library). The designed genes were inserted into an E. coli-expression vector, and using the medium supernatant in which mutants were expressed according to 1.1, the specific expression level was measured with Western blotting, and the specific binding activity and specific functional thermostability (heat treatment at 50°C) were measured with ELISA. As a result, it was possible to obtain mutants in which the expression level and the thermostability were improved compared to mutants in the training data, without reducing the binding activity (Figures 5 and 6).

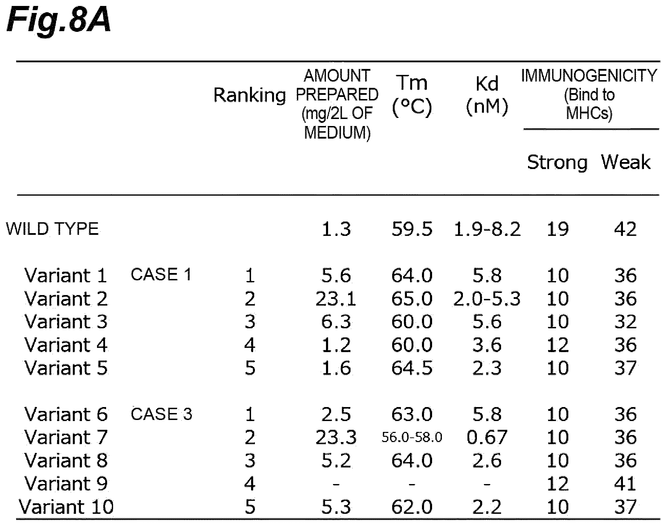

The mutants predicted as the Top 5 by the prediction system constructed using the dataset of the heat treatment at 50°C (Figure 7: Variants 1 to 10) were prepared in large quantities in E. coli, purified by size-exclusion chromatography (SEC) after immobilized metal affinity chromatography (IMAC) purification, and then the amount prepared per medium at SEC, the dissociation equilibrium constant by Biacore T200, and the thermal denaturation mid-point (Tm value) by the thermal shift assay method were calculated (Figure 8A). The Tm value indicates the temperatures at which 50% of the conformation is denatured, and serves as an indicator of the structural thermostability. As a result, for all the mutants, the dissociation equilibrium constants were almost unchanged, and the amounts prepared were improved compared to the wild type, and in addition to that, the Tm values were improved, indicating that not only the functional thermostability but also the structural thermostability were improved. Further, the immunogenicity was also assessed in terms of affinity with human major histocompatibility complex (MHC) class II molecules. Using MHC class II Binding Predictions provided by Immune Epitope Database (IEDB) (http://tools.immuneepitope.org/analyze/html/mhc_II_binding.html), the immunogenicity was predicted from the binding properties of the peptide sequence fragmented from Fv to the major histocompatibility complex (MHC) class II, indicating that both the number of peptide fragments that had been predicted to have strong binding and the number of peptide fragments that had been predicted to have weak binding were smaller values than those of the wild type.

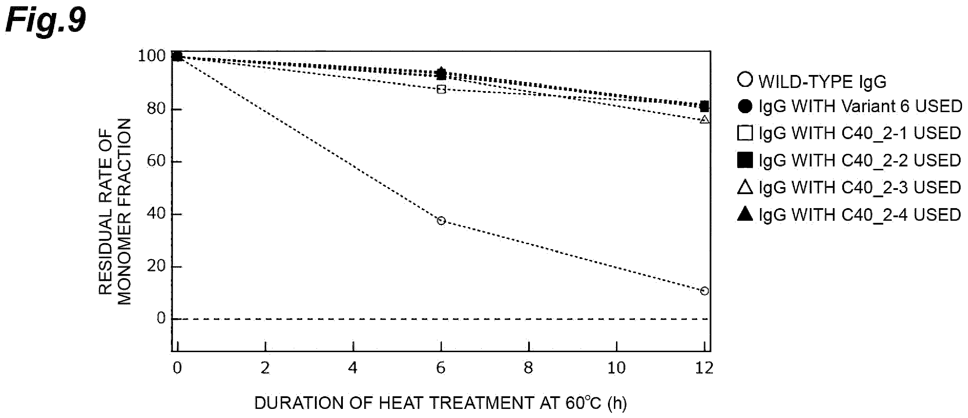

Among these mutants, the Top 1 mutant (Variant 6) in Case 3 and four mutants (C40_2-1, C40_2-2, C40_2-3, and C40_2-4) from Case 3 in which the expression levels in scFv were significantly improved, were prepared in Expi293F cells as full-length antibodies IgG, which indicated that the binding activity was equivalent to that of the wild type, whereas the amounts prepared were improved by 2.7 to 3.1 times (Figure 8B). For the structural thermostability, not only the measurement of Tm values by differential scanning calorimetry but also the calculation of values of stabilized score, weighted shoulder score (WSS), which was an indicator of the differential fluorescence at a measurement temperature higher than the Tm values, were performed (Kerwin et al. J Pharm Sci. 2020 Jan; 109(1):233-246). As a result, the Tm values were improved by 3.4 to 3.8°C, and WSS was improved by 2.4 to 2.8 times (Figure 8B). In addition, the heat degradation test revealed that the structural thermostability was improved to the point where no aggregation was observed when treated at 60°C (Figure 9).

1.4 Rule Extraction of Appeared Amino Acid

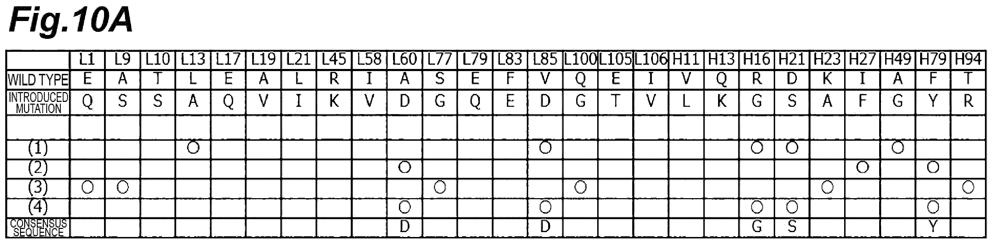

In order to extract the mutation residue positions that affect the characteristic values, the produced training data and the data of the mutant group produced according to the machine learning prediction (ML data) were used to separate wild-type amino acids and mutated amino acids, and the significant difference was determined by U-test. As a result, the positions where the introduced amino acids were superior in both the expression level and the functional thermostability were L13, L85, H16, H21, and H49 ((1) in Figure 10A), the positions where the introduced amino acids were superior in the expression level or the functional thermostability were L60, H27, and H79 ((2) in Figure 10A), and the positions where the wild type was superior in the expression level or the functional thermostability were L1, L9, L100, L77, H23, and H94 ((3) in Figure 10A). Further, for all the combinations of the residue positions of (1) and (2) in Figure 10A, an observed frequency table was created and the significant difference was verified by chi-square test; for "training data only" and "data obtained by combining training data and ML data", when the percentage of the number of mutants with characteristic values equal to or higher than those of the wild type was calculated, those with a high percentage of the number of mutants with characteristic values equal to or higher than those of the wild type and with a change of less than 10% in the "data obtained by combining training data and ML data" to the "training data only" were selected as combinations that improve their functions due to the mutation and have a weaker tendency toward function improvement. The averages of the expression level and functional thermostability were calculated for the mutant groups having the combinations of each mutant, and the combinations with the highest function were selected as consensus sequence candidates ((4) in Figure 10A).

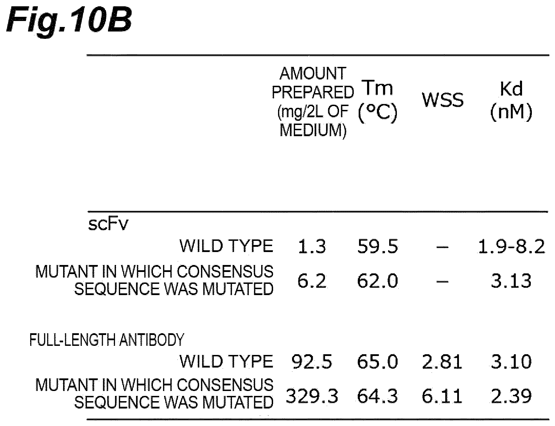

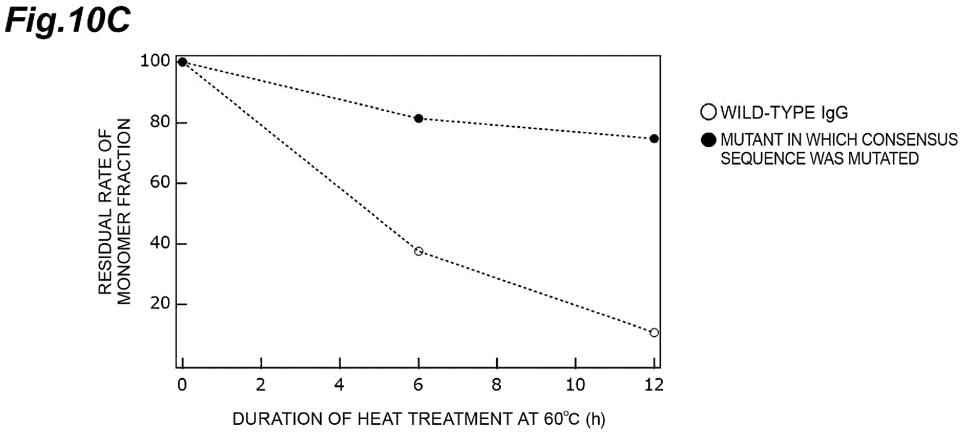

When the mutants in which mutations were introduced only into the consensus sequences (H16G, H21S, H79Y, L60D, and L85D) were prepared as scFv in large quantities in E. coli, the binding activity was equivalent to that of the wild type; whereas the amount prepared was increased by 4.8 times, and the Tm value calculated by the thermal shift assay method was improved by 2.5°C (Figure 10B). Further, when the mutants were prepared in Expi293F cells as full-length antibodies IgG, the binding activity was equivalent to that of the wild type, whereas the amount prepared was improved by 3.6 times. For the structural thermostability, the Tm value calculated by the thermal shift assay method was equivalent to that of the wild type, whereas the WSS value was improved by 2.2 times (Figure 10B). In addition, the heat degradation test revealed that the structural thermostability was improved to the point where no aggregation was observed when treated at 60°C (Figure 10C).

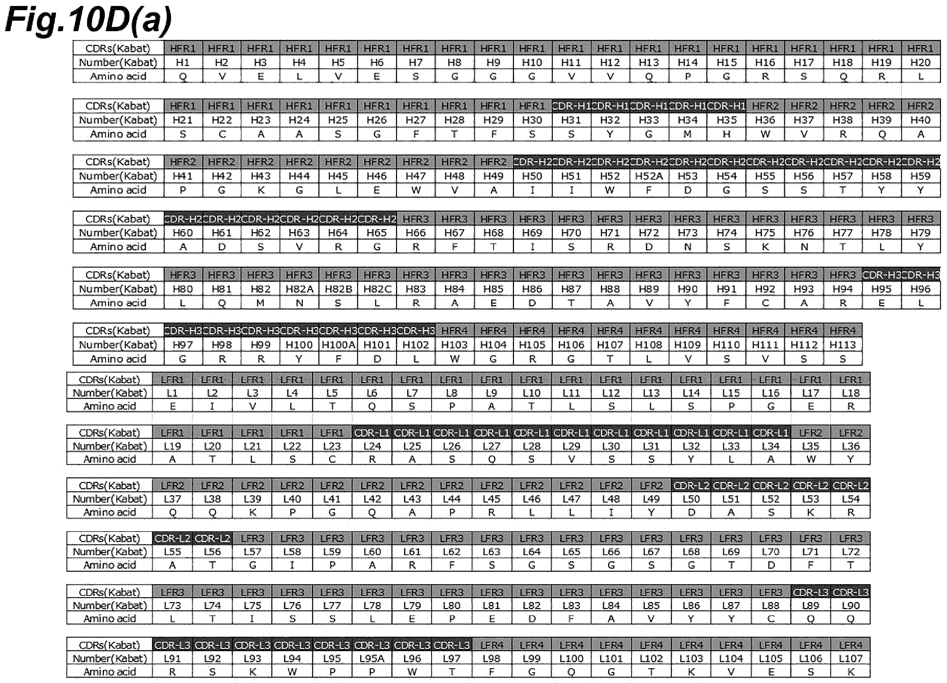

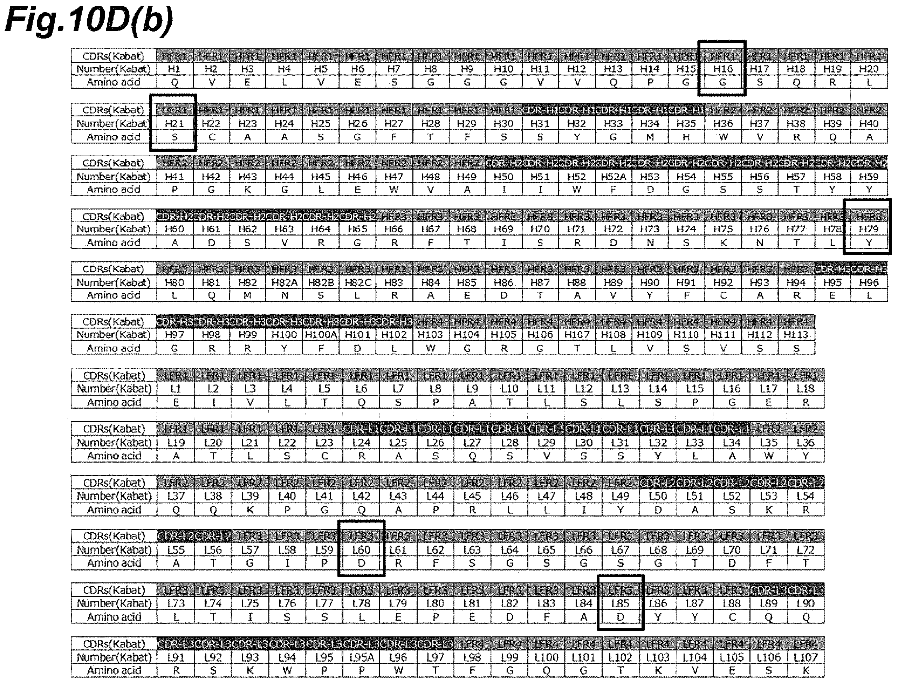

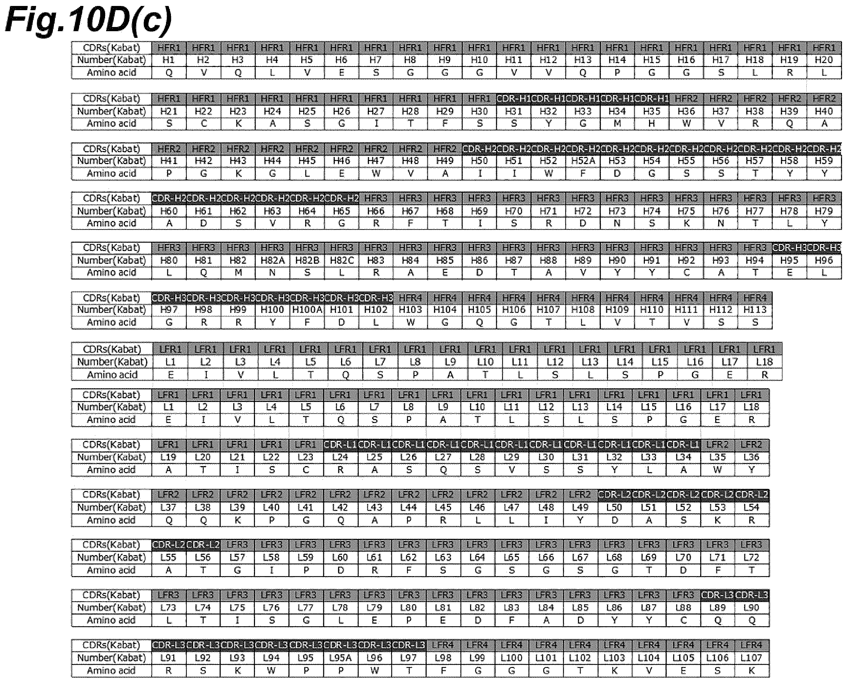

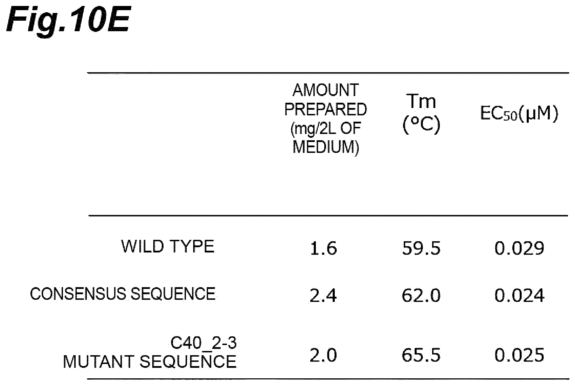

Also, scFv obtained by introducing only the consensus sequences described above and the same framework sequence as the C40_2-3 mutant into Fv of an anti-insulin-like growth factor-1 receptor (IGF-1R) antibody Teprotumumab, which was in the same germline as Nivolumab (H: IGHV3-33, L: IGKV3(D)-11) were prepared in E. coli in large quantities (Figure 10D). Since the same amino acids (H21S and H79Y) as the consensus sequence were used at H21 and H79 in Teprotumumab, mutations (H16G, L60D, and L85D) were introduced to obtain the mutants into which the consensus sequences were introduced. The binding activity was assessed by performing ELISA similar to that in 1.1 using IGF-1R as the target while changing scFv concentrations, and setting the scFv concentration that exhibits 50% binding as $EC_{50}$, which revealed that the binding activity of both scFv was equivalent to that of the wild type, whereas the amounts prepared and the Tm values improved by 1.25 to 1.5 times and 2.5 to 6°C, respectively (Figure 10E), indicating that sequences with the introduced mutations that have improved the functions and characteristics of Nivolumab can be applied to the same germline.

1.5 Introduction of Mutations into Remaining Mutation Residue Positions

Next, a mutant library was produced in which mutations were introduced into sites where mutation introduction had not been considered among the mutation residue positions (□ marks in Figure 2, second library). At that time, according to Figure 10A, some of the positions determined to be significant for the wild type or mutated amino acids (L1, L9, L60, L77, L85, H21, H23, H79, and H94) in the significant difference determination were immobilized to those amino acids.

83 mutants were produced, and many of them showed that the expression levels in E. coli were substantially improved. Therefore, samples obtained by IMAC-purifying medium supernatant fractions were used to measure the concentrations of expressed mutants using the BCA method, and the binding activity was measured with ELISA in the same manner as in 1.1. Then, the functional thermostability was measured using the medium supernatant fractions with ELISA in the same manner as in 1.1 (except that the heat treatment temperature was 60°C), and the thermal denaturation mid-points (Tm values) were also measured using the IMAC-purified samples by the thermal shift assay method (structural thermostability).

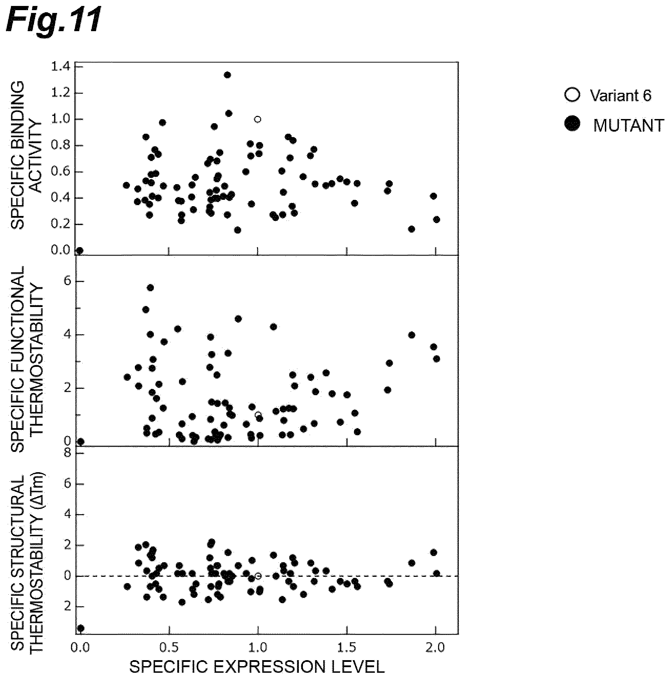

The expression level, binding activity, and functional thermostability of each mutant were each determined as a ratio (specific expression level, specific binding activity, and specific functional thermostability) relative to the assessed values of the Top 1 mutant (Variant 6 in Figure 8A) that the machine learning predicted by using the training data scored with characteristic values obtained by heat treatment at 50°C in 1.3, and the structural thermostability of each mutant was determined as a difference (specific structural thermostability) relative to the assessed value of Variant 6 (Figure 11). As a result, a certain number of mutants exhibited expression levels and functional thermostability exceeding those of Variant 6.

The construction of prediction systems was performed using the dataset of the heat treatment at 60°C described above, in the same Case 3 as in 1.2 (except that the reference values were standardized values when the specific expression level and specific binding activity of Variant 6 were 1: $1 \times 1$ for all in the case of the specific expression level and specific binding activity) and in the following 2 methods.

Case 4

Score value = f1(specific expression level - 1) $\times$ f2(specific binding activity - 1) $\times$ f3(specific functional thermostability) $\times$ f4(specific structural thermostability - 0)

f1(x) = sigmoid (x)
f2(x) = sigmoid (x)
f3(x) = gaussian (x), $\mu$ = 5.77, $\sigma$ = 4
f4(x) = sigmoid (x)

Case 5

Score value = f1(specific expression level - 1) $\times$ f2(specific binding activity - 1) $\times$ f3(specific functional thermostability - 0)

f1(x) = sigmoid (x)
f2(x) = sigmoid (x)
f3(x) = sigmoid (x)

In Case 4, the sigmoid function was used for training for evolution such that the specific expression level, specific binding activity, and specific structural thermostability become higher than the reference values (multiple number of standardized values when the specific expression level and specific binding activity of Variant 1 are 1: 1 x 1 for all in the case of the specific expression level and specific binding activity. Standardized values when the specific structural thermostability of Variant 1 is 0), and the Gaussian function was used for training for evolution such that the optimal value of the specific functional thermostability is 5.77 times the reference value (multiple number of standardized values when the specific functional thermostability of Variant 1 is 0: 1 $\times$ 1 for all).

In Case 5, the sigmoid function was used for all for training for evolution such that the specific expression level, specific binding activity, and specific structural thermostability become higher than the reference values (multiple number of standardized values when the specific expression level and specific binding activity of Variant 1 are 1: 1 $\times$ 1 for all in the case of the specific expression level and specific binding activity. Standardized values when the specific structural thermostability of Variant 6 is 0). The specific functional thermostability was not set as a target for training.

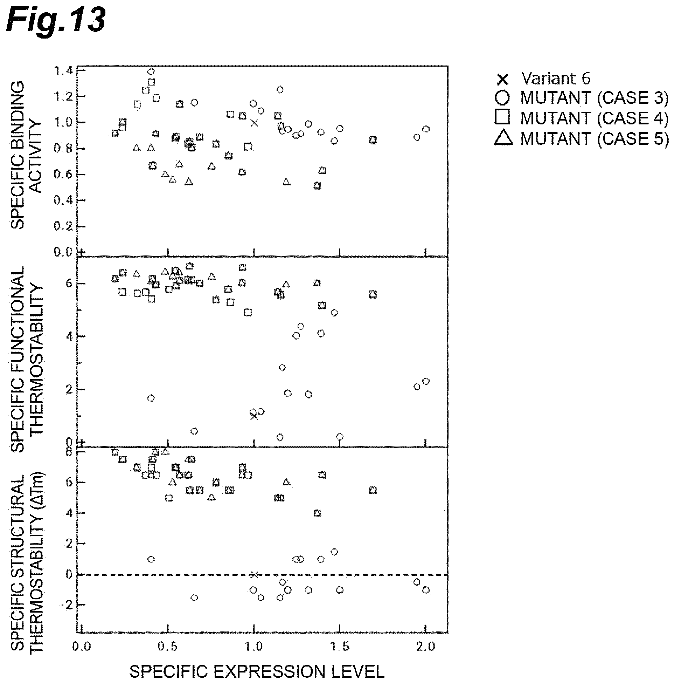

Using the constructed prediction system, predicted score values of all mutants (excluding 83 mutants which were already measured as training data, and Variant 6) contained in the sequence space formed by 17 residues (□ marks in Figure 2) were calculated. Among those, mutant library genes were produced such that they contain as many of the Top 100 as possible of each case. The designed genes were inserted into an E. coli-expression vector, and samples obtained by IMAC-purifying medium supernatant fractions in which mutants were expressed were used to measure the concentrations of expressed mutants by the BCA method, the binding activity by ELISA, and the structural thermostability by the thermal shift assay method. The functional thermostability was measured by ELISA using medium supernatant fractions which was heat treated at 60°C. As a result, it was possible to obtain a group of mutants in which the functional thermostability and the structural thermostability were improved compared to a group of mutants in the training data, without reducing the expression level and binding activity compared to Variant 6 (Figures 12 and 13).

Example 2: Maturation of Anti-CD38 Human Antibody Variable Region Fragment

Simultaneous optimization of the expression level, binding activity, and thermostability (functional thermostability and structural thermostability) was also attempted for Fv of the anti-CD38 human antibody Daratumumab.

2.1 Modification of Fv

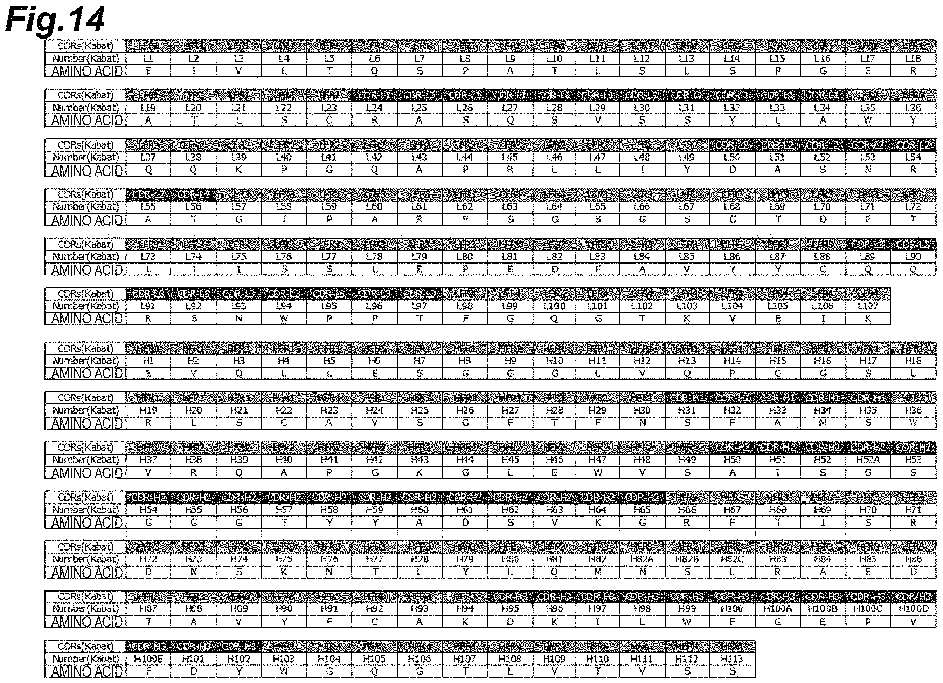

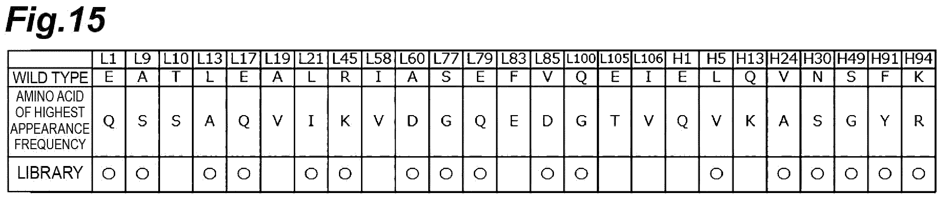

A sequence alignment of Fv of Daratumumab (light chain: SEQ ID NO: 3; heavy chain: SEQ ID NO: 4, Figure 14) was performed using the antibody database abYsis (Swindells, M. B. et al. J. Mol. Biol. 429, 356-364 (2017)), to calculate the appearance frequency of amino acids at respective residue positions in a human antibody. Then, the Fv was compared with the amino acid sequence, and a mutant library (the number of mutants: 93) was produced in which the original amino acid (wild type) or amino acids of high appearance frequencies appear at 17 residue positions (O marks in Figure 15) out of 25 residues of the residue positions different from those of the amino acids of high appearance frequencies.

The mutants described above were expressed as scFv in E. coli, the medium supernatant fractions were IMAC-purified, the concentrations of expressed mutants were measured using the BCA method, and the binding activity was measured with ELISA in the same manner as in 1.1, using IMAC-purified samples. The functional thermostability was assessed by values which were obtained by adding IMAC-purified samples that were heat treated at 55°C or 60°C for 1 hour, on a plate immobilized with a CD38 protein, measuring the amount of each mutant remained on the plate using an HRP-conjugated anti-FLAG tag antibody, and standardizing the measured values with the assessed values of the binding activity. In addition, the thermal denaturation mid-points (Tm values) were also measured using the IMAC-purified samples by the thermal shift assay method (structural thermostability).

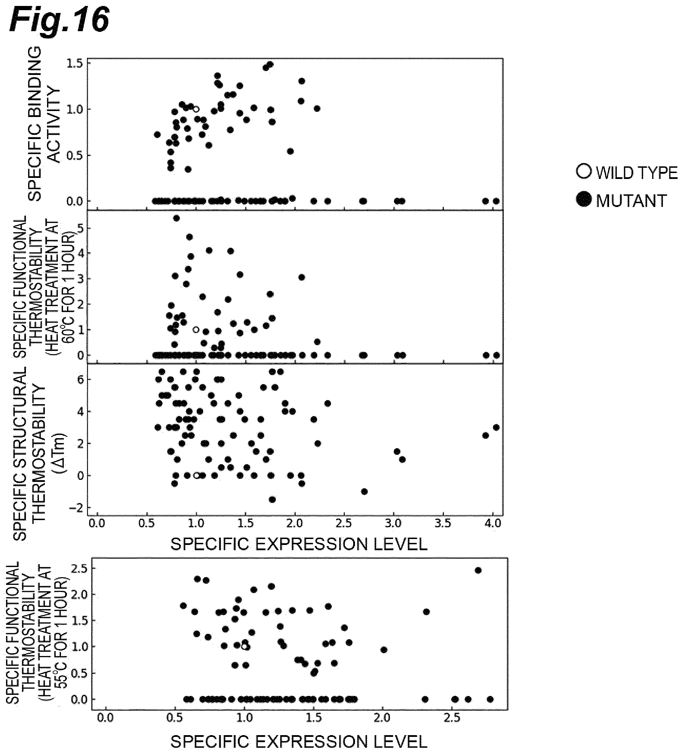

The expression level, binding activity, functional thermostability, and Tm value of each mutant were determined as a ratio (specific expression level, specific binding activity, and specific functional thermostability) relative to the assessed values of the expression level, binding activity, and functional thermostability of the wild type before mutation, respectively, and for Tm values, as a difference (specific structural thermostability, ΔTm) relative to that of the wild type before mutation. Plotting the specific expression level and specific binding activity, specific expression level and specific functional thermostability, and specific expression level and ΔTm revealed that there were many mutants in which ΔTm was improved compared to the wild type, but many of them had reduced binding activity and functional thermostability (Figure 16).

2.2 Production of Prediction System through Machine Learning

The above data was used as training data, and machine learning was conducted in which the functional assessment values of unknown mutants were predicted from the amino acid sequences, using PHYSBO. The four characteristic values (specific expression level, specific binding activity, specific functional thermostability, and specific structural thermostability) were scored as one value by Case 3 in 1.2 and the following methods. For the specific functional thermostability, data of the heat treatment at 60°C for 1 hour was used.

Case 6

Score value = f1(specific expression level - 1) × f2(specific binding activity - 1) × f3(specific functional thermostability) × f4(specific structural thermostability - 0)

f1(x) = sigmoid (x)
f2(x) = sigmoid (x)
f3(x) = gauss (x), $\mu$ = 1, $\sigma$ = 0.6
f4(x) = sigmoid (x)

Case 7

Score value = f1(specific expression level - 1) × f2(specific binding activity - 1) × f3(specific functional thermostability)

f1(x) = sigmoid (x)
f2(x) = sigmoid (x)
f3(x) = gauss (x), $\mu$ = 1, $\sigma$ = 0.6

Case 8

The same formula as in Case 7 is used, but the specific expression level of mutants with a specific expression level exceeding 1 is considered to be 1.

In Case 6, the sigmoid function was used for all for training for evolution such that the specific expression level, specific binding activity, and specific structural thermostability become higher than the reference values (multiple number of standardized values when the specific expression level and specific binding activity of the wild type are 1: 1 × 1 for all in the case of the specific expression level and specific binding activity), and the Gaussian function was used for training for evolution such that the optimal value of the specific functional thermostability is 1 time the reference value (multiple number of standardized values when the specific functional thermostability of the wild type is 1: 1 × 1 for all).

In Case 7, the sigmoid function was used for all for training for evolution such that the specific expression level and specific binding activity become higher than the reference values, and the Gaussian function was used for training for evolution such that the optimal value of the specific functional thermostability is 1 time the reference value.

In Case 8, the sigmoid function with the restriction that the specific expression level is set to a value equal to the reference value when the specific expression level exceeds the reference value was used for training for evolution such that the specific expression level becomes equal to or higher than the reference value, the sigmoid function was used for training for evolution such that the specific binding activity becomes higher than the reference value, and the Gaussian function was used for training for evolution such that the optimal value of the specific functional thermostability is 1 time the reference value.

2.3 Selection of Promising Mutant by Prediction System

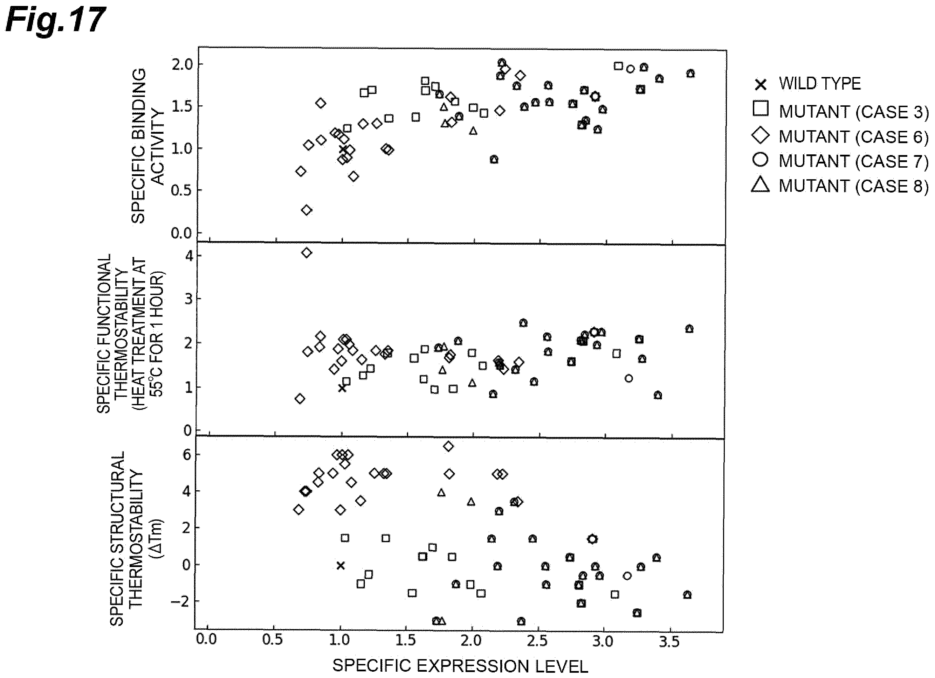

Using the constructed prediction system, predicted score values of all mutants (excluding 93 mutants which were already measured as training data, and the wild type) contained in the sequence space formed by 17 residues (○ marks in Figure 15) were calculated. Among those, the design of library genes such that they contain as many of the Top 100 as possible of each case was performed by selecting a similar template sequence from the training data and using degenerate codons to produce a mutant library (second library). The designed genes were inserted into an E. coli-expression vector, and samples obtained by IMAC-purifying from a medium supernatant in which mutants were expressed according to 2.1 were used to measure the specific expression level with the BCA method, the specific binding activity and specific functional thermostability (heat treatment at 55°C for 1 hour) with ELISA, and the specific structural stability (Tm value) with the thermal shift assay method. As a result, it was possible to obtain mutants exhibiting the binding activity equivalent to that of the wild type, with the expression level improved compared to mutants in the training data (Figure 17). Further, there was almost no mutant that exhibited the functional thermostability lower than that of the wild type as was seen in the training data, whereas there were many mutants that exhibited higher values than that of the wild type, and there were many mutants in which Tm values were also improved. In particular, the improvement of Tm values was prominently observed in Case 6, in which the specific structural thermostability was reflected in the score values.

Example 3: Maturation of Antibody Variable Region Fragment Including Solubility Characteristic

Simultaneous optimization of the characteristics of the solubility in addition to the expression level and binding activity was attempted using the anti-PD-1 human antibody Nivolumab with the same mutagenesis design as in Example 1 (O marks in Figure 2).

3.1 Modification of Fv

For Fv of Nivolumab, a mutant library (the number of mutants: 88) was produced in which the original amino acid (wild type) or amino acids of high appearance frequencies appear at the same residue positions (O marks in Figure 2) as in Example 1.

The mutants described above were expressed as scFv in E. coli, the medium supernatant fractions were IMAC-purified, the concentrations of expressed mutants (expression levels) were measured using the BCA method, and the binding activity was measured with ELISA in the same manner as in 1.1, using IMAC-purified samples. The measurement of solubility was performed by removing imidazole and the like from the IMAC-purified samples by dialysis or centrifugation enrichment, adding polyethylene glycol 4000 (PEG4000) at a final concentration of 20%, leaving the samples to stand at 25°C for 18 hours, and then, measuring the absorbance of the samples in which the precipitation had been removed by centrifugation manipulation at 280 nm, the absorbance serving as an indicator of solubility (PEG precipitation method).

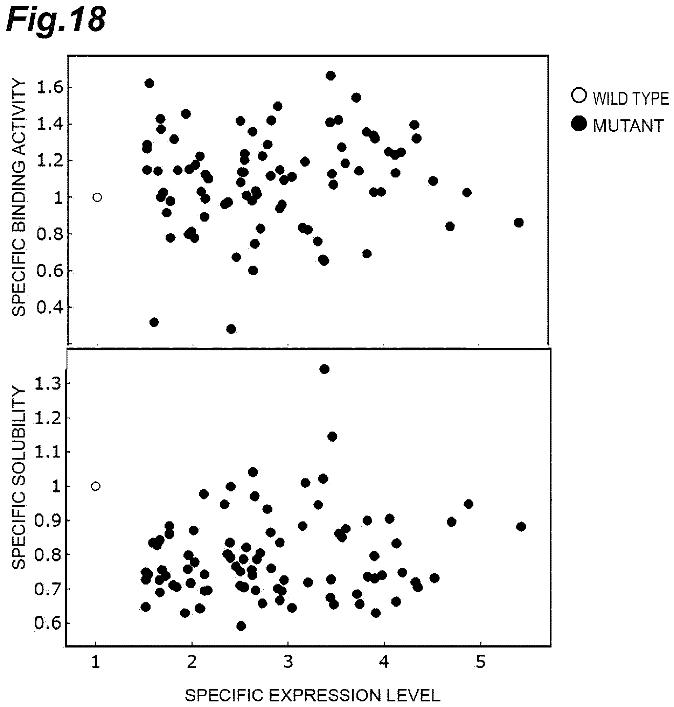

The expression level, binding activity, and solubility of each mutant were determined as a ratio (specific expression level, specific binding activity, and specific solubility) relative to the assessed values of the expression level, binding activity, and solubility of the wild type before mutation, respectively. Plotting the specific expression level and specific binding activity, and specific expression level and specific solubility revealed that there were a certain number of mutants in which the expression level was improved without reducing the target binding properties compared to the wild type, whereas many of the mutants in which the expression level was improved had reduced solubility (Figure 18).

3.2 Production of Prediction System through Machine Learning

The above data was used as training data, and machine learning was conducted in which the functional assessment values of unknown mutants were predicted from the amino acid sequences, using PHYSBO in the same manner as in Example 2. The three characteristic values (specific expression level, specific binding activity, and specific solubility) were scored as one value by the following 2 methods.

Case 9

$$\text{Score value} = f1(\text{specific binding activity}) \times f2(\text{specific solubility} - 1)$$

f1(x) = gaussian (x), $\mu$ = 1, $\sigma$ = 0.898, a = 0.5
f2(x) = sigmoid (x)

Case 10

Score value = f1(specific expression level $\times$ 0.25 - 1) $\times$ f2(specific binding activity) $\times$ f3(specific solubility - 1)

f1(x) = sigmoid (x)
f2(x) = gauss (x), $\mu$ = 1, $\sigma$ = 0.898, a = 0.5
f3(x) = sigmoid (x)

In Case 9, the Gaussian function was used for training for evolution such that the optimal value of the specific binding activity is 1 time the reference value (multiple number of standardized values when the specific binding activity of the wild type is 1: 1 $\times$ 1 in the case of the specific binding activity), and the sigmoid function was used for training for evolution such that the specific solubility becomes higher than the reference value (multiple number of standardized values when the specific solubility of the wild type is 1: 1 $\times$ 1 in the case of the specific solubility).
In Case 10, the sigmoid function was used for all for training for evolution such that the expression level and specific solubility become higher than the reference values (multiple number of standardized values when the specific expression level and specific solubility of the wild type are 1: 1 $\times$ 0.25 in the case of the specific expression level, and 1 $\times$ 1 in the case of the specific solubility), and the Gaussian function was used for training for evolution such that the optimal value of the specific binding activity is 1 time the reference value.

3.3 Selection of Promising Mutant by Prediction System

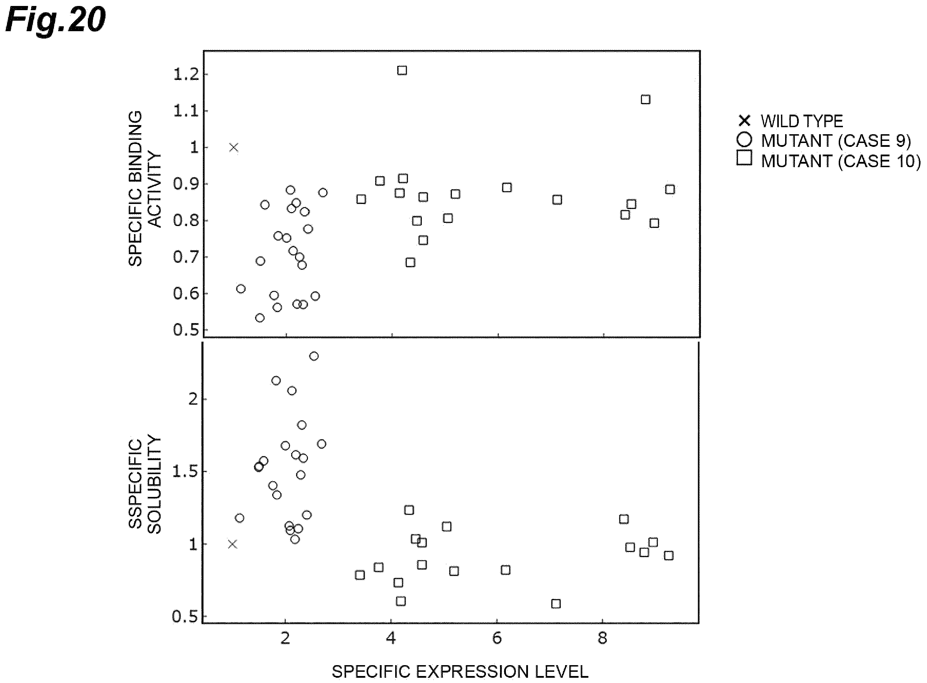

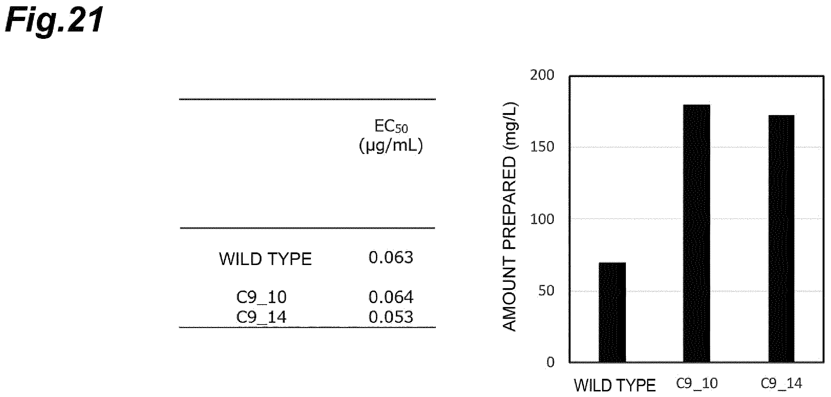

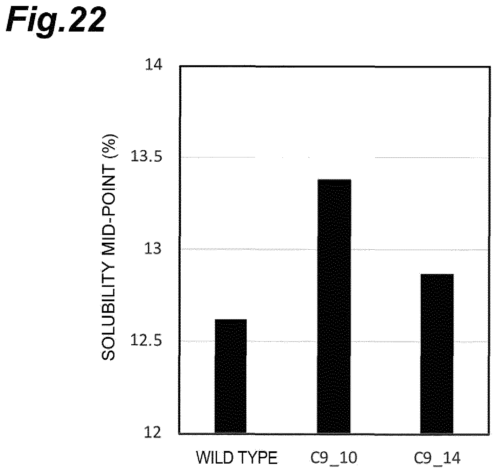

Using the constructed prediction system, predicted score values of all mutants (excluding 88 mutants which were already measured as training data, and the wild type) contained in the sequence space formed by 18 residues (○ marks in Figure 2) were calculated. Among those, the design of library genes such that they contain as many of the Top 100 as possible of each case and contain sequences in even higher sections among them was performed by selecting a similar template sequence from the training data and using degenerate codons to produce a mutant library (second library). The designed genes were inserted into an E. coli-expression vector, and samples obtained by IMAC-purifying from a medium supernatant in which mutants were expressed according to 3.1 were used to measure the specific expression level with the BCA method, the specific binding activity with ELISA, and the specific solubility with the PEG precipitation method. As a result, it was possible to obtain mutants in which the binding activity was slightly lower than that of the wild type according to ELISA assessment, but the expression level and solubility were improved compared to mutants in the training data (Figures 19 and 20). In particular, in Case 9, the mutants were included in which the improvement of expression level was about 2 times, but the solubility was improved by about 1.5 to 2 times.
When 2 mutants (C9_10, and C9_14) of Case 9 in Figure 19 were prepared in Expi293F cells as full-length antibodies IgG, the binding activity was equivalent to that of the wild type, whereas the expression level was improved by 2.5 times (Figure 21). The solubility was assessed by changing final concentrations of PEG4000, calculating solubility curves, and determining the concentrations of polyethylene glycol at which 50% of IgG precipitates (solubility mid-points). As a result, The resulting solubility mid-points were improved by 0.26% and 0.76% relative to the wild type (Figure 22). From that, it was revealed that among the mutants in which at least some of residues not being an amino acid of the highest appearance frequency were modified into the amino acid of the highest appearance frequency, there were mutants in which the expression level and the solubility were simultaneously improved without reducing the binding activity.

Example 4: Maturation of Antibody Using Full-Length Antibodies

Also, simultaneous optimization of the structural thermostability in addition to the expression level, binding activity, and solubility was attempted using mutants into which mutations were introduced into the full-length antibody IgG itself as a target to be measured.

4.1 Modification of Full-Length Antibodies

For Fv of Nivolumab, a mutant library (the number of mutants: 94) in which the original amino acid (wild type) or amino acids of high appearance frequencies appear at the same residue positions (O marks in Figure 2) as in Example 1 was expressed in Expi293F cells as IgG. The medium supernatant fractions were purified with a Protein A-immobilized column, and the concentrations of expressed mutants were measured using the BCA method; and the binding activity was assessed by values which were obtained by adding samples purified with the Protein A column on a plate immobilized with a PD-1 protein, which was a target protein, and washing, followed by measuring the amount of each mutant remained on the plate using an anti-human IgG antibody, and standardizing the measured values with the assessed values of the expression level. For the solubility, PEG4000 was added at a final concentration of 13% or 13.5%, the samples were left to stand at 25°C for 18 hours, and then, the absorbance of the samples in which the precipitation had been removed by centrifugation manipulation was measured at 280 nm to provide an indicator of solubility. For the structural thermostability, the thermal denaturation mid-points (Tm values) and stabilized score WSS values were measured by the thermal shift assay method (differential scanning fluorometry).

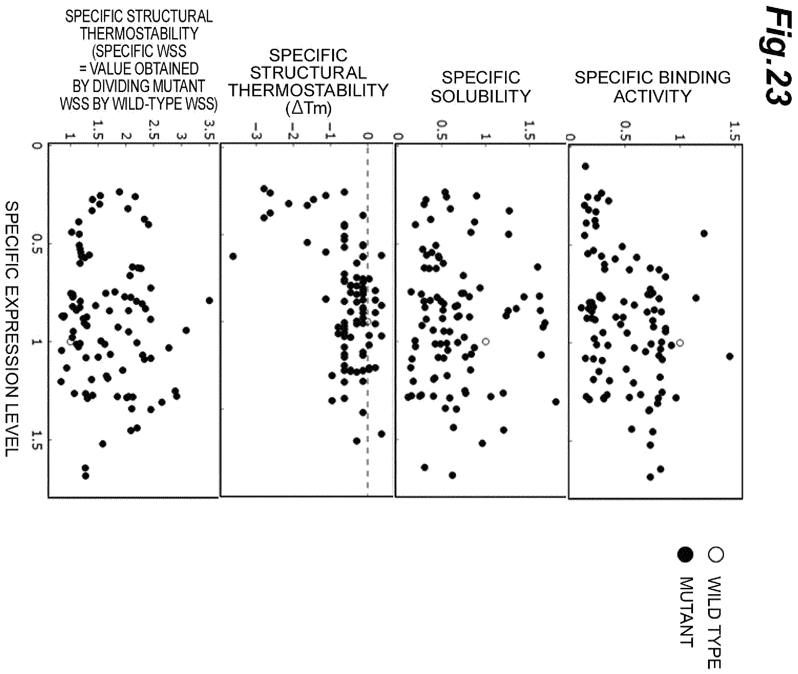

The expression level, binding activity, solubility, Tm values, and WSS values of each mutant were determined as a ratio (specific expression level, specific binding activity, and specific solubility) relative to the assessed values of the expression level, binding activity, and solubility of the wild type before mutation, respectively, and for Tm values, as a difference ($\Delta$Tm) relative to that of the wild type before mutation. For WSS values, a ratio (specific WSS) relative to the assessed value of the wild type before mutation and a difference ($\Delta$WSS) relative to that of the wild type before mutation were determined. Plotting the specific expression level and specific binding activity, specific expression level and specific solubility, specific expression level and $\Delta$Tm, and specific expression level and specific WSS values revealed that there were relatively many mutants in which the binding activity and solubility were reduced compared to the wild type, and for the structural thermostability, there were many mutants exhibiting Tm values equivalent to that of the wild type, but WSS values showing the stability tended to be increased (Figure 23).

4.2 Production of Prediction System through Machine Learning

The specific expression level, specific binding activity, specific solubility, $\Delta$Tm, and differences relative to the wild type before mutation for WSS values ($\Delta$WSS) of the above data were used as training data, and machine learning was conducted in which the functional assessment values of unknown mutants were predicted from the amino acid sequences, using PHYSBO in the same manner as in Example 3. The four characteristic values (specific expression level, specific binding activity, specific solubility, and $\Delta$WSS) were scored as one value by the following 3 methods.

Case 11

Score value = fl(specific expression level - 1) $\times$ f2(specific binding activity - 1) $\times$ f3(specific solubility - 1) $\times$ f4($\Delta$WSS/ standard deviation of $\Delta$WSS - 0)

    f1(x) = sigmoid (x)
    f2(x) = sigmoid (x)
    f3(x) = sigmoid (x)
    f4(x) = sigmoid (x), gain = 0.3

Case 12

$$\text{Score value} = \text{f1(specific binding activity)} \times \text{f2(specific solubility} - 1)$$

    f1(x) = gaussian (x), $\mu$ = 1, $\sigma$ = 0.847, a = 0.5
    f2(x) = sigmoid (x)

Case 13

$$\text{Score value} = \text{f1(specific solubility - 1)}$$

f1(x) = sigmoid (x)

In Case 11, the sigmoid function was used for training for evolution such that the specific expression level, specific binding activity, specific solubility, and $\Delta$WSS become higher than the reference values (multiple number of standardized values when the specific expression level, specific binding activity, and specific solubility of the wild type are 1: $1 \times 1$ for all in the case of the specific expression level, specific binding activity, and specific solubility. Values planned with WSS values of the wild type as 0).

In Case 12, the Gaussian function was used for training for evolution such that the optimal value of the specific binding activity is 1 time the reference value, and the sigmoid function was used for training for evolution such that the specific solubility becomes higher than the reference value.

In Case 13, the sigmoid function was used for training for evolution such that the specific solubility becomes higher than the reference value.

4.3 Selection of Promising Mutant by Prediction System

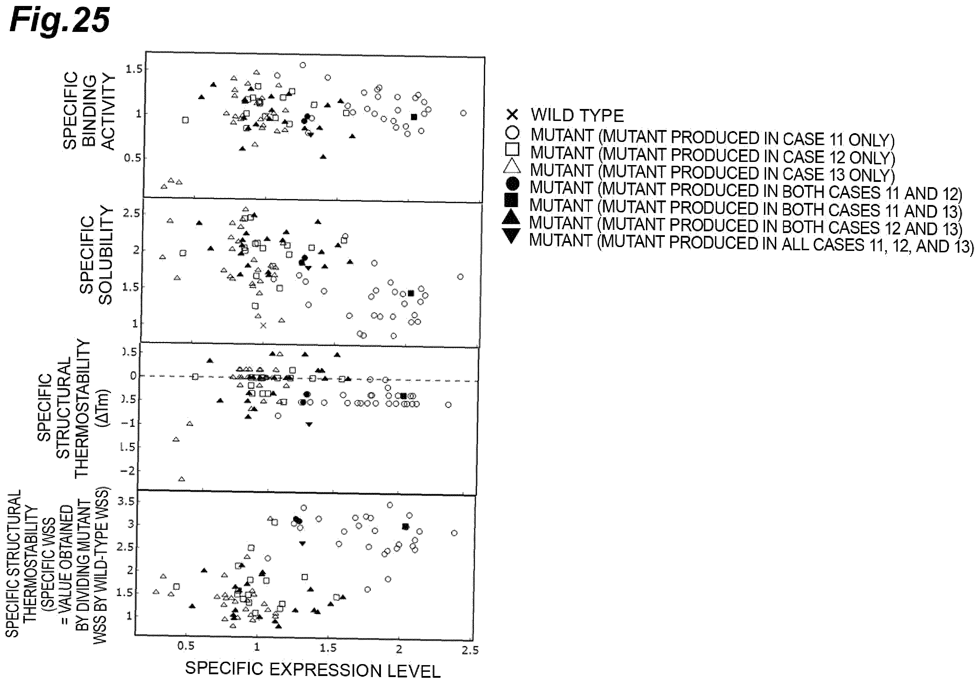

Using the constructed prediction system, predicted score values of all mutants (excluding 94 mutants which were already measured as training data, and the wild type) contained in the sequence space formed by 18 residues (○ marks in Figure 2) were calculated. Among those, the design of library genes such that they contain as many of the Top 100 as possible of each case and contain sequences in even higher sections among them was performed by selecting a similar template sequence selected from the training data and using degenerate codons to produce a mutant library (second library). The designed genes were inserted into a mammalian cell-expression vector, and samples obtained by purifying from a medium supernatant in which mutants were expressed according to 4.1 with a Protein A column were used to measure the specific expression level with the BCA method, the specific binding activity with ELISA, and the specific solubility with the PEG precipitation method. As a result, in addition to the improvement in expression level and solubility compared to mutants in the training data, it was possible to obtain mutants with the binding activity and structural stability equivalent to those of the wild type (Figures 24 and 25). This revealed that among the mutants in which at least some of residues not being an amino acid of the highest appearance frequency were modified into the amino acid of the highest appearance frequency, there were mutants in which the expression level and solubility were simultaneously improved without reducing the binding activity and structural thermostability.

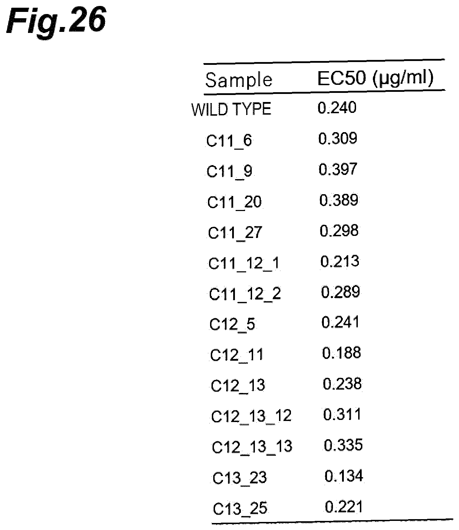

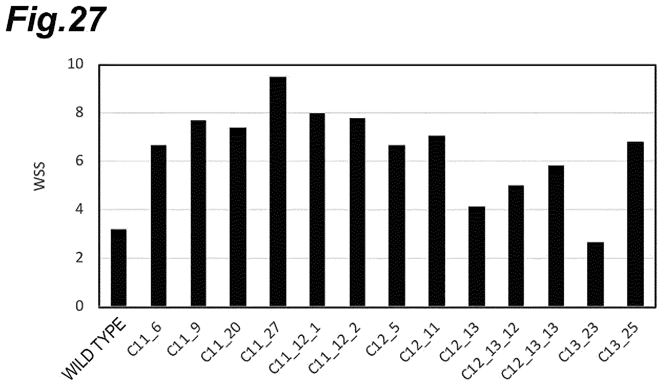

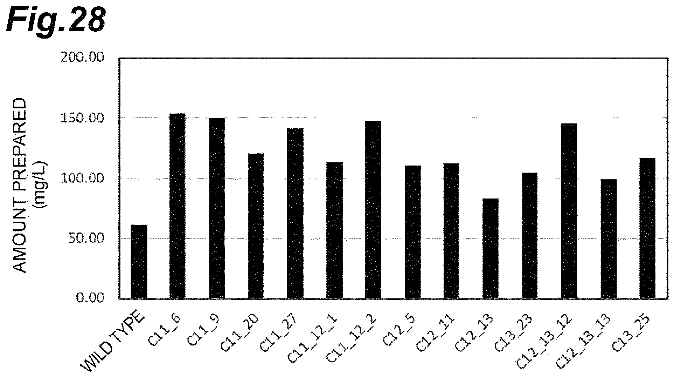

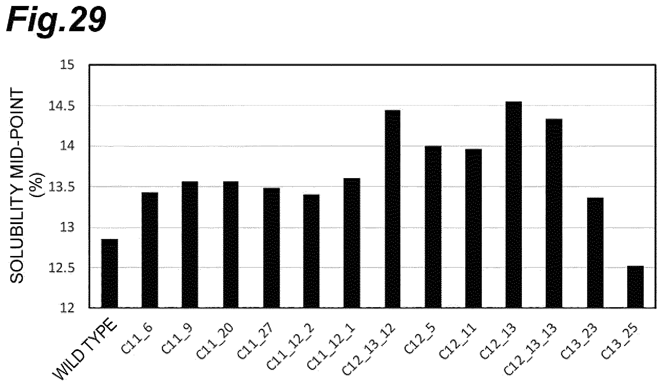

When 13 mutants in Figure 25 were further quantitatively measured, all of the 13 mutants exhibited the binding activity and structural thermostability equivalent to those of the wild type, while the amount prepared was improved by 1.3 to 2.5 times, and the solubility was improved by 0.50 to 1.69% (Figures 26, 27, 28, and 29). In particular, in Case 12, the solubility was improved the most.

4.4 Rule Extraction of Appeared Amino Acid

In order to extract the mutation residue positions that affect the characteristic values, the produced training data and the data of the mutant group produced according to the machine learning prediction (ML data) were used to separate wild-type amino acids and mutated amino acids, and the significant difference was determined by U-test. As a result, the positions where the mutated amino acids were superior in both binding activity and solubility were L21, L60, L77, H16, and H23.

**Industrial Applicability**

According to the present invention, it is possible to obtain amino acid sequence information on proteins with high industrial applicability, such as antibodies and enzymes for which two or more characteristics are optimized simultaneously. Accordingly, modification of the proteins for the purpose of functional improvement can be easily performed.

All publications, patents, and patent applications cited in this specification shall be incorporated herein as they are as reference.

**Claims**

1. A method of producing an antibody that is optimized for a plurality of characteristics including at least two of an expression level, binding activity, and stability, comprising:

   1) providing a library composed of mutants prepared by modifying at least some of residues not being an amino acid of the highest appearance frequency in a group of antibody sequences of a target antibody into the amino acid of the highest appearance frequency;
   2) determining respective characteristic values indicating the plurality of characteristics of some of the mutants in the library, and scoring the characteristic values as one value per mutant by normalizing and integrating the characteristic values;
   3) conducting machine learning by using the score values and ranking the mutants included in the library; and
   4) selecting an antibody that is optimized for the plurality of characteristics based on the ranking results.

2. The method according to claim 1, wherein the characteristic values are values each obtained by converting measurement data related to the characteristics of each mutant into numerical values, as a ratio or difference relative to a target value.

3. The method according to claim 1, wherein the scoring is performed according to the following formula (I):

   Score value = $f$(1st characteristic value - reference value of 1st characteristic) $\times$ $f$(2nd characteristic value - reference value of 2nd characteristic)... $\times$ $f$(nth characteristic value - reference value of nth characteristics)     [Formula (1)]

   wherein n is the number of characteristics to be optimized, f(x) is any selected from the group consisting of a sigmoid function (x), a hyperbolic tangent function (x), a Gaussian function (x), a lognormal distribution function (x), a ReLU function (x), a linear function (x), an n-dimensional function (x), an exponential function (x), a logarithmic function (x), a hyperbolic function (x), and a combination thereof.

4. The method according to claim 3, wherein f(x) is a sigmoid function (x), a hyperbolic tangent function (x), a Gaussian function (x), or a lognormal distribution function (x).

5. The method according to claim 1, wherein the machine learning is conducted by any selected from Bayesian linear regression, linear regression, Gaussian process regression, logistic regression, decision tree, simple perceptron, multilayer perceptron, neural network, deep neural network, k-nearest neighbor algorithm, and support vector machine.

6. The method according to claim 1, wherein the plurality of characteristics further include solubility.

7. The method according to claim 1, wherein the stability is functional thermostability and structural thermostability.

8. A method of producing an antibody that is optimized for a plurality of characteristics including at least two of an expression level, binding activity, and stability, comprising:

   1) providing a first library composed of mutants prepared by modifying at least some of residues not being an amino acid of the highest appearance frequency in a group of antibody sequences of a target antibody, into the amino acid of the highest appearance frequency;
   2) determining respective characteristic values indicating the plurality of characteristics of some of the mutants in the first library, and scoring the plurality of characteristic values as one value per mutant by normalizing and integrating the plurality of characteristic values;
   3) conducting machine learning by using the score values and ranking the mutants;
   4) obtaining a second library, based on the ranking results; and
   5) screening the second library to determine an antibody that is optimized for the plurality of characteristics.

9. A method of producing a library consisting of a protein that is optimized for a plurality of characteristics including at least two of an expression level, binding activity, and stability, comprising:

   1) providing a first library composed of mutants prepared by modifying at least some of residues not being an amino acid of the highest appearance frequency in a group of antibody sequences of a target antibody, into the amino acid of the highest appearance frequency;

2) determining respective characteristic values indicating the plurality of characteristics of some of the mutants in the first library, and scoring the plurality of characteristic values as one value per mutant by normalizing and integrating the plurality of characteristic values;

3) conducting machine learning by using the score values and ranking the mutants; and

4) obtaining a second library, based on the ranking results.

10. The method according to claim 8 or 9, wherein the plurality of characteristic values are values each obtained by converting measurement data related to the characteristics of each mutant into numerical values, as a ratio or difference relative to a target value.

11. The method according to claim 8 or 9, wherein the scoring is performed according to the following formula (I):

Score value = $f$(1st characteristic value - reference value of 1st characteristic) $\times$ $f$(2nd characteristic value - reference value of 2nd characteristic)... $\times$ $f$(nth characteristic value - reference value of nth characteristics)          [Formula (1)]

wherein n is the number of characteristics to be optimized, f(x) is any selected from the group consisting of a sigmoid function (x), a hyperbolic tangent function (x), a Gaussian function (x), a lognormal distribution function (x), a ReLU function (x), a linear function (x), an n-dimensional function (x), an exponential function (x), a logarithmic function (x), a hyperbolic function (x), and a combination thereof.

12. The method according to claim 11, wherein f(x) is a sigmoid function (x), a hyperbolic tangent function (x), a Gaussian function (x), or a lognormal distribution function (x).

13. The method according to claim 8 or 9, wherein the machine learning is conducted by any selected from Bayesian linear regression, linear regression, Gaussian process regression, logistic regression, decision tree, simple perceptron, multilayer perceptron, neural network, deep neural network, k-nearest neighbor algorithm, and support vector machine.

14. The method according to claim 8 or 9, wherein the plurality of characteristics further include solubility.

15. The method according to claim 8 or 9, wherein the stability is functional thermostability and structural thermostability.

16. A method of producing an antibody that is optimized for a plurality of characteristics including at least two of a binding activity and solubility, comprising:

1) providing a library composed of mutants prepared by modifying at least some of residues not being an amino acid of the highest appearance frequency in a group of antibody sequences of a target antibody, into the amino acid of the highest appearance frequency;

2) determining respective characteristic values indicating the plurality of characteristics of some of the mutants in the library, and scoring the characteristic values as one value per mutant by normalizing and integrating the characteristic values;

3) conducting machine learning by using the score values and ranking the mutants included in the library; and

4) selecting an antibody that is optimized for the plurality of characteristics of the antibody, based on the ranking results.

17. An antibody Fv variant, wherein amino acid residues at positions 60 and 85 in a light chain amino acid sequence set forth in SEQ ID NO: 1 are substituted with aspartic acid and aspartic acid, respectively, and amino acid residues at positions 16, 21, and 79 in a heavy chain amino acid sequence set forth in SEQ ID NO: 2 are substituted with glycine, serine, and tyrosine, respectively.

18. An antibody Fv variant, wherein amino acid residues at positions 1, 9, 13, 17, 21, 45, 60, 77, 79, 85, and 100 in a light chain amino acid sequence set forth in SEQ ID NO: 1, and amino acid residues at positions 16, 21, 23, 27, 49, 79, and 94 in a heavy chain amino acid sequence set forth in SEQ ID NO: 2 are each substituted with sets of amino acids shown in Figures 5 and 7.

19. An antibody Fv variant, wherein amino acid residues at positions 1, 9, 10, 13, 17, 19, 21, 45, 58, 60, 77, 79, 83, 85, 100, 105, and 106 in a light chain amino acid sequence set forth in SEQ ID NO: 1, and amino acid residues at positions 11, 13, 16, 21, 23, 27, 49, 79, and 94 in a heavy chain amino acid sequence set forth in SEQ ID NO: 2 are each substituted with sets of amino acids shown in Figure 12.

20. An antibody Fv variant, wherein amino acid residues at positions 21, 60, and 77 in a light chain amino acid sequence set forth in SEQ ID NO: 1 are substituted with isoleucine, aspartic acid, and glycine, respectively, and amino acid residues at positions 16 and 23 in a heavy chain amino acid sequence set forth in SEQ ID NO: 2 are substituted with glycine and alanine, respectively.

21. An antibody Fv variant, wherein amino acid residues at positions 1, 9, 10, 13, 17, 19, 21, 45, 58, 60, 77, 79, 83, 85, 100, 105, and 106 in a light chain amino acid sequence set forth in SEQ ID NO: 1, and amino acid residues at positions 11, 13, 16, 21, 23, 27, 49, 79, and 94 in a heavy chain amino acid sequence set forth in SEQ ID NO: 2 are each substituted with sets of amino acids shown in Figure 19.

22. An antibody Fv variant, wherein amino acid residues at positions 1, 9, 10, 13, 17, 19, 21, 45, 58, 60, 77, 79, 83, 85, 100, 105, and 106 in a light chain amino acid sequence set forth in SEQ ID NO: 1, and amino acid residues at positions 11, 13, 16, 21, 23, 27, 49, 79, and 94 in a heavy chain amino acid sequence set forth in SEQ ID NO: 2 are each substituted with sets of amino acids shown in Figure 24.

# Fig.1

## Light Chain (Kabat numbering)

| CDRs(Kabat) | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 |
|---|---|---|---|---|---|---|---|---|---|---|---|---|---|---|---|---|---|---|
| Number(Kabat) | L1 | L2 | L3 | L4 | L5 | L6 | L7 | L8 | L9 | L10 | L11 | L12 | L13 | L14 | L15 | L16 | L17 | L18 |
| AMINO ACID | E | I | V | L | T | Q | S | P | A | T | L | S | L | S | P | G | E | R |

| CDRs(Kabat) | LFR1 | LFR1 | LFR1 | LFR1 | LFR1 | CDR-L1 | CDR-L1 | CDR-L1 | CDR-L1 | CDR-L1 | CDR-L1 | CDR-L1 | CDR-L1 | CDR-L1 | CDR-L1 | CDR-L1 | LFR2 | LFR2 |
|---|---|---|---|---|---|---|---|---|---|---|---|---|---|---|---|---|---|---|
| Number(Kabat) | L19 | L20 | L21 | L22 | L23 | L24 | L25 | L26 | L27 | L28 | L29 | L30 | L31 | L32 | L33 | L34 | L35 | L36 |
| AMINO ACID | A | T | L | S | C | R | A | S | Q | S | V | S | S | Y | L | A | W | Y |

| CDRs(Kabat) | LFR2 | LFR2 | LFR2 | LFR2 | LFR2 | LFR2 | LFR2 | LFR2 | LFR2 | LFR2 | LFR2 | LFR2 | LFR2 | CDR-L2 | CDR-L2 | CDR-L2 | CDR-L2 | CDR-L2 |
|---|---|---|---|---|---|---|---|---|---|---|---|---|---|---|---|---|---|---|
| Number(Kabat) | L37 | L38 | L39 | L40 | L41 | L42 | L43 | L44 | L45 | L46 | L47 | L48 | L49 | L50 | L51 | L52 | L53 | L54 |
| AMINO ACID | Q | Q | K | P | G | Q | A | P | R | L | L | I | Y | D | A | S | N | R |

| CDRs(Kabat) | CDR-L2 | CDR-L2 | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 |
|---|---|---|---|---|---|---|---|---|---|---|---|---|---|---|---|---|---|---|
| Number(Kabat) | L55 | L56 | L57 | L58 | L59 | L60 | L61 | L62 | L63 | L64 | L65 | L66 | L67 | L68 | L69 | L70 | L71 | L72 |
| AMINO ACID | A | T | G | I | P | A | R | F | S | G | S | G | S | G | T | D | F | T |

| CDRs(Kabat) | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 | CDR-L3 | CDR-L3 |
|---|---|---|---|---|---|---|---|---|---|---|---|---|---|---|---|---|---|---|
| Number(Kabat) | L73 | L74 | L75 | L76 | L77 | L78 | L79 | L80 | L81 | L82 | L83 | L84 | L85 | L86 | L87 | L88 | L89 | L90 |
| AMINO ACID | L | T | I | S | S | L | E | P | E | D | F | A | V | Y | Y | C | Q | Q |

| CDRs(Kabat) | CDR-L3 | CDR-L3 | CDR-L3 | CDR-L3 | CDR-L3 | CDR-L3 | CDR-L3 | LFR4 | LFR4 | LFR4 | LFR4 | LFR4 | LFR4 | LFR4 | LFR4 | LFR4 |
|---|---|---|---|---|---|---|---|---|---|---|---|---|---|---|---|---|
| Number(Kabat) | L91 | L92 | L93 | L94 | L95 | L96 | L97 | L98 | L99 | L100 | L101 | L102 | L103 | L104 | L105 | L106 | L107 |
| AMINO ACID | S | S | N | W | P | R | T | F | G | Q | G | T | K | V | E | I | K |

## Heavy Chain (Kabat numbering)

| CDRs(Kabat) | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 |
|---|---|---|---|---|---|---|---|---|---|---|---|---|---|---|---|---|---|---|---|
| Number(Kabat) | H1 | H2 | H3 | H4 | H5 | H6 | H7 | H8 | H9 | H10 | H11 | H12 | H13 | H14 | H15 | H16 | H17 | H18 | H19 |
| AMINO ACID | Q | V | Q | L | V | E | S | G | G | G | V | V | Q | P | G | R | S | L | R |

| CDRs(Kabat) | HFR1 | HFR1 | HFR1 | HFR1 | HFR1 | HFR1 | HFR1 | HFR1 | HFR1 | HFR1 | HFR1 | CDR-H1 | CDR-H1 | CDR-H1 | CDR-H1 | CDR-H1 | HFR2 | HFR2 | HFR2 |
|---|---|---|---|---|---|---|---|---|---|---|---|---|---|---|---|---|---|---|---|
| Number(Kabat) | H20 | H21 | H22 | H23 | H24 | H25 | H26 | H27 | H28 | H29 | H30 | H31 | H32 | H33 | H34 | H35 | H36 | H37 | H38 |
| AMINO ACID | L | D | C | K | A | S | G | I | T | F | S | N | S | G | M | H | W | V | R |

| CDRs(Kabat) | HFR2 | HFR2 | HFR2 | HFR2 | HFR2 | HFR2 | HFR2 | HFR2 | HFR2 | HFR2 | HFR2 | CDR-H2 | CDR-H2 | CDR-H2 | CDR-H2 | CDR-H2 | CDR-H2 | CDR-H2 |
|---|---|---|---|---|---|---|---|---|---|---|---|---|---|---|---|---|---|---|
| Number(Kabat) | H39 | H40 | H41 | H42 | H43 | H44 | H45 | H46 | H47 | H48 | H49 | H50 | H51 | H52 | H52A | H53 | H54 | H55 | H56 |
| AMINO ACID | R | A | P | G | K | G | L | E | W | V | A | V | I | W | Y | D | G | S | K |

| CDRs(Kabat) | CDR-H2 | CDR-H2 | CDR-H2 | CDR-H2 | CDR-H2 | CDR-H2 | CDR-H2 | CDR-H2 | CDR-H2 | HFR3 | HFR3 | HFR3 | HFR3 | HFR3 | HFR3 | HFR3 | HFR3 | HFR3 | HFR3 |
|---|---|---|---|---|---|---|---|---|---|---|---|---|---|---|---|---|---|---|---|
| Number(Kabat) | H57 | H58 | H59 | H60 | H61 | H62 | H63 | H64 | H65 | H66 | H67 | H68 | H69 | H70 | H71 | H72 | H73 | H74 | H75 |
| AMINO ACID | R | Y | Y | A | D | S | V | K | G | R | F | T | I | S | R | D | N | S | K |

| CDRs(Kabat) | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 |
|---|---|---|---|---|---|---|---|---|---|---|---|---|---|---|---|---|---|---|---|
| Number(Kabat) | H76 | H77 | H78 | H79 | H80 | H81 | H82 | H82A | H82B | H82C | H83 | H84 | H85 | H86 | H87 | H88 | H89 | H90 | H91 |
| AMINO ACID | N | T | L | F | L | Q | M | N | S | L | R | A | E | D | T | A | V | Y | Y |

| CDRs(Kabat) | HFR3 | HFR3 | HFR3 | CDR-H3 | CDR-H3 | CDR-H3 | CDR-H3 | HFR4 | HFR4 | HFR4 | HFR4 | HFR4 | HFR4 | HFR4 | HFR4 | HFR4 | HFR4 | HFR4 |
|---|---|---|---|---|---|---|---|---|---|---|---|---|---|---|---|---|---|---|
| Number(Kabat) | H92 | H93 | H94 | H95 | H96 | H101 | H102 | H103 | H104 | H105 | H106 | H107 | H108 | H109 | H110 | H111 | H112 | H113 |
| AMINO ACID | C | A | T | N | D | D | Y | W | G | Q | G | T | L | V | T | V | S | S |

# Fig.2

| | L1 | L9 | L10 | L13 | L17 | L19 | L21 | L45 | L58 | L60 | L77 | L79 | L83 | L85 | L100 | L105 | L106 | H11 | H13 | H16 | H21 | H23 | H27 | H49 | H79 | H94 |
|---|---|---|---|---|---|---|---|---|---|---|---|---|---|---|---|---|---|---|---|---|---|---|---|---|---|---|
| WILD TYPE | E | A | T | L | E | A | L | R | I | A | S | E | F | V | Q | E | I | V | Q | R | D | K | I | A | F | T |
| AMINO ACID OF HIGHEST APPEARANCE FREQUENCY | Q | S | S | A | Q | V | I | K | V | D | G | Q | E | D | G | T | V | L | K | G | S | A | F | G | Y | R |
| FIRST LIBRARY | ○ | ○ | | ○ | ○ | | ○ | ○ | | ○ | ○ | ○ | | ○ | ○ | | | | | ○ | ○ | ○ | ○ | ○ | ○ | ○ |
| SECOND LIBRARY | | | □ | □ | □ | □ | □ | □ | □ | | | □ | □ | | □ | □ | □ | □ | □ | | | | □ | □ | | |

*Fig.3*

## *Fig.4*

## Fig.5A

| | L1 | L9 | L13 | L17 | L21 | L45 | L60 | L77 | L79 | L85 | L100 | H16 | H21 | H23 | H27 | H49 | H79 | H94 |
|---|---|---|---|---|---|---|---|---|---|---|---|---|---|---|---|---|---|---|
| | | | | | | | | SEQUENCE | | | | | | | | | | |
| WILD TYPE | E | A | L | E | L | R | A | S | E | V | Q | R | D | K | I | A | F | T |
| CASE 1 C40_1-1 | Q | A | L | E | I | K | D | S | Q | D | G | G | S | K | F | G | F | T |
| C40_1-2 | Q | A | L | E | I | K | A | S | Q | D | G | G | S | K | F | G | F | T |
| C40_1-3 | Q | A | L | E | L | K | D | S | Q | D | G | G | S | K | F | G | F | T |
| C40_1-4 | Q | A | A | E | L | K | A | S | E | D | G | G | S | K | F | G | F | T |
| C40_1-5 | Q | A | A | Q | L | K | A | S | E | D | G | G | S | K | F | A | F | T |
| CASE 2 C40_2-1 | E | A | L | E | I | R | D | S | E | D | G | G | S | K | I | A | Y | T |
| C40_2-2 | E | A | L | E | I | R | D | S | E | D | G | G | S | K | I | G | Y | T |
| C40_2-3 | E | A | L | E | I | R | D | G | E | D | G | G | S | K | I | A | Y | T |
| C40_2-4 | E | A | L | E | I | R | D | G | E | D | G | G | S | K | I | G | Y | T |
| C40_2-5 | E | A | A | E | L | K | D | S | E | V | G | G | D | K | I | A | Y | T |
| C40_2-6 | E | A | A | E | L | K | D | S | E | D | G | G | D | K | I | A | Y | T |
| C40_2-7 | E | A | A | E | I | K | D | S | E | V | G | G | S | K | I | G | Y | T |
| C40_2-8 | E | A | A | E | L | K | D | G | E | D | G | G | D | K | I | A | Y | T |
| C40_2-9 | E | A | L | Q | L | R | D | G | Q | V | G | R | S | K | F | A | F | T |
| CASE 3 C40_3-1 | Q | A | L | Q | L | R | D | G | Q | V | G | R | S | K | I | A | F | T |
| C40_3-2 | E | A | L | Q | L | R | D | G | Q | V | G | R | S | K | I | A | F | T |
| C40_3-3 | Q | A | A | Q | L | K | A | S | E | D | G | G | S | K | F | G | F | T |
| C40_3-4 | Q | A | A | Q | L | K | A | S | E | D | G | G | D | K | I | G | F | T |
| C40-3-5 | Q | A | A | Q | L | K | A | G | E | D | G | G | D | K | F | G | F | T |

EP 4 625 420 A1

## Fig.5B

| | | | | | | | | | SEQUENCE | | | | | | | | | |
|---|---|---|---|---|---|---|---|---|---|---|---|---|---|---|---|---|---|---|
| | | L1 | L9 | L13 | L17 | L21 | L45 | L60 | L77 | L79 | L85 | L100 | H16 | H21 | H23 | H27 | H49 | H79 | H94 |
| WILD TYPE | | E | A | L | E | L | R | A | S | E | V | Q | R | D | K | I | A | F | T |
| CASE 1 | C50_1-1 | D | A | L | E | I | N | A | S | Q | D | Q | G | S | A | F | G | Y | T |
| | C50_1-2 | E | A | L | E | I | R | A | S | Q | D | Q | G | S | A | F | G | Y | T |
| | C50_1-3 | E | A | L | E | L | K | A | S | Q | D | Q | G | S | A | F | G | Y | T |
| | C50_1-4 | D | A | L | E | L | K | A | S | Q | D | Q | G | S | A | F | G | Y | T |
| | C50_1-5 | Q | A | L | E | L | R | A | S | Q | D | Q | G | S | A | F | G | F | T |
| | C50_1-6 | E | A | L | E | L | N | A | S | Q | D | Q | G | S | K | F | G | Y | T |
| | C50_1-7 | E | A | L | E | L | R | A | S | Q | D | Q | G | S | A | F | G | F | T |
| | C50_1-8 | E | A | L | E | L | K | A | S | Q | D | Q | G | S | K | F | G | F | T |
| | C50_1-9 | E | A | L | E | L | R | A | S | Q | D | Q | G | S | A | F | G | Y | T |
| | C50_1-10 | Q | A | L | E | L | K | A | S | Q | D | Q | G | S | A | F | G | Y | T |
| | C50_1-11 | E | A | L | E | L | N | A | S | Q | D | Q | G | S | A | F | G | Y | T |
| | C50_1-12 | Q | A | L | E | L | R | A | S | Q | D | Q | G | S | A | F | G | Y | T |
| | C50_1-13 | Q | A | L | E | L | K | A | S | Q | D | Q | G | S | A | F | G | F | T |
| | C50_1-14 | E | S | A | Q | I | R | D | S | Q | D | G | G | D | K | F | G | Y | R |
| | C50_1-15 | E | S | A | Q | I | R | D | S | Q | Y | G | G | S | K | F | G | Y | T |
| | C50_1-16 | E | S | A | Q | I | R | D | S | Q | D | G | G | D | K | F | G | Y | T |
| | C50_1-17 | E | S | A | Q | I | R | D | S | Q | D | G | G | S | K | F | G | Y | T |
| | C50_1-18 | E | S | A | Q | I | R | D | S | Q | D | G | G | D | E | F | G | Y | T |
| | C50_1-19 | E | S | A | Q | I | R | D | S | Q | D | G | G | N | K | F | G | Y | T |
| | C50_1-20 | E | S | A | Q | I | R | D | S | Q | D | Q | G | S | K | F | G | Y | T |
| CASE 3 | C50_3-1 | Q | A | L | Q | L | R | A | S | Q | D | G | G | S | K | F | G | Y | T |
| | C50_3-2 | Q | A | L | E | L | R | A | S | Q | D | R | G | S | K | I | G | Y | T |
| | C50_3-3 | Q | A | L | Q | L | R | A | S | Q | D | G | R | S | K | I | G | Y | T |
| | C50_3-4 | Q | A | L | E | L | R | A | S | Q | D | G | R | S | K | F | G | Y | T |
| | C50_3-5 | Q | A | L | E | L | R | A | S | Q | D | G | G | S | K | F | G | Y | T |
| | C50_3-6 | Q | A | L | Q | L | R | A | S | Q | D | G | G | S | K | I | G | Y | T |
| | C50_3-7 | Q | S | A | Q | I | R | D | G | Q | D | G | G | S | K | F | G | Y | T |
| | C50_3-8 | E | S | A | Q | I | R | D | G | Q | D | G | G | S | K | F | G | Y | T |
| | C50_3-9 | Q | A | L | E | L | R | A | S | Q | D | G | G | S | K | I | G | Y | T |

Fig.6

## Fig.7

| | Ranking | | SEQUENCE | | | | | | | | | | | | | | | | | | |
|---|---|---|---|---|---|---|---|---|---|---|---|---|---|---|---|---|---|---|---|---|---|
| | | | L1 | L9 | L13 | L17 | L21 | L45 | L60 | L77 | L79 | L85 | L100 | H16 | H21 | H23 | H27 | H49 | H79 | H94 |
| WILD TYPE | | | E | A | L | E | L | R | A | S | E | V | Q | R | D | K | I | A | F | T |
| CASE 1 | Variant 1 | 1 | E | S | A | Q | I | R | D | S | Q | D | G | G | S | K | F | G | Y | T |
| | Variant 2 | 2 | E | S | A | Q | I | R | D | S | Q | D | Q | G | S | K | F | G | Y | T |
| | Variant 3 | 3 | Q | A | L | E | L | R | A | S | Q | D | Q | G | S | A | F | G | Y | T |
| | Variant 4 | 4 | Q | A | L | E | L | R | A | S | Q | D | Q | G | S | A | F | G | F | T |
| | Variant 5 | 5 | E | S | A | Q | I | R | A | S | Q | D | G | G | S | K | F | G | Y | T |
| CASE 3 | Variant 6 | 1 | Q | S | A | Q | I | R | D | G | Q | D | G | G | S | K | F | G | Y | T |
| | Variant 7 | 2 | Q | S | A | Q | L | R | D | G | Q | D | G | G | S | K | F | G | Y | T |
| | Variant 8 | 3 | Q | S | A | Q | I | R | D | G | Q | D | Q | G | S | K | F | G | Y | T |
| | Variant 9 | 4 | Q | S | A | Q | L | K | A | S | Q | D | G | G | S | A | F | G | F | T |
| | Variant 10 | 5 | Q | S | A | Q | L | R | A | G | Q | D | G | G | S | K | F | G | Y | T |

EP 4 625 420 A1

# Fig.8A

| | | Ranking | AMOUNT PREPARED (mg/2L OF MEDIUM) | Tm (°C) | Kd (nM) | IMMUNOGENICITY (Bind to MHCs) | |
|---|---|---|---|---|---|---|---|
| | | | | | | Strong | Weak |
| WILD TYPE | | | 1.3 | 59.5 | 1.9-8.2 | 19 | 42 |
| Variant 1 | CASE 1 | 1 | 5.6 | 64.0 | 5.8 | 10 | 36 |
| Variant 2 | | 2 | 23.1 | 65.0 | 2.0-5.3 | 10 | 36 |
| Variant 3 | | 3 | 6.3 | 60.0 | 5.6 | 10 | 32 |
| Variant 4 | | 4 | 1.2 | 60.0 | 3.6 | 12 | 36 |
| Variant 5 | | 5 | 1.6 | 64.5 | 2.3 | 10 | 37 |
| Variant 6 | CASE 3 | 1 | 2.5 | 63.0 | 5.8 | 10 | 36 |
| Variant 7 | | 2 | 23.3 | 56.0-58.0 | 0.67 | 10 | 36 |
| Variant 8 | | 3 | 5.2 | 64.0 | 2.6 | 10 | 36 |
| Variant 9 | | 4 | - | - | - | 12 | 41 |
| Variant 10 | | 5 | 5.3 | 62.0 | 2.2 | 10 | 37 |

# Fig.8B

| | | AMOUNT PREPARED (mg/2L OF MEDIUM) | Tm (°C) | WSS | Kd (nM) | IMMUNOGENICITY (Bind to MHCs) | |
|---|---|---|---|---|---|---|---|
| | | | | | | Strong | Weak |
| WILD TYPE | | 92.5 | 68.02 | 2.57 | 3.10 | 19 | 42 |
| Variant6 | CASE 3 | 251.9 | 72.20 | − | 1.16 | 10 | 36 |
| C40_2-1 | CASE 2 | 287.2 | 71.73 | 6.80 | 3.65 | 13 | 36 |
| C40_2-2 | | 253.2 | 71.49 | 6.21 | 3.37 | 13 | 36 |
| C40_2-3 | | 228.7 | 71.84 | 7.07 | 3.64 | 10 | 33 |
| C40_2-4 | | 276.0 | 71.61 | 6.70 | 3.31 | 10 | 33 |

Fig.9

# Fig.10A

| | L1 | L9 | L10 | L13 | L17 | L19 | L21 | L45 | L58 | L60 | L77 | L79 | L83 | L85 | L100 | L105 | L106 | H11 | H13 | H16 | H21 | H23 | H27 | H49 | H79 | H94 |
|---|---|---|---|---|---|---|---|---|---|---|---|---|---|---|---|---|---|---|---|---|---|---|---|---|---|---|
| WILD TYPE | E | A | T | L | E | A | L | R | I | A | S | E | F | V | Q | E | I | V | Q | R | D | K | I | A | F | T |
| INTRODUCED MUTATION | Q | S | S | A | Q | V | I | K | V | D | G | Q | E | D | G | T | V | L | K | G | S | A | F | G | Y | R |
| | | | | | | | | | | | | | | | | | | | | | | | | | | |
| (1) | | | | ○ | | | | | | | | | | ○ | | | | | | ○ | ○ | | | ○ | | |
| (2) | | | | | | | | | | ○ | | | | | | | | | | | | | ○ | | ○ | |
| (3) | ○ | ○ | | | | | | | | | ○ | | | | ○ | | | | | | | ○ | | | | ○ |
| (4) | | | | | | | | | | ○ | | | | ○ | | | | | | ○ | ○ | | | | ○ | |
| CONSENSUS SEQUENCE | | | | | | | | | | D | | | | D | | | | | | G | S | | | | Y | |

EP 4 625 420 A1

# Fig.10B

| | AMOUNT PREPARED (mg/2L OF MEDIUM) | Tm (°C) | WSS | Kd (nM) |
|---|---|---|---|---|
| scFv | | | | |
| WILD TYPE | 1.3 | 59.5 | – | 1.9-8.2 |
| MUTANT IN WHICH CONSENSUS SEQUENCE WAS MUTATED | 6.2 | 62.0 | – | 3.13 |
| FULL-LENGTH ANTIBODY | | | | |
| WILD TYPE | 92.5 | 65.0 | 2.81 | 3.10 |
| MUTANT IN WHICH CONSENSUS SEQUENCE WAS MUTATED | 329.3 | 64.3 | 6.11 | 2.39 |

# Fig.10C

O WILD-TYPE IgG

● MUTANT IN WHICH CONSENSUS
SEQUENCE WAS MUTATED

EP 4 625 420 A1

## Fig.10D(a)

| CDRs(Kabat) | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 |
|---|---|---|---|---|---|---|---|---|---|---|---|---|---|---|---|---|---|---|---|---|
| Number(Kabat) | H1 | H2 | H3 | H4 | H5 | H6 | H7 | H8 | H9 | H10 | H11 | H12 | H13 | H14 | H15 | H16 | H17 | H18 | H19 | H20 |
| Amino acid | Q | V | E | L | V | E | S | G | G | G | V | V | Q | P | G | R | S | Q | R | L |

| CDRs(Kabat) | HFR1 | HFR1 | HFR1 | HFR1 | HFR1 | HFR1 | HFR1 | HFR1 | HFR1 | HFR1 | CDR-H1 | CDR-H1 | CDR-H1 | CDR-H1 | CDR-H1 | HFR2 | HFR2 | HFR2 | HFR2 | HFR2 |
|---|---|---|---|---|---|---|---|---|---|---|---|---|---|---|---|---|---|---|---|---|
| Number(Kabat) | H21 | H22 | H23 | H24 | H25 | H26 | H27 | H28 | H29 | H30 | H31 | H32 | H33 | H34 | H35 | H36 | H37 | H38 | H39 | H40 |
| Amino acid | S | C | A | A | S | G | F | T | F | S | S | Y | G | M | H | W | V | R | Q | A |

| CDRs(Kabat) | HFR2 | HFR2 | HFR2 | HFR2 | HFR2 | HFR2 | HFR2 | HFR2 | HFR2 | CDR-H2 | CDR-H2 | CDR-H2 | CDR-H2 | CDR-H2 | CDR-H2 | CDR-H2 | CDR-H2 | CDR-H2 | CDR-H2 | CDR-H2 |
|---|---|---|---|---|---|---|---|---|---|---|---|---|---|---|---|---|---|---|---|---|
| Number(Kabat) | H41 | H42 | H43 | H44 | H45 | H46 | H47 | H48 | H49 | H50 | H51 | H52 | H52A | H53 | H54 | H55 | H56 | H57 | H58 | H59 |
| Amino acid | P | G | K | G | L | E | W | V | A | I | I | W | F | D | G | S | S | T | Y | Y |

| CDRs(Kabat) | CDR-H2 | CDR-H2 | CDR-H2 | CDR-H2 | CDR-H2 | CDR-H2 | HFR3 | HFR3 | HFR3 | HFR3 | HFR3 | HFR3 | HFR3 | HFR3 | HFR3 | HFR3 | HFR3 | HFR3 | HFR3 | HFR3 |
|---|---|---|---|---|---|---|---|---|---|---|---|---|---|---|---|---|---|---|---|---|
| Number(Kabat) | H60 | H61 | H62 | H63 | H64 | H65 | H66 | H67 | H68 | H69 | H70 | H71 | H72 | H73 | H74 | H75 | H76 | H77 | H78 | H79 |
| Amino acid | A | D | S | V | R | G | R | F | T | I | S | R | D | N | S | K | N | T | L | Y |

| CDRs(Kabat) | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | CDR-H3 | CDR-H3 |
|---|---|---|---|---|---|---|---|---|---|---|---|---|---|---|---|---|---|---|---|---|
| Number(Kabat) | H80 | H81 | H82 | H82A | H82B | H82C | H83 | H84 | H85 | H86 | H87 | H88 | H89 | H90 | H91 | H92 | H93 | H94 | H95 | H96 |
| Amino acid | L | Q | M | N | S | L | R | A | E | D | T | A | V | Y | F | C | A | R | E | L |

| CDRs(Kabat) | CDR-H3 | CDR-H3 | CDR-H3 | CDR-H3 | CDR-H3 | CDR-H3 | CDR-H3 | HFR4 | HFR4 | HFR4 | HFR4 | HFR4 | HFR4 | HFR4 | HFR4 | HFR4 | HFR4 | HFR4 |
|---|---|---|---|---|---|---|---|---|---|---|---|---|---|---|---|---|---|---|
| Number(Kabat) | H97 | H98 | H99 | H100 | H100A | H101 | H102 | H103 | H104 | H105 | H106 | H107 | H108 | H109 | H110 | H111 | H112 | H113 |
| Amino acid | G | R | R | Y | F | D | L | W | G | R | G | T | L | V | S | V | S | S |

| CDRs(Kabat) | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 |
|---|---|---|---|---|---|---|---|---|---|---|---|---|---|---|---|---|---|---|
| Number(Kabat) | L1 | L2 | L3 | L4 | L5 | L6 | L7 | L8 | L9 | L10 | L11 | L12 | L13 | L14 | L15 | L16 | L17 | L18 |
| Amino acid | E | I | V | L | T | Q | S | P | A | T | L | S | L | S | P | G | E | R |

| CDRs(Kabat) | LFR1 | LFR1 | LFR1 | LFR1 | LFR1 | CDR-L1 | CDR-L1 | CDR-L1 | CDR-L1 | CDR-L1 | CDR-L1 | CDR-L1 | CDR-L1 | CDR-L1 | CDR-L1 | CDR-L1 | LFR2 | LFR2 |
|---|---|---|---|---|---|---|---|---|---|---|---|---|---|---|---|---|---|---|
| Number(Kabat) | L19 | L20 | L21 | L22 | L23 | L24 | L25 | L26 | L27 | L28 | L29 | L30 | L31 | L32 | L33 | L34 | L35 | L36 |
| Amino acid | A | T | L | S | C | R | A | S | Q | S | V | S | S | Y | L | A | W | Y |

| CDRs(Kabat) | LFR2 | LFR2 | LFR2 | LFR2 | LFR2 | LFR2 | LFR2 | LFR2 | LFR2 | LFR2 | LFR2 | LFR2 | LFR2 | CDR-L2 | CDR-L2 | CDR-L2 | CDR-L2 | CDR-L2 |
|---|---|---|---|---|---|---|---|---|---|---|---|---|---|---|---|---|---|---|
| Number(Kabat) | L37 | L38 | L39 | L40 | L41 | L42 | L43 | L44 | L45 | L46 | L47 | L48 | L49 | L50 | L51 | L52 | L53 | L54 |
| Amino acid | Q | Q | K | P | G | Q | A | P | R | L | L | I | Y | D | A | S | K | R |

| CDRs(Kabat) | CDR-L2 | CDR-L2 | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 |
|---|---|---|---|---|---|---|---|---|---|---|---|---|---|---|---|---|---|---|
| Number(Kabat) | L55 | L56 | L57 | L58 | L59 | L60 | L61 | L62 | L63 | L64 | L65 | L66 | L67 | L68 | L69 | L70 | L71 | L72 |
| Amino acid | A | T | G | I | P | A | R | F | S | G | S | G | S | G | T | D | F | T |

| CDRs(Kabat) | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 | CDR-L3 | CDR-L3 |
|---|---|---|---|---|---|---|---|---|---|---|---|---|---|---|---|---|---|---|
| Number(Kabat) | L73 | L74 | L75 | L76 | L77 | L78 | L79 | L80 | L81 | L82 | L83 | L84 | L85 | L86 | L87 | L88 | L89 | L90 |
| Amino acid | L | T | I | S | S | L | E | P | E | D | F | A | V | Y | Y | C | Q | Q |

| CDRs(Kabat) | CDR-L3 | CDR-L3 | CDR-L3 | CDR-L3 | CDR-L3 | CDR-L3 | CDR-L3 | CDR-L3 | LFR4 | LFR4 | LFR4 | LFR4 | LFR4 | LFR4 | LFR4 | LFR4 | LFR4 | LFR4 |
|---|---|---|---|---|---|---|---|---|---|---|---|---|---|---|---|---|---|---|
| Number(Kabat) | L91 | L92 | L93 | L94 | L95 | L95A | L96 | L97 | L98 | L99 | L100 | L101 | L102 | L103 | L104 | L105 | L106 | L107 |
| Amino acid | R | S | K | W | P | P | W | T | F | G | Q | G | T | K | V | E | S | K |

# Fig.10D(b)

| CDRs(Kabat) | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 |
|---|---|---|---|---|---|---|---|---|---|---|---|---|---|---|---|---|---|---|---|---|
| Number(Kabat) | H1 | H2 | H3 | H4 | H5 | H6 | H7 | H8 | H9 | H10 | H11 | H12 | H13 | H14 | H15 | H16 | H17 | H18 | H19 | H20 |
| Amino acid | Q | V | E | L | V | E | S | G | G | G | V | V | Q | P | G | G | S | Q | R | L |

| CDRs(Kabat) | HFR1 | HFR1 | HFR1 | HFR1 | HFR1 | HFR1 | HFR1 | HFR1 | HFR1 | HFR1 | CDR-H1 | CDR-H1 | CDR-H1 | CDR-H1 | CDR-H1 | HFR2 | HFR2 | HFR2 | HFR2 | HFR2 |
|---|---|---|---|---|---|---|---|---|---|---|---|---|---|---|---|---|---|---|---|---|
| Number(Kabat) | H21 | H22 | H23 | H24 | H25 | H26 | H27 | H28 | H29 | H30 | H31 | H32 | H33 | H34 | H35 | H36 | H37 | H38 | H39 | H40 |
| Amino acid | S | C | A | A | S | G | F | T | F | S | S | Y | G | M | H | W | V | R | Q | A |

| CDRs(Kabat) | HFR2 | HFR2 | HFR2 | HFR2 | HFR2 | HFR2 | HFR2 | HFR2 | HFR2 | CDR-H2 | CDR-H2 | CDR-H2 | CDR-H2 | CDR-H2 | CDR-H2 | CDR-H2 | CDR-H2 | CDR-H2 | CDR-H2 | CDR-H2 |
|---|---|---|---|---|---|---|---|---|---|---|---|---|---|---|---|---|---|---|---|---|
| Number(Kabat) | H41 | H42 | H43 | H44 | H45 | H46 | H47 | H48 | H49 | H50 | H51 | H52 | H52A | H53 | H54 | H55 | H56 | H57 | H58 | H59 |
| Amino acid | P | G | K | G | L | E | W | V | A | I | I | W | F | D | G | S | S | T | Y | Y |

| CDRs(Kabat) | CDR-H2 | CDR-H2 | CDR-H2 | CDR-H2 | CDR-H2 | CDR-H2 | HFR3 | HFR3 | HFR3 | HFR3 | HFR3 | HFR3 | HFR3 | HFR3 | HFR3 | HFR3 | HFR3 | HFR3 | HFR3 | HFR3 |
|---|---|---|---|---|---|---|---|---|---|---|---|---|---|---|---|---|---|---|---|---|
| Number(Kabat) | H60 | H61 | H62 | H63 | H64 | H65 | H66 | H67 | H68 | H69 | H70 | H71 | H72 | H73 | H74 | H75 | H76 | H77 | H78 | H79 |
| Amino acid | A | D | S | V | R | G | R | F | T | I | S | R | D | N | S | K | N | T | L | Y |

| CDRs(Kabat) | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | CDR-H3 | CDR-H3 |
|---|---|---|---|---|---|---|---|---|---|---|---|---|---|---|---|---|---|---|---|---|
| Number(Kabat) | H80 | H81 | H82 | H82A | H82B | H82C | H83 | H84 | H85 | H86 | H87 | H88 | H89 | H90 | H91 | H92 | H93 | H94 | H95 | H96 |
| Amino acid | L | Q | M | N | S | L | R | A | E | D | T | A | V | Y | F | C | A | R | E | L |

| CDRs(Kabat) | CDR-H3 | CDR-H3 | CDR-H3 | CDR-H3 | CDR-H3 | CDR-H3 | CDR-H3 | HFR4 | HFR4 | HFR4 | HFR4 | HFR4 | HFR4 | HFR4 | HFR4 | HFR4 | HFR4 |
|---|---|---|---|---|---|---|---|---|---|---|---|---|---|---|---|---|---|
| Number(Kabat) | H97 | H98 | H99 | H100 | H100A | H101 | H102 | H103 | H104 | H105 | H106 | H107 | H108 | H109 | H110 | H111 | H112 | H113 |
| Amino acid | G | R | R | Y | F | D | L | W | G | R | G | T | L | V | S | V | S | S |

| CDRs(Kabat) | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 |
|---|---|---|---|---|---|---|---|---|---|---|---|---|---|---|---|---|---|---|
| Number(Kabat) | L1 | L2 | L3 | L4 | L5 | L6 | L7 | L8 | L9 | L10 | L11 | L12 | L13 | L14 | L15 | L16 | L17 | L18 |
| Amino acid | E | I | V | L | T | Q | S | P | A | T | L | S | L | S | P | G | E | R |

| CDRs(Kabat) | LFR1 | LFR1 | LFR1 | LFR1 | LFR1 | CDR-L1 | CDR-L1 | CDR-L1 | CDR-L1 | CDR-L1 | CDR-L1 | CDR-L1 | CDR-L1 | CDR-L1 | CDR-L1 | CDR-L1 | LFR2 | LFR2 |
|---|---|---|---|---|---|---|---|---|---|---|---|---|---|---|---|---|---|---|
| Number(Kabat) | L19 | L20 | L21 | L22 | L23 | L24 | L25 | L26 | L27 | L28 | L29 | L30 | L31 | L32 | L33 | L34 | L35 | L36 |
| Amino acid | A | T | L | S | C | R | A | S | Q | S | V | S | S | Y | L | A | W | Y |

| CDRs(Kabat) | LFR2 | LFR2 | LFR2 | LFR2 | LFR2 | LFR2 | LFR2 | LFR2 | LFR2 | LFR2 | LFR2 | LFR2 | LFR2 | CDR-L2 | CDR-L2 | CDR-L2 | CDR-L2 | CDR-L2 |
|---|---|---|---|---|---|---|---|---|---|---|---|---|---|---|---|---|---|---|
| Number(Kabat) | L37 | L38 | L39 | L40 | L41 | L42 | L43 | L44 | L45 | L46 | L47 | L48 | L49 | L50 | L51 | L52 | L53 | L54 |
| Amino acid | Q | Q | K | P | G | Q | A | P | R | L | L | I | Y | D | A | S | K | R |

| CDRs(Kabat) | CDR-L2 | CDR-L2 | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 |
|---|---|---|---|---|---|---|---|---|---|---|---|---|---|---|---|---|---|---|
| Number(Kabat) | L55 | L56 | L57 | L58 | L59 | L60 | L61 | L62 | L63 | L64 | L65 | L66 | L67 | L68 | L69 | L70 | L71 | L72 |
| Amino acid | A | T | G | I | P | D | R | F | S | G | S | G | S | G | T | D | F | T |

| CDRs(Kabat) | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 | CDR-L3 | CDR-L3 |
|---|---|---|---|---|---|---|---|---|---|---|---|---|---|---|---|---|---|---|
| Number(Kabat) | L73 | L74 | L75 | L76 | L77 | L78 | L79 | L80 | L81 | L82 | L83 | L84 | L85 | L86 | L87 | L88 | L89 | L90 |
| Amino acid | L | T | I | S | S | L | E | P | E | D | F | A | D | Y | Y | C | Q | Q |

| CDRs(Kabat) | CDR-L3 | CDR-L3 | CDR-L3 | CDR-L3 | CDR-L3 | CDR-L3 | CDR-L3 | CDR-L3 | LFR4 | LFR4 | LFR4 | LFR4 | LFR4 | LFR4 | LFR4 | LFR4 | LFR4 | LFR4 |
|---|---|---|---|---|---|---|---|---|---|---|---|---|---|---|---|---|---|---|
| Number(Kabat) | L91 | L92 | L93 | L94 | L95 | L95A | L96 | L97 | L98 | L99 | L100 | L101 | L102 | L103 | L104 | L105 | L106 | L107 |
| Amino acid | R | S | K | W | P | P | W | T | F | G | Q | G | T | K | V | E | S | K |

# Fig.10D(c)

| CDRs(Kabat) | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 | HFR1 |
|---|---|---|---|---|---|---|---|---|---|---|---|---|---|---|---|---|---|---|---|---|
| Number(Kabat) | H1 | H2 | H3 | H4 | H5 | H6 | H7 | H8 | H9 | H10 | H11 | H12 | H13 | H14 | H15 | H16 | H17 | H18 | H19 | H20 |
| Amino acid | Q | V | Q | L | V | E | S | G | G | G | V | V | Q | P | G | G | S | L | R | L |

| CDRs(Kabat) | HFR1 | HFR1 | HFR1 | HFR1 | HFR1 | HFR1 | HFR1 | HFR1 | HFR1 | HFR1 | CDR-H1 | CDR-H1 | CDR-H1 | CDR-H1 | CDR-H1 | HFR2 | HFR2 | HFR2 | HFR2 | HFR2 |
|---|---|---|---|---|---|---|---|---|---|---|---|---|---|---|---|---|---|---|---|---|
| Number(Kabat) | H21 | H22 | H23 | H24 | H25 | H26 | H27 | H28 | H29 | H30 | H31 | H32 | H33 | H34 | H35 | H36 | H37 | H38 | H39 | H40 |
| Amino acid | S | C | K | A | S | G | I | T | F | S | S | Y | G | M | H | W | V | R | Q | A |

| CDRs(Kabat) | HFR2 | HFR2 | HFR2 | HFR2 | HFR2 | HFR2 | HFR2 | HFR2 | HFR2 | CDR-H2 | CDR-H2 | CDR-H2 | CDR-H2 | CDR-H2 | CDR-H2 | CDR-H2 | CDR-H2 | CDR-H2 | CDR-H2 | CDR-H2 |
|---|---|---|---|---|---|---|---|---|---|---|---|---|---|---|---|---|---|---|---|---|
| Number(Kabat) | H41 | H42 | H43 | H44 | H45 | H46 | H47 | H48 | H49 | H50 | H51 | H52 | H52A | H53 | H54 | H55 | H56 | H57 | H58 | H59 |
| Amino acid | P | G | K | G | L | E | W | V | A | I | I | W | F | D | G | S | S | T | Y | Y |

| CDRs(Kabat) | CDR-H2 | CDR-H2 | CDR-H2 | CDR-H2 | CDR-H2 | CDR-H2 | HFR3 | HFR3 | HFR3 | HFR3 | HFR3 | HFR3 | HFR3 | HFR3 | HFR3 | HFR3 | HFR3 | HFR3 | HFR3 | HFR3 |
|---|---|---|---|---|---|---|---|---|---|---|---|---|---|---|---|---|---|---|---|---|
| Number(Kabat) | H60 | H61 | H62 | H63 | H64 | H65 | H66 | H67 | H68 | H69 | H70 | H71 | H72 | H73 | H74 | H75 | H76 | H77 | H78 | H79 |
| Amino acid | A | D | S | V | R | G | R | F | T | I | S | R | D | N | S | K | N | T | L | Y |

| CDRs(Kabat) | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | HFR3 | CDR-H3 | CDR-H3 |
|---|---|---|---|---|---|---|---|---|---|---|---|---|---|---|---|---|---|---|---|---|
| Number(Kabat) | H80 | H81 | H82 | H82A | H82B | H82C | H83 | H84 | H85 | H86 | H87 | H88 | H89 | H90 | H91 | H92 | H93 | H94 | H95 | H96 |
| Amino acid | L | Q | M | N | S | L | R | A | E | D | T | A | V | Y | Y | C | A | T | E | L |

| CDRs(Kabat) | CDR-H3 | CDR-H3 | CDR-H3 | CDR-H3 | CDR-H3 | CDR-H3 | CDR-H3 | HFR4 | HFR4 | HFR4 | HFR4 | HFR4 | HFR4 | HFR4 | HFR4 | HFR4 | HFR4 | HFR4 |
|---|---|---|---|---|---|---|---|---|---|---|---|---|---|---|---|---|---|---|
| Number(Kabat) | H97 | H98 | H99 | H100 | H100A | H101 | H102 | H103 | H104 | H105 | H106 | H107 | H108 | H109 | H110 | H111 | H112 | H113 |
| Amino acid | G | R | R | Y | F | D | L | W | G | Q | G | T | L | V | T | V | S | S |

| CDRs(Kabat) | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 |
|---|---|---|---|---|---|---|---|---|---|---|---|---|---|---|---|---|---|---|
| Number(Kabat) | L1 | L2 | L3 | L4 | L5 | L6 | L7 | L8 | L9 | L10 | L11 | L12 | L13 | L14 | L15 | L16 | L17 | L18 |
| Amino acid | E | I | V | L | T | Q | S | P | A | T | L | S | L | S | P | G | E | R |

| CDRs(Kabat) | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 | LFR1 |
|---|---|---|---|---|---|---|---|---|---|---|---|---|---|---|---|---|---|---|
| Number(Kabat) | L1 | L2 | L3 | L4 | L5 | L6 | L7 | L8 | L9 | L10 | L11 | L12 | L13 | L14 | L15 | L16 | L17 | L18 |
| Amino acid | E | I | V | L | T | Q | S | P | A | T | L | S | L | S | P | G | E | R |

| CDRs(Kabat) | LFR1 | LFR1 | LFR1 | LFR1 | LFR1 | CDR-L1 | CDR-L1 | CDR-L1 | CDR-L1 | CDR-L1 | CDR-L1 | CDR-L1 | CDR-L1 | CDR-L1 | CDR-L1 | CDR-L1 | LFR2 | LFR2 |
|---|---|---|---|---|---|---|---|---|---|---|---|---|---|---|---|---|---|---|
| Number(Kabat) | L19 | L20 | L21 | L22 | L23 | L24 | L25 | L26 | L27 | L28 | L29 | L30 | L31 | L32 | L33 | L34 | L35 | L36 |
| Amino acid | A | T | I | S | C | R | A | S | Q | S | V | S | S | Y | L | A | W | Y |

| CDRs(Kabat) | LFR2 | LFR2 | LFR2 | LFR2 | LFR2 | LFR2 | LFR2 | LFR2 | LFR2 | LFR2 | LFR2 | LFR2 | LFR2 | CDR-L2 | CDR-L2 | CDR-L2 | CDR-L2 | CDR-L2 |
|---|---|---|---|---|---|---|---|---|---|---|---|---|---|---|---|---|---|---|
| Number(Kabat) | L37 | L38 | L39 | L40 | L41 | L42 | L43 | L44 | L45 | L46 | L47 | L48 | L49 | L50 | L51 | L52 | L53 | L54 |
| Amino acid | Q | Q | K | P | G | Q | A | P | R | L | L | I | Y | D | A | S | K | R |

| CDRs(Kabat) | CDR-L2 | CDR-L2 | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 |
|---|---|---|---|---|---|---|---|---|---|---|---|---|---|---|---|---|---|---|
| Number(Kabat) | L55 | L56 | L57 | L58 | L59 | L60 | L61 | L62 | L63 | L64 | L65 | L66 | L67 | L68 | L69 | L70 | L71 | L72 |
| Amino acid | A | T | G | I | P | D | R | F | S | G | S | G | S | G | T | D | F | T |

| CDRs(Kabat) | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 | LFR3 | CDR-L3 | CDR-L3 |
|---|---|---|---|---|---|---|---|---|---|---|---|---|---|---|---|---|---|---|
| Number(Kabat) | L73 | L74 | L75 | L76 | L77 | L78 | L79 | L80 | L81 | L82 | L83 | L84 | L85 | L86 | L87 | L88 | L89 | L90 |
| Amino acid | L | T | I | S | G | L | E | P | E | D | F | A | D | Y | Y | C | Q | Q |

| CDRs(Kabat) | CDR-L3 | CDR-L3 | CDR-L3 | CDR-L3 | CDR-L3 | CDR-L3 | CDR-L3 | CDR-L3 | LFR4 | LFR4 | LFR4 | LFR4 | LFR4 | LFR4 | LFR4 | LFR4 | LFR4 | LFR4 |
|---|---|---|---|---|---|---|---|---|---|---|---|---|---|---|---|---|---|---|
| Number(Kabat) | L91 | L92 | L93 | L94 | L95 | L95A | L96 | L97 | L98 | L99 | L100 | L101 | L102 | L103 | L104 | L105 | L106 | L107 |
| Amino acid | R | S | K | W | P | P | W | T | F | G | G | G | T | K | V | E | S | K |

# Fig.10E

| | AMOUNT PREPARED (mg/2L OF MEDIUM) | Tm (°C) | $EC_{50}(\mu M)$ |
|---|---|---|---|
| WILD TYPE | 1.6 | 59.5 | 0.029 |
| CONSENSUS SEQUENCE | 2.4 | 62.0 | 0.024 |
| C40_2-3 MUTANT SEQUENCE | 2.0 | 65.5 | 0.025 |

## Fig.11

*Fig.12(a)*

SEQUENCE

| | L1 | L9 | L10 | L13 | L17 | L19 | L21 | L45 | L58 | L60 | L77 | L79 | L83 | L85 | L100 | L105 | L106 | H11 | H13 | H16 | H21 | H23 | H27 | H49 | H79 | H94 |
|---|---|---|---|---|---|---|---|---|---|---|---|---|---|---|---|---|---|---|---|---|---|---|---|---|---|---|
| WILD TYPE | E | A | T | L | E | A | L | R | I | A | S | E | F | V | Q | E | I | Q | Q | R | D | K | I | A | F | T |
| CASE 3 | | | | | | | | | | | | | | | | | | | | | | | | | | |
| ML2C3_1 | E | A | T | A | E | A | I | K | V | D | S | Q | F | D | G | T | L | V | Q | G | S | K | F | G | Y | T |
| ML2C3_2 | E | A | T | A | E | A | I | K | V | D | S | Q | F | D | G | T | I | V | Q | R | S | K | F | G | Y | T |
| ML2C3_3 | E | A | T | A | E | A | I | K | V | D | S | Q | F | D | G | T | L | V | Q | G | S | K | F | G | Y | T |
| ML2C3_4 | E | A | T | A | E | A | I | K | V | D | S | Q | F | D | G | T | L | V | Q | R | S | K | F | G | Y | T |
| ML2C3_5 | E | A | T | A | E | A | I | K | V | D | S | Q | F | D | G | T | I | V | Q | G | S | K | F | G | Y | T |
| ML2C3_6 | E | A | T | A | E | A | I | K | V | D | S | Q | F | D | G | E | I | V | Q | G | S | K | F | G | Y | T |
| ML2C3_7 | E | A | T | A | E | A | I | K | V | D | S | Q | F | D | G | T | I | V | Q | R | S | K | F | G | Y | T |
| ML2C3_8 | E | A | T | A | Q | V | I | K | V | D | S | Q | F | D | G | E | V | V | Q | R | S | K | F | G | Y | T |
| ML2C3_9 | E | A | T | A | E | A | I | K | V | D | S | Q | F | D | G | T | V | V | Q | R | S | K | F | G | Y | T |
| ML2C3_10 | E | A | T | A | Q | A | I | K | V | D | S | Q | F | D | G | E | V | V | Q | R | S | K | F | G | Y | T |
| ML2C3_11 | E | A | T | A | Q | V | I | R | V | D | S | Q | F | D | G | E | V | V | Q | R | S | K | F | G | Y | T |
| ML2C3_12 | E | A | T | A | E | A | I | K | V | D | S | Q | F | D | G | T | I | V | Q | R | S | K | F | G | Y | T |
| ML2C3_13 | E | A | T | A | E | A | I | R | V | D | S | Q | F | D | G | T | V | V | Q | R | S | K | F | G | Y | T |
| ML2C3_14 | E | A | T | A | Q | A | I | R | V | D | S | Q | F | D | G | T | I | V | Q | R | S | K | F | G | Y | T |
| ML2C3_15 | E | A | T | A | E | A | I | R | V | D | S | Q | F | D | G | T | V | V | Q | R | S | K | F | G | Y | T |

## Fig.12(b)

| | | L1 | L9 | L10 | L13 | L17 | L19 | L21 | L45 | L58 | L60 | L77 | L79 | L83 | L85 | L100 | L105 | L106 | H11 | H13 | H16 | H21 | H23 | H27 | H49 | H79 | H94 |
|---|---|---|---|---|---|---|---|---|---|---|---|---|---|---|---|---|---|---|---|---|---|---|---|---|---|---|---|
| WILD TYPE | | E | A | T | L | E | A | L | R | I | A | S | E | F | V | Q | E | I | V | Q | R | D | K | I | A | F | T |
| CASE 4 | ML2C4_1 | E | A | S | A | E | V | L | K | V | D | S | Q | E | D | G | E | V | V | Q | G | S | K | I | G | Y | T |
| | ML2C4_2 | E | A | S | A | E | V | I | K | V | D | S | Q | E | D | G | E | V | V | Q | G | S | K | F | G | Y | T |
| | ML2C4_3 | E | A | S | A | E | V | L | K | V | D | S | Q | E | D | G | E | V | V | Q | G | S | K | F | G | Y | T |
| | ML2C4_4 | E | A | S | A | Q | V | L | K | V | D | S | Q | E | D | G | E | V | V | Q | G | S | K | F | G | Y | T |
| | ML2C4_5 | E | A | S | A | E | A | I | K | V | D | S | Q | E | D | G | E | V | V | Q | G | S | K | F | G | Y | T |
| | ML2C4_6 | E | A | S | A | E | V | I | K | V | D | S | Q | E | D | G | E | V | V | Q | G | S | K | I | G | Y | T |
| | ML2C4_7 | E | A | T | A | E | V | I | K | V | D | S | Q | E | D | G | E | V | V | Q | G | S | K | F | G | Y | T |
| | ML2C4_8 | E | A | T | A | E | V | L | K | V | D | S | Q | E | D | G | E | V | V | Q | G | S | K | I | G | Y | T |
| | ML2C4_9 | E | A | S | A | Q | V | I | K | V | D | S | Q | E | D | G | E | V | V | Q | G | S | K | F | G | Y | T |
| | ML2C4_10 | E | A | T | A | E | V | L | K | V | D | S | Q | E | D | G | E | V | V | Q | G | S | K | F | G | Y | T |
| | ML2C4_11 | E | A | S | A | E | V | I | K | V | D | S | Q | E | D | G | E | V | V | K | G | S | K | F | G | Y | T |
| | ML2C4_12 | E | A | S | A | E | V | L | K | V | D | S | Q | E | D | G | T | V | V | Q | G | S | K | F | G | Y | T |
| | ML2C4_13 | E | A | S | A | Q | V | L | K | V | D | S | Q | E | D | G | T | V | V | Q | G | S | K | F | G | Y | T |
| | ML2C4_14 | E | A | T | A | E | V | I | K | V | D | S | Q | E | D | G | E | V | V | Q | G | S | K | I | G | Y | T |
| | ML2C4_15 | E | A | S | A | Q | V | I | K | V | D | S | Q | E | D | G | E | V | V | K | G | S | K | F | G | Y | T |
| | ML2C4_16 | E | A | T | A | E | V | I | K | V | D | S | Q | E | D | G | E | V | V | K | G | S | K | F | G | Y | T |
| | ML2C4_17 | E | A | T | A | Q | V | L | K | V | D | S | Q | E | D | G | E | V | V | Q | G | S | K | F | G | Y | T |
| | ML2C4_18 | E | A | S | A | Q | V | L | K | V | D | S | Q | E | D | G | E | V | V | Q | G | S | K | I | G | Y | T |
| | ML2C4_19 | E | A | T | A | Q | V | I | K | V | D | S | Q | E | D | G | E | V | V | K | G | S | K | F | G | Y | T |
| | ML2C4_20 | E | A | S | A | E | V | I | K | V | D | S | Q | E | D | G | E | V | V | K | G | S | K | I | G | Y | T |
| | ML2C4_21 | E | A | T | A | Q | V | I | K | V | D | S | Q | E | D | G | E | V | V | Q | G | S | K | F | G | Y | T |
| | ML2C4_22 | E | A | S | A | E | V | L | K | V | D | S | Q | E | D | G | E | V | V | K | G | S | K | F | G | Y | T |
| | ML2C4_23 | E | A | T | A | E | V | I | K | V | D | S | Q | E | D | G | E | V | V | K | G | S | K | I | G | Y | T |
| | ML2C4_24 | E | A | T | A | E | V | I | K | V | D | S | Q | E | D | G | T | V | V | Q | G | S | K | F | G | Y | T |
| | ML2C4_25 | E | A | S | A | Q | V | I | K | V | D | S | Q | E | D | G | E | V | V | K | G | S | K | I | G | Y | T |
| | ML2C4_26 | E | A | S | A | E | V | I | K | V | D | S | Q | E | D | G | E | V | V | Q | G | S | K | F | A | Y | T |
| | ML2C4_27 | E | A | T | A | Q | V | I | K | V | D | S | Q | E | D | G | E | V | V | Q | G | S | K | I | G | Y | T |
| | ML2C4_28 | E | A | S | A | Q | V | L | K | V | D | S | Q | E | D | G | E | V | V | K | G | S | K | I | G | Y | T |

# Fig.12(c)

EP 4 625 420 A1

| | | L1 | L9 | L10 | L13 | L17 | L19 | L21 | L45 | L58 | L60 | L77 | L79 | L83 | L85 | L100 | L105 | L106 | H11 | H13 | H16 | H21 | H23 | H27 | H49 | H79 | H94 |
|---|---|---|---|---|---|---|---|---|---|---|---|---|---|---|---|---|---|---|---|---|---|---|---|---|---|---|---|
| WILD TYPE | | E | A | T | L | E | A | L | R | I | A | S | E | F | V | Q | E | I | V | Q | R | D | K | I | A | F | T |
| CASE 5 | ML2C5_1 | E | A | S | A | Q | V | L | K | V | D | S | Q | E | D | G | E | V | V | Q | G | S | K | F | G | Y | T |
| | ML2C5_2 | E | A | S | A | Q | V | I | K | V | D | S | Q | E | D | G | E | V | V | Q | G | S | K | F | G | Y | T |
| | ML2C5_3 | E | A | S | A | E | V | L | K | V | D | S | Q | E | D | G | E | V | V | Q | G | S | K | F | G | Y | T |
| | ML2C5_4 | E | A | S | A | E | V | I | K | V | D | S | Q | E | D | G | E | V | V | Q | G | S | K | F | G | Y | T |
| | ML2C5_5 | E | A | T | A | E | V | L | K | V | D | S | Q | E | D | G | T | V | V | Q | G | S | K | F | A | Y | T |
| | ML2C5_6 | E | A | S | A | Q | V | I | K | V | D | S | Q | E | D | G | E | V | V | K | G | S | K | F | G | Y | T |
| | ML2C5_7 | E | A | T | A | E | V | I | K | V | D | S | Q | E | D | G | T | V | V | Q | G | S | K | F | A | Y | T |
| | ML2C5_8 | E | A | T | A | E | V | L | K | V | D | S | Q | E | D | G | E | V | V | Q | G | S | K | F | A | Y | T |
| | ML2C5_9 | E | A | S | A | E | V | L | K | V | D | S | Q | E | D | G | E | V | V | Q | G | S | K | F | A | Y | T |
| | ML2C5_10 | E | A | T | A | E | V | L | K | V | D | S | Q | E | D | G | E | V | V | Q | G | S | K | F | G | Y | T |
| | ML2C5_11 | E | A | T | A | Q | V | I | K | V | D | S | Q | E | D | G | E | V | V | K | G | S | K | F | G | Y | T |
| | ML2C5_12 | E | A | S | A | Q | V | L | K | V | D | S | Q | E | D | G | T | V | V | Q | G | S | K | F | G | Y | T |
| | ML2C5_13 | E | A | T | A | Q | V | L | K | V | D | S | Q | E | D | G | E | V | V | Q | G | S | K | F | G | Y | T |
| | ML2C5_14 | E | A | T | A | Q | V | I | K | V | D | S | Q | E | D | G | E | V | V | Q | G | S | K | F | G | Y | T |
| | ML2C5_15 | E | A | S | A | E | V | I | K | V | D | S | Q | E | D | G | E | V | V | Q | G | S | K | F | A | F | T |
| | ML2C5_16 | E | A | T | A | E | V | I | K | V | D | S | Q | E | D | G | E | V | V | Q | G | S | K | F | G | F | T |
| | ML2C5_17 | E | A | S | A | E | V | I | K | V | D | S | Q | E | D | G | E | V | V | K | G | S | K | F | G | Y | T |
| | ML2C5_18 | E | A | S | A | Q | V | L | K | V | D | S | Q | E | D | G | E | V | V | Q | G | S | K | F | A | Y | T |
| | ML2C5_19 | E | A | T | A | E | V | I | K | V | D | S | Q | E | D | G | T | V | V | Q | G | S | K | F | G | Y | T |
| | ML2C5_20 | E | A | S | A | Q | V | I | K | V | D | S | Q | E | D | G | E | V | V | Q | G | S | K | F | A | Y | T |
| | ML2C5_21 | E | A | S | A | E | V | L | K | V | D | S | Q | E | D | G | E | V | V | K | G | S | K | F | G | Y | T |
| | ML2C5_22 | E | A | T | A | E | V | I | K | V | D | S | Q | E | D | G | E | V | V | K | G | S | K | F | G | Y | T |
| | ML2C5_23 | E | A | S | A | E | V | L | K | V | D | S | Q | E | D | G | T | V | V | Q | G | S | K | F | G | Y | T |
| | ML2C5_24 | E | A | T | A | Q | V | I | K | V | D | S | Q | E | D | G | E | V | V | Q | G | S | K | F | A | Y | T |
| | ML2C5_25 | E | A | S | A | E | A | I | K | V | D | S | Q | E | D | G | E | V | V | Q | G | S | K | F | G | Y | T |
| | ML2C5_26 | E | A | S | A | E | V | I | K | V | D | S | Q | E | D | G | T | V | V | Q | G | S | K | F | A | Y | T |
| | ML2C5_27 | E | A | S | A | E | V | L | K | V | D | S | Q | E | D | G | E | V | V | Q | G | S | K | I | G | Y | T |
| | ML2C5_28 | E | A | S | A | Q | V | L | K | V | D | S | Q | E | D | G | E | V | V | K | G | S | K | I | G | Y | T |

*Fig.13*

## Fig.14

**Light chain (Kabat numbering)**

| CDRs (Kabat) | Number (Kabat) | AMINO ACID |
|---|---|---|
| LFR1 | L1 | E |
| LFR1 | L2 | I |
| LFR1 | L3 | V |
| LFR1 | L4 | L |
| LFR1 | L5 | T |
| LFR1 | L6 | Q |
| LFR1 | L7 | S |
| LFR1 | L8 | P |
| LFR1 | L9 | A |
| LFR1 | L10 | T |
| LFR1 | L11 | L |
| LFR1 | L12 | S |
| LFR1 | L13 | L |
| LFR1 | L14 | S |
| LFR1 | L15 | P |
| LFR1 | L16 | G |
| LFR1 | L17 | E |
| LFR1 | L18 | R |
| LFR1 | L19 | A |
| LFR1 | L20 | T |
| LFR1 | L21 | L |
| LFR1 | L22 | S |
| LFR1 | L23 | C |
| CDR-L1 | L24 | R |
| CDR-L1 | L25 | A |
| CDR-L1 | L26 | S |
| CDR-L1 | L27 | Q |
| CDR-L1 | L28 | S |
| CDR-L1 | L29 | V |
| CDR-L1 | L30 | S |
| CDR-L1 | L31 | S |
| CDR-L1 | L32 | Y |
| CDR-L1 | L33 | L |
| CDR-L1 | L34 | A |
| CDR-L1 | L35 | W |
| CDR-L1 | L36 | Y |
| LFR2 | L37 | Q |
| LFR2 | L38 | Q |
| LFR2 | L39 | K |
| LFR2 | L40 | P |
| LFR2 | L41 | G |
| LFR2 | L42 | Q |
| LFR2 | L43 | A |
| LFR2 | L44 | P |
| LFR2 | L45 | R |
| LFR2 | L46 | L |
| LFR2 | L47 | L |
| LFR2 | L48 | I |
| LFR2 | L49 | Y |
| CDR-L2 | L50 | D |
| CDR-L2 | L51 | A |
| CDR-L2 | L52 | S |
| CDR-L2 | L53 | N |
| CDR-L2 | L54 | R |
| CDR-L2 | L55 | A |
| CDR-L2 | L56 | T |
| LFR3 | L57 | G |
| LFR3 | L58 | I |
| LFR3 | L59 | P |
| LFR3 | L60 | A |
| LFR3 | L61 | R |
| LFR3 | L62 | F |
| LFR3 | L63 | S |
| LFR3 | L64 | G |
| LFR3 | L65 | S |
| LFR3 | L66 | G |
| LFR3 | L67 | S |
| LFR3 | L68 | G |
| LFR3 | L69 | T |
| LFR3 | L70 | D |
| LFR3 | L71 | F |
| LFR3 | L72 | T |
| LFR3 | L73 | L |
| LFR3 | L74 | T |
| LFR3 | L75 | I |
| LFR3 | L76 | S |
| LFR3 | L77 | S |
| LFR3 | L78 | L |
| LFR3 | L79 | Q |
| LFR3 | L80 | P |
| LFR3 | L81 | E |
| LFR3 | L82 | D |
| LFR3 | L83 | F |
| LFR3 | L84 | A |
| LFR3 | L85 | V |
| LFR3 | L86 | Y |
| LFR3 | L87 | Y |
| LFR3 | L88 | C |
| CDR-L3 | L89 | Q |
| CDR-L3 | L90 | Q |
| CDR-L3 | L91 | S |
| CDR-L3 | L92 | S |
| CDR-L3 | L93 | N |
| CDR-L3 | L94 | W |
| CDR-L3 | L95 | P |
| CDR-L3 | L96 | P |
| CDR-L3 | L97 | T |
| LFR4 | L98 | F |
| LFR4 | L99 | G |
| LFR4 | L100 | Q |
| LFR4 | L101 | G |
| LFR4 | L102 | T |
| LFR4 | L103 | K |
| LFR4 | L104 | V |
| LFR4 | L105 | E |
| LFR4 | L106 | I |
| LFR4 | L107 | K |

**Heavy chain (Kabat numbering)**

| CDRs (Kabat) | Number (Kabat) | AMINO ACID |
|---|---|---|
| HFR1 | H1 | E |
| HFR1 | H2 | V |
| HFR1 | H3 | Q |
| HFR1 | H4 | L |
| HFR1 | H5 | L |
| HFR1 | H6 | E |
| HFR1 | H7 | S |
| HFR1 | H8 | G |
| HFR1 | H9 | G |
| HFR1 | H10 | G |
| HFR1 | H11 | L |
| HFR1 | H12 | V |
| HFR1 | H13 | Q |
| HFR1 | H14 | P |
| HFR1 | H15 | G |
| HFR1 | H16 | G |
| HFR1 | H17 | S |
| HFR1 | H18 | L |
| HFR1 | H19 | R |
| HFR1 | H20 | L |
| HFR1 | H21 | S |
| HFR1 | H22 | C |
| HFR1 | H23 | A |
| HFR1 | H24 | V |
| HFR1 | H25 | S |
| HFR1 | H26 | G |
| HFR1 | H27 | F |
| HFR1 | H28 | T |
| HFR1 | H29 | F |
| HFR1 | H30 | N |
| CDR-H1 | H31 | S |
| CDR-H1 | H32 | F |
| CDR-H1 | H33 | A |
| CDR-H1 | H34 | M |
| CDR-H1 | H35 | S |
| CDR-H1 | H36 | W |
| HFR2 | H37 | V |
| HFR2 | H38 | R |
| HFR2 | H39 | Q |
| HFR2 | H40 | A |
| HFR2 | H41 | P |
| HFR2 | H42 | G |
| HFR2 | H43 | K |
| HFR2 | H44 | G |
| HFR2 | H45 | L |
| HFR2 | H46 | E |
| HFR2 | H47 | W |
| HFR2 | H48 | V |
| CDR-H2 | H49 | S |
| CDR-H2 | H50 | A |
| CDR-H2 | H51 | I |
| CDR-H2 | H52 | S |
| CDR-H2 | H52A | G |
| CDR-H2 | H53 | S |
| CDR-H2 | H54 | G |
| CDR-H2 | H55 | G |
| CDR-H2 | H56 | G |
| CDR-H2 | H57 | T |
| CDR-H2 | H58 | Y |
| CDR-H2 | H59 | Y |
| CDR-H2 | H60 | A |
| CDR-H2 | H61 | D |
| CDR-H2 | H62 | S |
| CDR-H2 | H63 | V |
| CDR-H2 | H64 | K |
| CDR-H2 | H65 | G |
| HFR3 | H66 | R |
| HFR3 | H67 | F |
| HFR3 | H68 | T |
| HFR3 | H69 | I |
| HFR3 | H70 | S |
| HFR3 | H71 | R |
| HFR3 | H72 | D |
| HFR3 | H73 | N |
| HFR3 | H74 | S |
| HFR3 | H75 | K |
| HFR3 | H76 | N |
| HFR3 | H77 | T |
| HFR3 | H78 | L |
| HFR3 | H79 | Y |
| HFR3 | H80 | L |
| HFR3 | H81 | Q |
| HFR3 | H82 | M |
| HFR3 | H82A | N |
| HFR3 | H82B | S |
| HFR3 | H82C | L |
| HFR3 | H83 | R |
| HFR3 | H84 | A |
| HFR3 | H85 | E |
| HFR3 | H86 | D |
| HFR3 | H87 | T |
| HFR3 | H88 | A |
| HFR3 | H89 | V |
| HFR3 | H90 | Y |
| HFR3 | H91 | F |
| HFR3 | H92 | C |
| CDR-H3 | H93 | A |
| CDR-H3 | H94 | K |
| CDR-H3 | H95 | D |
| CDR-H3 | H96 | Q |
| CDR-H3 | H97 | I |
| CDR-H3 | H98 | L |
| CDR-H3 | H99 | W |
| CDR-H3 | H100 | F |
| CDR-H3 | H100A | G |
| CDR-H3 | H100B | E |
| CDR-H3 | H100C | P |
| CDR-H3 | H100D | V |
| CDR-H3 | H100E | F |
| CDR-H3 | H101 | D |
| CDR-H3 | H102 | Y |
| HFR4 | H103 | W |
| HFR4 | H104 | G |
| HFR4 | H105 | Q |
| HFR4 | H106 | G |
| HFR4 | H107 | T |
| HFR4 | H108 | L |
| HFR4 | H109 | V |
| HFR4 | H110 | T |
| HFR4 | H111 | V |
| HFR4 | H112 | S |
| HFR4 | H113 | S |

EP 4 625 420 A1

# Fig.15

| | L1 | L9 | L10 | L13 | L17 | L19 | L21 | L45 | L58 | L60 | L77 | L79 | L83 | L85 | L100 | L105 | L106 | H1 | H5 | H13 | H24 | H30 | H49 | H91 | H94 |
|---|---|---|---|---|---|---|---|---|---|---|---|---|---|---|---|---|---|---|---|---|---|---|---|---|---|
| WILD TYPE | E | A | T | L | E | A | L | R | I | A | S | E | F | V | Q | E | I | E | L | Q | V | N | S | F | K |
| AMINO ACID OF HIGHEST APPEARANCE FREQUENCY | Q | S | S | A | Q | V | I | K | V | D | G | Q | E | D | G | T | V | Q | V | K | A | S | G | Y | R |
| LIBRARY | O | O | | O | O | | O | O | | O | O | O | | O | O | | | | O | | O | O | O | O | O |

## Fig.16

Fig.17

EP 4 625 420 A1

# Fig.18

# Fig.19(a)

| | | L1 | L9 | L13 | L17 | L21 | L45 | L60 | L77 | L79 | L85 | L100 | H16 | H21 | H23 | H27 | H49 | H79 | H94 |
|---|---|---|---|---|---|---|---|---|---|---|---|---|---|---|---|---|---|---|---|
| | | | | | | | | | SEQUENCE | | | | | | | | | | |
| WILD TYPE | | E | A | L | E | L | R | A | S | E | V | Q | R | D | K | I | A | F | T |
| CASE 9 | C9_1 | E | A | A | E | I | K | D | G | E | V | G | G | S | K | I | A | Y | T |
| | C9_2 | E | A | A | E | I | K | A | G | E | V | G | G | S | K | I | A | Y | T |
| | C9_3 | E | A | L | E | I | K | D | G | E | V | G | G | S | K | I | A | Y | T |
| | C9_4 | E | A | A | E | I | K | A | G | E | V | G | G | S | A | I | A | Y | T |
| | C9_5 | E | A | A | Q | I | K | D | G | E | V | G | G | S | K | I | A | Y | T |
| | C9_6 | E | A | A | Q | I | K | D | G | E | V | G | G | S | A | I | A | Y | T |
| | C9_7 | E | A | A | E | I | K | A | G | E | V | G | G | S | A | I | A | F | T |
| | C9_8 | E | A | A | E | I | K | D | G | E | V | G | G | S | A | I | A | F | T |
| | C9_9 | E | A | A | E | I | K | A | G | E | V | G | G | S | K | I | A | F | T |
| | C9_10 | E | A | L | E | I | K | D | G | E | V | G | G | S | A | I | A | Y | T |
| | C9_11 | E | A | L | E | I | K | D | G | E | V | G | G | S | K | I | A | F | T |
| | C9_12 | E | A | A | Q | I | K | D | G | E | V | G | G | S | A | I | A | F | T |
| | C9_13 | E | A | L | Q | I | K | D | G | E | V | G | G | S | K | I | A | F | T |
| | C9_14 | E | A | A | Q | I | K | A | G | E | V | G | G | S | A | I | A | Y | T |
| | C9_15 | E | A | A | Q | I | K | A | G | E | V | G | G | S | A | I | A | F | T |
| | C9_16 | E | A | A | Q | I | K | A | G | E | V | G | G | S | K | I | A | Y | T |
| | C9_17 | E | A | L | Q | I | K | D | G | E | V | G | G | S | K | I | A | Y | T |
| | C9_18 | E | A | L | Q | I | K | D | G | E | V | G | G | S | A | I | A | F | T |
| | C9_19 | E | A | L | E | I | K | D | G | E | V | G | G | S | A | I | A | F | T |
| | C9_20 | E | A | L | Q | I | K | D | G | E | V | G | G | S | A | I | A | Y | T |

# Fig.19(b)

| | | L1 | L9 | L13 | L17 | L21 | L45 | L60 | L77 | L79 | L85 | L100 | H16 | H21 | H23 | H27 | H49 | H79 | H94 |
|---|---|---|---|---|---|---|---|---|---|---|---|---|---|---|---|---|---|---|---|
| | | | | | | | | | | | SEQUENCE | | | | | | | | |
| WILD TYPE | | E | A | L | E | L | R | A | S | E | V | Q | R | D | K | I | A | F | T |
| CASE 10 | C10_1 | E | A | A | E | I | K | D | G | E | V | G | G | S | K | I | G | Y | T |
| | C10_2 | E | A | A | E | I | K | D | G | E | V | G | G | S | K | F | G | Y | T |
| | C10_3 | E | A | A | E | I | K | D | S | E | V | G | G | S | K | I | G | Y | T |
| | C10_4 | E | A | L | E | I | K | D | G | E | V | G | G | S | K | I | G | Y | T |
| | C10_5 | E | A | A | E | I | K | D | G | E | V | G | G | S | A | I | G | Y | T |
| | C10_6 | E | A | A | E | I | K | D | S | E | V | G | G | S | A | I | G | Y | T |
| | C10_7 | E | A | A | E | I | K | D | G | E | V | Q | G | S | A | I | G | Y | T |
| | C10_8 | E | A | L | E | I | K | D | S | E | V | G | G | S | A | F | G | Y | T |
| | C10_9 | E | A | L | E | I | K | D | G | E | V | G | G | S | K | F | G | Y | T |
| | C10_10 | E | A | A | E | I | K | D | S | E | V | Q | G | S | A | F | G | Y | T |
| | C10_11 | E | A | A | E | I | K | D | G | E | V | G | G | S | A | F | G | Y | T |
| | C10_12 | E | A | A | E | I | K | D | G | E | V | Q | G | S | A | F | G | Y | T |
| | C10_13 | E | A | L | E | I | K | D | S | E | V | G | G | S | A | I | G | Y | T |
| | C10_14 | E | A | L | E | I | K | D | G | E | V | G | G | S | A | F | G | Y | T |
| | C10_15 | E | A | L | E | I | K | D | S | E | V | G | G | S | K | I | G | Y | T |
| | C10_16 | E | A | A | E | I | K | D | S | E | V | G | G | S | A | F | G | Y | T |
| | C10_17 | E | A | L | E | I | K | D | G | E | V | G | G | S | A | I | G | Y | T |
| | C10_18 | E | A | L | E | I | K | D | G | E | V | Q | G | S | A | F | G | Y | T |

*Fig.20*

Fig.21

| | EC$_{50}$ (µg/mL) |
|---|---|
| WILD TYPE | 0.063 |
| C9_10 | 0.064 |
| C9_14 | 0.053 |

## Fig.22

## Fig.23

# Fig.24(a)

| | | | | | | | | | | SEQUENCE | | | | | | | | |
|---|---|---|---|---|---|---|---|---|---|---|---|---|---|---|---|---|---|---|
| | L1 | L9 | L13 | L17 | L21 | L45 | L60 | L77 | L79 | L85 | L100 | H16 | H21 | H23 | H27 | H49 | H79 | H94 |
| WILD TYPE | E | A | L | E | L | R | A | S | E | V | Q | R | D | K | I | A | F | T |
| CASE 11 C11_1 | E | A | L | E | L | R | A | G | E | V | G | G | S | A | F | G | F | T |
| C11_2 | E | A | L | E | L | R | D | G | E | V | G | G | S | A | F | G | F | T |
| C11_3 | E | A | L | E | L | R | A | G | E | V | Q | G | S | A | F | G | F | T |
| C11_4 | E | A | L | E | L | R | D | G | E | V | Q | G | S | A | F | G | F | T |
| C11_5 | E | A | L | E | L | R | D | G | E | V | G | G | S | A | F | G | Y | T |
| C11_6 | E | A | L | E | L | R | D | G | E | V | Q | G | S | A | F | G | Y | T |
| C11_7 | E | A | L | E | I | R | D | G | E | V | Q | G | S | A | F | G | F | T |
| C11_8 | E | A | L | E | I | R | D | G | E | V | G | G | S | A | F | G | Y | T |
| C11_9 | E | A | L | E | L | R | D | G | E | V | Q | G | S | A | F | A | Y | T |
| C11_10 | E | A | L | E | I | R | D | G | E | V | Q | G | S | A | F | G | Y | T |
| C11_11 | E | A | L | E | L | R | D | G | E | V | Q | G | S | A | F | A | F | T |
| C11_12 | E | A | L | E | I | R | A | G | E | V | G | G | S | A | F | G | F | T |
| C11_13 | E | A | L | E | L | R | D | G | E | V | G | G | S | A | F | A | F | T |
| C11_14 | E | A | L | E | I | R | D | G | E | V | G | G | S | A | I | G | F | T |
| C11_15 | E | A | L | E | L | R | A | G | E | V | Q | G | S | A | F | G | Y | T |
| C11_16 | E | A | L | E | I | R | D | G | E | V | Q | G | S | A | F | A | Y | T |
| C11_17 | E | A | L | E | L | R | A | G | E | V | Q | G | S | A | F | A | F | T |
| C11_18 | E | A | L | E | L | R | D | G | E | V | G | G | S | A | F | A | Y | T |
| C11_19 | E | A | L | E | I | R | D | G | E | V | G | G | S | A | I | G | Y | T |
| C11_20 | E | A | L | E | I | R | D | G | E | V | Q | G | S | A | I | G | F | T |
| C11_21 | E | A | L | E | I | R | D | G | E | V | G | G | S | A | F | A | Y | T |
| C11_22 | E | A | L | E | L | R | A | G | E | V | G | G | S | A | F | G | Y | T |
| C11_23 | E | A | L | E | L | R | A | G | E | D | G | G | S | A | F | G | F | T |
| C11_24 | E | A | L | E | L | R | D | G | E | D | Q | G | S | A | F | G | F | T |
| C11_25 | E | A | L | E | L | R | A | G | E | V | Q | G | S | A | F | A | Y | T |
| C11_26 | E | A | A | E | I | R | D | G | E | V | Q | G | S | A | F | G | F | T |
| C11_27 | Q | A | L | E | I | R | D | G | E | V | Q | G | S | A | F | G | Y | T |

EP 4 625 420 A1

# Fig.24(b)

| | L1 | L9 | L13 | L17 | L21 | L45 | L60 | L77 | L79 | L85 | L100 | H16 | H21 | H23 | H27 | H49 | H79 | H94 |
|---|----|----|-----|-----|-----|-----|-----|-----|-----|-----|------|-----|-----|-----|-----|-----|-----|-----|
| WILD TYPE | E | A | L | E | L | R | A | S | E | V | Q | R | D | K | I | A | F | T |
| C12_1 | E | A | L | E | L | R | D | G | E | V | Q | G | D | A | I | G | F | T |
| C12_2 | E | A | L | E | L | R | D | S | E | V | Q | G | D | A | I | A | F | T |
| C12_3 | E | A | L | E | L | R | D | G | E | V | Q | G | D | A | I | G | Y | T |
| C12_4 | E | A | L | E | L | R | D | S | E | V | Q | G | D | A | I | A | Y | T |
| C12_5 | E | A | L | E | I | R | D | G | E | V | Q | G | D | A | I | G | Y | T |
| C12_6 | E | A | L | E | I | R | D | G | E | V | G | G | D | A | I | G | F | T |
| C12_7 | E | A | A | E | L | R | D | G | E | V | Q | G | D | A | I | G | F | T |
| C12_8 | E | A | A | E | L | R | D | G | E | V | Q | G | D | A | I | A | F | T |
| C12_9 | E | A | L | E | L | R | D | G | E | D | Q | G | D | A | I | G | F | T |
| C12_10 | E | A | A | E | L | R | D | G | E | V | Q | G | D | A | I | A | Y | T |
| C12_11 | E | A | A | E | I | R | D | G | E | V | Q | G | D | A | I | G | Y | T |
| C12_12 | E | A | L | E | L | R | D | S | E | V | Q | G | D | A | I | G | F | T |
| C12_13 | E | A | L | E | I | R | D | G | E | D | Q | G | D | A | I | G | Y | T |
| C12_14 | Q | A | A | Q | I | K | A | S | E | V | Q | G | S | A | I | G | Y | T |
| C12_15 | E | A | L | E | L | R | A | G | E | V | Q | G | D | A | I | A | F | T |
| C12_16 | E | A | L | E | I | R | D | S | E | V | Q | G | D | A | I | G | F | T |

SEQUENCE

EP 4 625 420 A1

# Fig.24(c)

| | L1 | L9 | L13 | L17 | L21 | L45 | L60 | L77 | L79 | L85 | L100 | H16 | H21 | H23 | H27 | H49 | H79 | H94 |
|---|---|---|---|---|---|---|---|---|---|---|---|---|---|---|---|---|---|---|
| WILD TYPE | E | A | L | E | L | R | A | S | E | V | Q | R | D | K | I | A | F | T |
| C13_1 | Q | A | A | E | I | R | D | G | E | V | Q | G | D | A | I | A | F | T |
| C13_2 | Q | A | L | E | I | R | D | S | E | V | Q | G | D | A | I | A | F | T |
| C13_3 | E | S | L | E | I | R | A | S | E | V | Q | G | D | A | I | A | F | T |
| C13_4 | Q | A | L | E | I | R | D | G | E | V | Q | G | D | A | F | A | F | T |
| C13_5 | E | S | L | E | I | R | D | S | E | V | Q | G | D | A | I | A | F | T |
| C13_6 | Q | A | A | E | I | R | D | S | E | V | Q | G | D | A | I | A | F | T |
| C13_7 | E | A | L | E | I | R | D | G | E | D | Q | G | D | A | F | A | F | T |
| C13_8 | Q | A | L | E | I | R | A | S | E | V | Q | G | D | A | I | A | F | T |
| C13_9 | Q | A | L | E | I | R | D | S | E | D | Q | G | D | A | I | A | F | T |
| C13_10 | E | A | L | E | I | R | D | G | E | V | Q | G | D | A | F | A | F | T |
| C13_11 | E | A | L | E | I | R | D | G | E | D | Q | G | D | A | F | A | Y | T |
| C13_12 | Q | A | L | E | I | R | D | G | E | D | Q | G | D | A | F | A | F | T |
| C13_13 | Q | A | L | E | I | R | D | G | E | D | Q | G | D | A | I | A | F | R |
| C13_14 | E | A | L | E | I | R | A | S | E | V | Q | G | D | A | I | A | F | T |
| C13_15 | Q | A | A | E | I | R | D | G | E | V | Q | G | D | A | I | A | Y | T |
| C13_16 | Q | A | L | E | I | R | D | G | E | V | Q | G | D | A | I | A | F | R |
| C13_17 | E | S | A | E | I | R | D | S | E | V | Q | G | D | A | I | A | F | T |
| C13_18 | E | A | L | E | I | R | D | S | E | D | Q | G | D | A | I | A | F | T |
| C13_19 | Q | A | L | E | I | R | D | G | E | D | Q | G | D | A | I | A | Y | T |
| C13_20 | Q | A | L | E | I | R | A | S | E | D | Q | G | D | A | I | A | F | T |
| C13_21 | E | A | A | E | I | R | D | S | E | V | Q | G | D | A | I | A | F | T |
| C13_22 | Q | A | L | E | I | R | D | S | E | V | Q | G | D | A | I | A | F | R |
| C13_23 | Q | A | L | E | L | R | D | G | E | D | Q | G | D | A | I | A | F | T |
| C13_24 | Q | A | L | E | I | R | D | G | E | V | Q | G | S | A | F | A | F | T |
| C13_25 | E | A | L | E | I | R | D | G | E | V | Q | G | D | A | F | A | Y | T |

# Fig.24(d)

EP 4 625 420 A1

| | | | | | | | | | | | SEQUENCE | | | | | | | |
|---|---|---|---|---|---|---|---|---|---|---|---|---|---|---|---|---|---|---|
| | L1 | L9 | L13 | L17 | L21 | L45 | L60 | L77 | L79 | L85 | L100 | H16 | H21 | H23 | H27 | H49 | H79 | H94 |
| WILD TYPE | E | A | L | E | L | R | A | S | E | V | Q | R | D | K | I | A | F | T |
| C11_12_1 | E | A | L | E | I | R | D | G | E | V | Q | G | S | A | I | G | Y | T |
| C11_12_2 | E | A | L | E | I | R | D | G | E | V | Q | G | S | A | I | A | Y | T |

EP 4 625 420 A1

## Fig.24(e)

| | | L1 | L9 | L13 | L17 | L21 | L45 | L60 | L77 | L79 | L85 | L100 | H16 | H21 | H23 | H27 | H49 | H79 | H94 |
|---|---|----|----|-----|-----|-----|-----|-----|-----|-----|-----|------|-----|-----|-----|-----|-----|-----|-----|
| | | | | | | | | | | | | | | | | | | | |
| WILD TYPE | | E | A | L | E | L | R | A | S | E | V | Q | R | D | K | I | A | F | T |
| C11_13_1 | | E | A | L | E | I | R | D | G | E | V | G | G | S | A | F | G | F | T |

SEQUENCE

# Fig.24(f)

| | L1 | L9 | L13 | L17 | L21 | L45 | L60 | L77 | L79 | L85 | L100 | H16 | H21 | H23 | H27 | H49 | H79 | H94 |
|---|---|---|---|---|---|---|---|---|---|---|---|---|---|---|---|---|---|---|
| | | | | | | | SEQUENCE | | | | | | | | | | | |
| WILD TYPE | E | A | L | E | L | R | A | S | E | V | Q | R | D | K | I | A | F | T |
| C12_13_1 | E | A | L | E | L | R | D | G | E | V | Q | G | D | A | I | A | Y | T |
| C12_13_2 | E | A | L | E | L | R | D | G | E | V | Q | G | D | A | I | A | F | T |
| C12_13_3 | E | A | L | E | I | R | D | G | E | V | Q | G | D | A | I | A | F | T |
| C12_13_4 | E | A | L | E | I | R | D | G | E | V | Q | G | D | A | I | G | F | T |
| C12_13_5 | E | A | L | E | I | R | D | G | E | V | Q | G | D | A | I | A | Y | T |
| C12_13_6 | E | A | A | E | I | R | D | G | E | V | Q | G | D | A | I | G | F | T |
| C12_13_7 | E | A | L | E | I | R | D | G | E | D | Q | G | D | A | I | G | F | T |
| C12_13_8 | E | A | L | E | I | R | D | G | E | D | Q | G | D | A | I | A | F | T |
| C12_13_9 | E | A | L | E | I | R | D | G | E | D | Q | G | D | A | I | A | Y | T |
| C12_13_10 | Q | A | L | E | I | R | D | G | E | V | Q | G | D | A | I | G | F | T |
| C12_13_11 | Q | A | L | E | I | R | D | G | E | V | Q | G | D | A | I | A | F | T |
| C12_13_12 | E | A | A | E | I | R | D | G | E | V | Q | G | D | A | I | A | F | T |
| C12_13_13 | Q | A | L | E | I | R | D | G | E | V | Q | G | D | A | I | A | Y | T |
| C12_13_14 | E | A | L | E | L | R | D | G | E | V | Q | G | D | A | F | A | Y | T |
| C12_13_15 | Q | A | A | E | I | R | D | G | E | V | Q | G | D | A | I | G | F | T |
| C12_13_16 | E | A | L | E | L | R | D | G | E | D | Q | G | D | A | I | A | F | T |
| C12_13_17 | E | A | L | E | L | R | D | G | E | D | Q | G | D | A | I | A | Y | T |
| C12_13_18 | E | A | A | E | I | R | D | G | E | V | Q | G | D | A | I | A | Y | T |
| C12_13_19 | E | A | L | E | I | R | D | S | E | V | Q | G | D | A | I | A | F | T |
| C12_13_20 | Q | A | L | E | I | R | D | G | E | D | Q | G | D | A | I | A | F | T |

EP 4 625 420 A1

EP 4 625 420 A1

# Fig.24(g)

| | SEQUENCE | | | | | | | | | | | | | | | | | |
|---|---|---|---|---|---|---|---|---|---|---|---|---|---|---|---|---|---|---|
| | L1 | L9 | L13 | L17 | L21 | L45 | L60 | L77 | L79 | L85 | L100 | H16 | H21 | H23 | H27 | H49 | H79 | H94 |
| WILD TYPE | E | A | L | E | L | R | A | S | E | V | Q | R | D | K | I | A | F | T |
| C11_12_13_1 | Q | A | A | Q | I | K | A | S | E | V | G | G | S | A | I | G | Y | T |

# Fig.25

X WILD TYPE
○ MUTANT (MUTANT PRODUCED IN CASE 11 ONLY)
□ MUTANT (MUTANT PRODUCED IN CASE 12 ONLY)
△ MUTANT (MUTANT PRODUCED IN CASE 13 ONLY)
● MUTANT (MUTANT PRODUCED IN BOTH CASES 11 AND 12)
■ MUTANT (MUTANT PRODUCED IN BOTH CASES 11 AND 13)
▲ MUTANT (MUTANT PRODUCED IN BOTH CASES 12 AND 13)
▼ MUTANT (MUTANT PRODUCED IN ALL CASES 11, 12, AND 13)

EP 4 625 420 A1

# Fig.26

| Sample | EC50 (µg/ml) |
| --- | --- |
| WILD TYPE | 0.240 |
| C11_6 | 0.309 |
| C11_9 | 0.397 |
| C11_20 | 0.389 |
| C11_27 | 0.298 |
| C11_12_1 | 0.213 |
| C11_12_2 | 0.289 |
| C12_5 | 0.241 |
| C12_11 | 0.188 |
| C12_13 | 0.238 |
| C12_13_12 | 0.311 |
| C12_13_13 | 0.335 |
| C13_23 | 0.134 |
| C13_25 | 0.221 |

## Fig.27

## Fig.28

Fig.29

## INTERNATIONAL SEARCH REPORT

| International application No. |
|---|
| **PCT/JP2023/043058** |

### A. CLASSIFICATION OF SUBJECT MATTER

*G16B 40/00*(2019.01)i; *C07K 16/00*(2006.01)i; *C12P 21/08*(2006.01)i; *C40B 40/10*(2006.01)i
FI: G16B40/00 ZNA; C40B40/10; C12P21/08; C07K16/00

According to International Patent Classification (IPC) or to both national classification and IPC

### B. FIELDS SEARCHED

Minimum documentation searched (classification system followed by classification symbols)

G06Q10/00-99/00; G16B5/00-99/00; G16Z99/00; C07K16/00; C12P21/08; C40B40/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | SAITO, Y. et al. Machine-Learning-Guided Mutagenesis for Directed Evolution of Fluorescent Proteins. ACS Synth. Biol. 2018, abstract, pp. 2014-2017, 2020<br>    abstract, pp. 2014-2017, 2020 | 1-22 |
| Y | WO 2022/026551 A1 (FLAGSHIP PIONEERING INNOVATIONS VI LLC) 03 February 2022 (2022-02-03)<br>    paragraphs [0034]-[0036], [0041], [0048]-[0055] | 1-22 |
| Y | JP 2010-532324 A (ESBATECH, AN ALCON BIOMEDICAL RESEARCH UNIT LLC) 07 October 2010 (2010-10-07)<br>    paragraphs [0007], [0056]-[0057], [0096], [0119]-[0123] | 1-22 |
| A | JP 2022-531295 A (BOARD OF REGENTS, THE UNIV. OF TEXAS SYSTEM) 08 July 2022 (2022-07-08)<br>    paragraphs [0008], [0045]-[0048], [0052], [0079]-[0092] | 1-22 |

☑ Further documents are listed in the continuation of Box C.     ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **08 February 2024** | **20 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

| International application No. |
| --- |
| **PCT/JP2023/043058** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2014/0032186 A1 (DNA TWOPOINTO, INC.) 30 January 2014 (2014-01-30) paragraphs [0053]-[0063], [0083]-[0085], [0098], [0111]-[0119], [0121], [0150]-[0157], [0179]-[0184], [0190]-[0213] | 1-22 |
| A | JP 2008-503589 A (MAXYGEN, INC.) 07 February 2008 (2008-02-07) paragraphs [0033]-[0048], [0058]-[0060], [0081], [0094], [0111]-[0115] | 1-22 |
| A | JP 2020-77206 A (MIRACA RES INSTITUTE GK) 21 May 2020 (2020-05-21) paragraphs [0012]-[0014], [0017]-[0022], [0038], [0049]-[0051], [0057]-[0059], [0068]-[0077], [0104]-[0116], [0131]-[0136] | 1-22 |

Form PCT/ISA/210 (second sheet) (July 2022)

## INTERNATIONAL SEARCH REPORT

| International application No. |
|---|
| **PCT/JP2023/043058** |

---

**Box No. I**      **Nucleotide and/or amino acid sequence(s) (Continuation of item 1.c of the first sheet)**

---

1. With regard to any nucleotide and/or amino acid sequence disclosed in the international application, the international search was carried out on the basis of a sequence listing:

     a.   ☑ forming part of the international application as filed.

     b.   ☐ furnished subsequent to the international filing date for the purposes of international search (Rule 13*ter*.1(a)),

         ☐ accompanied by a statement to the effect that the sequence listing does not go beyond the disclosure in the international application as filed.


2.   ☐ With regard to any nucleotide and/or amino acid sequence disclosed in the international application, this report has been established to the extent that a meaningful search could be carried out without a WIPO Standard ST.26 compliant sequence listing.


3. Additional comments:

---

## INTERNATIONAL SEARCH REPORT

| International application No. |
| --- |
| **PCT/JP2023/043058** |

| **Box No. III** | **Observations where unity of invention is lacking (Continuation of item 3 of first sheet)** |
| --- | --- |

This International Searching Authority found multiple inventions in this international application, as follows:

The invention in claims 1-16 shares the common technical feature of proteins with amino acid variations (antibodies) with the invention in claims 17-22.
    However, in light of the disclosure of documents 1-3 cited in the international search report, said technical feature does not make a contribution over the prior art, and thus it cannot be said that the technical feature is a special technical feature. Also, there are no other same or corresponding special technical features between these inventions
    Thus, the inventions in claims 1-22 include the following two inventions.
(Invention 1) Invention in claims 1-16 (method of antibody construction)
(Invention 2) Invention in claims 17-22 (antibody variants)

1. ☐   As all required additional search fees were timely paid by the applicant, this international search report covers all searchable claims.

2. ☑   As all searchable claims could be searched without effort justifying additional fees, this Authority did not invite payment of additional fees.

3. ☐   As only some of the required additional search fees were timely paid by the applicant, this international search report covers only those claims for which fees were paid, specifically claims Nos.:

4. ☐   No required additional search fees were timely paid by the applicant. Consequently, this international search report is restricted to the invention first mentioned in the claims; it is covered by claims Nos.:

**Remark on Protest**    ☐   The additional search fees were accompanied by the applicant's protest and, where applicable, the payment of a protest fee.

       ☐   The additional search fees were accompanied by the applicant's protest but the applicable protest fee was not paid within the time limit specified in the invitation.

       ☐   No protest accompanied the payment of additional search fees.

Form PCT/ISA/210 (continuation of first sheet) (July 2022)

# EP 4 625 420 A1

## INTERNATIONAL SEARCH REPORT
### Information on patent family members

International application No.

**PCT/JP2023/043058**

| Patent document cited in search report | | | Publication date (day/month/year) | Patent family member(s) | | | Publication date (day/month/year) |
|---|---|---|---|---|---|---|---|
| WO | 2022/026551 | A1 | 03 February 2022 | JP | 2023-536118 | A | |
| | | | | paragraphs [0029]-[0032], [0037], [0042]-[0049] | | | |
| | | | | EP | 4189687 | A1 | |
| | | | | CN | 116157870 | A | |
| | | | | KR | 10-2023-0051515 | A | |
| JP | 2010-532324 | A | 07 October 2010 | US | 2009/0028848 | A1 | |
| | | | | paragraphs [0009], [0093]-[0094], [0138], [0179]-[0183] | | | |
| | | | | WO | 2009/000098 | A2 | |
| | | | | EP | 2164961 | A2 | |
| | | | | CN | 101688200 | A | |
| | | | | KR | 10-2010-0028053 | A | |
| JP | 2022-531295 | A | 08 July 2022 | US | 2022/0076787 | A1 | |
| | | | | paragraphs [0008], [0068]-[0071], [0075], [0102]-[0115] | | | |
| | | | | US | 2023/0162816 | A1 | |
| | | | | WO | 2020/247126 | A2 | |
| | | | | EP | 3962932 | A2 | |
| | | | | CN | 113727994 | A | |
| | | | | KR | 10-2022-0002978 | A | |
| US | 2014/0032186 | A1 | 30 January 2014 | US | 2008/0050357 | A1 | |
| | | | | US | 2011/0166844 | A1 | |
| | | | | WO | 2005/012877 | A2 | |
| | | | | WO | 2005/013090 | A2 | |
| | | | | EP | 1660970 | A2 | |
| | | | | EP | 2434420 | A2 | |
| JP | 2008-503589 | A | 07 February 2008 | US | 2005/0084907 | A1 | |
| | | | | paragraphs [0039]-[0059], [0076]-[0078], [0100], [0114], [0131]-[0136] | | | |
| | | | | US | 2007/0239364 | A1 | |
| | | | | US | 2008/0132416 | A1 | |
| | | | | US | 2008/0147369 | A1 | |
| | | | | US | 2008/0220990 | A1 | |
| | | | | US | 2010/0004135 | A1 | |
| | | | | US | 2010/0004136 | A1 | |
| | | | | US | 2010/0005047 | A1 | |
| | | | | US | 2014/0249035 | A1 | |
| | | | | US | 2015/0065357 | A1 | |
| | | | | WO | 2006/002267 | A1 | |
| | | | | EP | 1761879 | A1 | |
| JP | 2020-77206 | A | 21 May 2020 | (Family: none) | | | |

Form PCT/ISA/210 (patent family annex) (July 2022)

76

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2022194776 A
- WO 2005012877 A
- WO 2005013090 A

### Non-patent literature cited in the description

- **SAITO et al.** Machine-Learning-Guided Mutagenesis for Directed Evolution of Fluorescent Proteins. *ACS Synth Biol.*, 2018, vol. 7 (9), 2014-2022
- **LIU et al.** Antibody complementarity determining region design using high-capacity machine learning. *Bioinformatics*, 2020, vol. 36 (7), 2126-2133
- **SAKA et al.** Antibody design using LSTM based deep generative model from phage display library for affinity maturation. *Scientific Reports*, 2021, vol. 11 (1), 5852
- **VAN WESTEN et al.** *J Cheminform.*, 2013, vol. 5, 41
- **UENO et al.** *Mater. Discov.*, 2016, vol. 4, 18-21, https://github.com/tsudalab/combo
- **SWINDELLS, M. B. et al.** *J. Mol. Biol.*, 2017, vol. 429, 356-364
- **NIWA et al.** *PNAS*, 17 March 2009, vol. 106 (11), 4201-4206
- **ITO et al.** *Chemistry Letters*, 2021, vol. 50, 1867-187
- **KERWIN et al.** *J Pharm Sci.*, January 2020, vol. 109 (1), 233-246